(19) Europäisches Patentamt
European Patent Office
Office européen des brevets

(11) Veröffentlichungsnummer: **0 314 852 B1**

(12) # EUROPÄISCHE PATENTSCHRIFT

(45) Veröffentlichungstag der Patentschrift: **18.11.93**

(21) Anmeldenummer: **87810755.6**

(22) Anmeldetag: **14.12.87**

(51) Int. Cl.5: **C07D 233/90**, C07D 409/04, C07D 405/04, C07D 401/04, A01N 43/50, A01N 43/16, A01N 43/10, A01N 43/08, A01N 43/42

(54) **Verfahren zur Synthese von 1-substituierten Imidazol-5-carbonsäuren und -carbonsäure-derivaten.**

(30) Priorität: **06.11.87 CH 4331/87**

(43) Veröffentlichungstag der Anmeldung:
**10.05.89 Patentblatt 89/19**

(45) Bekanntmachung des Hinweises auf die Patenterteilung:
**18.11.93 Patentblatt 93/46**

(84) Benannte Vertragsstaaten:
**BE CH LI**

(56) Entgegenhaltungen:
**EP-A- 0 273 531**
**EP-A- 0 277 384**
**US-A- 3 991 072**

(73) Patentinhaber: **CIBA-GEIGY AG**
**Klybeckstrasse 141**
**CH-4002 Basel(CH)**

(72) Erfinder: **Töpfl, Werner, Dr.**
**Dorneckstrasse 68**
**CH-4143 Dornach(CH)**

EP 0 314 852 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Synthese von 1-substituierten Imidazol-5-carbonsäuren und -carbonsäurederivaten sowie neue Zwischenprodukte zur Durchführung dieses Verfahrens und Verfahren zur Herstellung der neuen Zwischenprodukte.

Imidazole der Formel I sind wertvolle herbizide bzw. pflanzenwachstumsregulatorische Wirkstoffe. Diese herbizid wirksamen Imidazole werden umfasst von der gattungsbildenden Formel I

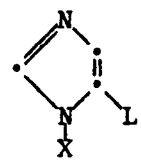

(I),

worin

L und X — gemeinsam für $L^1$ und $X^1$; $L^2$ und $X^2$; $L^3$ und $X^3$; oder $L^4$ und $X^4$ stehen; und

$L^1$ — $COOR^1$; $CONR^2R^3$; $CONR^4NHR^3$; oder CN;

$R^1$ — Wasserstoff; $C_1$-$C_7$-Alkyl; $C_3$-$C_7$-Alkenyl; $C_3$-$C_7$-Alkinyl; $C_3$-$C_7$-Cycloalkyl; $C_1$-$C_7$-Alkoxy-$C_1$-$C_7$-alkyl; oder Aryl-$C_1$-$C_5$-alkyl;

die Reste $R^2$, $R^3$ und $R^4$ unabhängig voneinander Wasserstoff; $C_1$-$C_5$-Alkyl; $C_3$-$C_5$-Alkenyl; $C_3$-$C_5$-Alkinyl; $C_3$-$C_7$-Cycloalkyl; Aryl; oder durch Aryl, $C_3$-$C_7$-Cycloalkyl, $C_3$-$C_7$-Cycloalkoxy, $C_1$-$C_5$-Alkoxy, Hydroxy, Carboxyl oder $C_1$-$C_5$-Alkyloxycarbonyl substituiertes $C_1$-$C_5$-alkyl; oder

$R_2$ und $R_3$ — gemeinsam mit dem Stickstoffatom, an welches sie gebunden sind einen unsubstituierten oder mit 1 bis 3 $C_1$-$C_5$-Alkylgruppen substituierten Piperidinyl, Pyrrolidinyl, 2-Oxopiperidinyl, 2-Oxopyrrolidinyl, Morpholinyl, Thiomorpholinyl, Piperazinyl oder 4-($C_1$-$C_4$)-Alkylpiperazinylring;

$X^1$ — 1-Indanyl, 1-Tetrahydronaphthalinyl, 5-Benzocycloheptanyl, 4-Tetrahydrobenzothienyl, 4-Tetrahydrobenzofuranyl, 5-Tetrahydrochinolinyl, 5-Tetrahydroisochinolinyl, 8-Tetrahydrochinolinyl, 8-Tetrahydroisochinolinyl, 9,10-Dihydro-9-anthracenyl, 9H-Fluoren-9-yl, 5-Dibenzo[a,d]cycloheptenyl, 5-Dibenzo[a,d]cycloheptanyl oder 1-Dihydronaphthalinyl, welches unsubstituiert oder bis zu sechsfach gleich oder verschieden durch $C_1$-$C_5$-Alkyl, Aryl-$C_1$-$C_5$-alkyl, Di-Aryl-$C_1$-$C_5$-alkyl, $C_1$-$C_5$-Alkoxy, Halogen, $C_3$-$C_7$-Alkenyl, Amino, Nitro, $C_1$-$C_5$-Alkylcarbonylamino, Trifluormethyl oder Difluormethoxy substituiert ist oder in welchem zwei geminal gebundene Substituenten gemeinsam mit dem Kohlenstoffatom, an welches sie gebunden sind, eine $C_3$-$C_7$-Spirocycloalkylgruppe bilden oder in welchem zwei Substituenten gemeinsam für eine $C_1$-$C_5$-Alkylen- oder $C_5$-$C_7$-Cycloalkylengruppe stehen, wobei diese Alkylen- oder Cycloalkylengruppe ihrerseits gegebenenfalls bis zu zweifach unabhängig voneinander durch $C_1$-$C_5$-Alkyl, Aryl-$C_1$-$C_5$-alkyl, Di-Aryl-$C_1$-$C_5$-alkyl, $C_1$-$C_5$-Alkoxy, Halogen, $C_3$-$C_7$-Alkenyl, Trifluormethyl, Difluormethoxy oder Aryl substituiert sein kann; oder

$X^1$ — die Gruppe

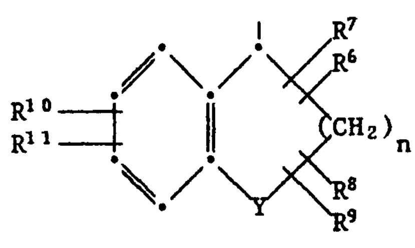

; oder

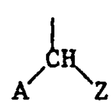

;

n — null; eins; oder zwei;

Y — die Gruppen $-CH_2S(O)_m-$, oder $-CH_2-N(R^{12})-$ in welchen das Heteroatom an das benzolische Ringkohlenstoffatom gebunden ist und worin m für 0, 1 oder 2 steht, bedeutet;

$R^6$, $R^7$, $R^8$ und $R^9$ — unabhängig voneinander Wasserstoff; $C_1$-$C_5$-Alkyl; Aryl-$C_1$-$C_5$-alkyl; Di-Aryl-$C_1$-$C_5$-alkyl; $C_1$-$C_5$-Alkoxy; Halogen; $C_3$-$C_7$-Alkenyl; Trifluormethyl; Difluormethoxy; oder Aryl; oder

$R^6$ und $R^7$ — gemeinsam einen annellierten Benzolrest, der gegebenenfalls bis zu zweifach durch $C_1$-$C_5$-Alkyl, $C_1$-$C_5$-Alkoxy, Halogen, ein- bis dreifach halogensubstituiertes $C_1$-$C_5$-Alkyl, ein- bis dreifach halogensubstituiertes $C_1$-$C_5$-Alkoxy, Nitro, Amino oder -NH-CO-M substituiert sein kann; oder

$R^6$ und $R^7$ — zusammen mit dem Kohlenstoffatom, an welches sie geminal gebunden sind, einen spirocyclischen $C_3$-$C_7$-Ring; oder

$R^6$ und $R^7$ — zusammen eine $C_1$-$C_5$-Alkylen- oder $C_5$-$C_7$-Cycloalkylengruppe, die gegebenenfalls bis zu zweifach unabhängig voneinander durch $C_1$-$C_5$-Alkyl, Aryl-$C_1$-$C_5$-alkyl, $C_1$-$C_5$-Alkoxy, Halogen, $C_3$-$C_7$-Alkenyl, Trifluormethyl, Difluormethoxy oder Aryl substituiert sein kann; und

$R^{10}$ und $R^{11}$ — unabhängig voneinander Wasserstoff; $C_1$-$C_5$-Alkyl; $C_1$-$C_5$-Alkoxy; Halogen; Trifluormethyl; Difluormethoxy; Cyano; Nitro; Amino; Mono-$C_1$-$C_5$-alkylamino; Di-$C_1$-$C_5$-alkylamino; oder -NH-CO-M; und

$R^{12}$ — Wasserstoff; $C_1$-$C_5$-Alkyl; $C_1$-$C_5$-Alkanoyl; oder 4-Methylphenylsulfonyl; und

A — Wasserstoff; gegebenenfalls bis zu zweifach durch $C_1$-$C_5$-Alkyl substituiertes $C_3$-$C_7$-Cycloalkyl; gegebenenfalls durch $C_1$-$C_7$-Alkyloxy oder Aryl substituiertes $C_1$-$C_7$-Alkyl; durch ein $C_1$-$C_7$-Alkoxy und einen Arylrest substituiertes $C_1$-$C_7$-Alkyl; oder ein unsubstituiertes oder bis zu zweifach unabhängig voneinander durch $C_1$-$C_5$-Alkyl, $C_1$-$C_5$-Alkoxy, Halogen, Nitro, Amino, Mono-$C_1$-$C_5$-Alkylamino, Di-$C_1$-$C_5$-Alkylamino, -NH-CO-M, Cyano, Trifluormethyl oder Difluormethoxy substituiertes Pyridinyl, Pyrimidinyl, Naphthalinyl, Furanyl oder Thiophenyl;
wobei im Rahmen der Definition von A Aryl für Phenyl, Pyridinyl, Pyrimidinyl, Naphthalinyl, Furanyl oder Thiophenyl steht und in der dieser Rest bis zu zweifach, im Falle von Aryl = Phenyl auch bis zu dreifach, unabhängig voneinander durch $C_1$-$C_5$-Alkyl, $C_1$-$C_5$-Alkoxy, Halogen, Nitro, Amino, Mono-$C_1$-$C_5$-alkylamino, Di-$C_1$-$C_5$-alkylamino, -NH-CO-M, Cyano, Trifluormethyl oder Difluormethoxy substituiert sein kann; und

Z — unsubstituiertes oder bis zu dreifach unabhängig voneinander durch $C_1$-$C_5$-Alkyl, $C_1$-$C_5$-Alkoxy, Halogen, Cyano, Nitro, Amino, Mono-$C_1$-$C_5$-alkylamino, Di-$C_1$-$C_5$-alkylamino, -NH-CO-M, Trifluormethyl oder Difluormethoxy substituiertes Naphthalinyl, Thiophenyl, Furanyl, Pyrimidinyl, Phenyl oder Pyridinyl; und

M — $C_1$-$C_5$-Alkyl; und
Aryl im Rahmen vorstehender Definitionen der Reste X und L ausserdem einen unsubstituierten oder bis zu dreifach gleich oder verschieden durch $C_1$-$C_5$-Alkyl, $C_1$-$C_5$-Alkyloxy oder Halogen substituierten Phenylrest; und

$L^2$ — $CO-D-R^{13}$;

D — O; oder $NR^{13}$;

$R^{13}$ — Wasserstoff; Phenyl; $C_3$-$C_6$-Alkenyl; unsubstituiertes oder bis zu dreifach gleich oder unterschiedlich durch $C_1$-$C_6$-Alkoxy, $C_1$-$C_3$-Dialkylamino oder Halogen substituiertes $C_1$-$C_{12}$-Alkyl; und

$R^{13}$ — wenn D für 0 steht auch ein Kation eines Metalls der I., II. oder VII. Gruppe des Periodensystems oder Ammonium; und

$X^2$ — einen Rest

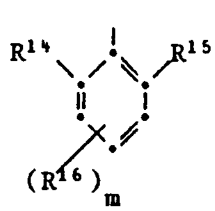

| | |
|---|---|
| m | die Zahlen 0, 1, 2 oder 3; und |
| $R^{14}$ und $R^{15}$ | unabhängig voneinander $C_1$-$C_4$-Alkyl; |
| $R^{16}$ | gleiche oder verschiedene Reste aus der Gruppe $C_1$-$C_4$-Alkyl, $C_1$-$C_4$-Alkoxy und Halogen; |
| $L^3$ | CN; $COOR^{17}$; oder $CONR^{18}R^{19}$; und |
| $R^{17}$ | unsubstituiertes oder durch Halogen substituiertes $C_1$-$C_7$-Alkyl, $C_3$-$C_7$-Alkenyl, $C_3$-$C_7$-Alkinyl, $C_1$-$C_7$-Alkoxy-$C_1$-$C_7$-alkyl, $C_3$-$C_7$-Cycloalkyl oder Aryl-$C_1$-$C_5$-alkyl; und |
| $R^{18}$ und $R^{19}$, | die gleich oder verschieden sein können, Waserstoff; $C_1$-$C_4$-Alkyl; $C_3$-$C_7$-Alkenyl; $C_3$-$C_7$-Alkinyl; $C_1$-$C_7$-Alkoxy-$C_1$-$C_7$-alkyl; oder |
| $R^{18}$ und $R^{19}$ | gemeinsam mit dem Stickstoffatom, an das sie gebunden sind, einen gesättigten oder ungesättigten drei- bis siebengliedrigen Ring, der bis zu drei Heteroatome aus der Gruppe O, N und S enthält und der unsubstituiert oder durch ($C_1$-$C_4$)-Alkyl oder Halogen substituiert ist und eine Carbonylgruppe enthalten kann; und |
| $X^3$ | einen Rest |

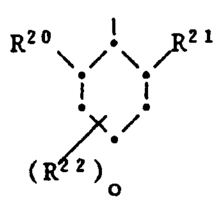

oder

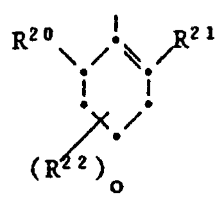

;

| | |
|---|---|
| o | 0 bis 6; |
| $R^{20}$ und $R^{21}$ | unabhängig voneinander Wasserstoff oder $C_1$-$C_4$-Alkyl; und |
| $R^{22}$ | jeweils unabhängig voneinander $C_1$-$C_4$-Alkyl; $C_1$-$C_4$-Alkoxy; $C_1$-$C_4$-Hydroxyalkyl; $C_1$-$C_4$-Alkoxy-$C_1$-$C_4$-alkyl; oder Halogen; und |
| $L^4$ | CO-$R^{23}$; oder CN; |
| $R^{23}$ | Hydroxy; $C_1$-$C_6$-Alkoxy; Hydroxy-($C_2$-$C_6$)-alkoxy; ($C_2$-$C_6$)-Alkoxyalkoxy; Amino; $C_1$-$C_6$-Alkylamino; Di-($C_1$-$C_6$)-Alkylamino; Di-($C_1$-$C_6$)-Alkylamino-($C_1$-$C_3$)-alkylamino; $C_1$-$C_3$-Alkoxyamino; Anilino; N-Pyrrolidino; N-Piperidino; N-Morpholino; Hydrazino; N'-($C_1$-$C_3$)-Alkylhydrazino; N,N'-Dimethylhydrazino; oder N'-Phenylhydrazino; und |
| $X^4$ | den Rest |

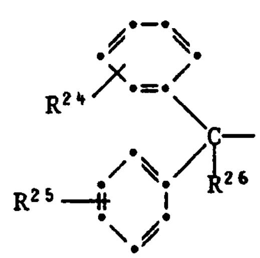

und

| | |
|---|---|
| $R^{24}$ und $R^{25}$ | die gleich oder verschiedene sein können Wasserstoff; Halogen; $C_1$-$C_3$-Alkyl; Trifluormethyl; Hydroxy; $C_1$-$C_3$-Alkoxy; Halogen-($C_1$-$C_3$)-alkoxy; $C_1$-$C_3$-Alkylthio; Cyano; Nitro; oder Acetamido; und |
| $R^{26}$ | Wasserstoff; oder Phenyl; |

bedeutet.

Es sind herbizid bzw. pflanzenwuchsregulatorisch wirksame Imidazol-5-carbonsäuren und Carbonsäurederivate bekannt geworden, die in der Position 1 des Imidazolsystems mit raumerfüllenden Resten substituiert sind.

So sind Verbindungen der Formel I, in denen X und L für $X^1$ und $L^1$ stehen in den Europäischen Patentanmeldungen EP-A-207 563, EP-A 240 050 und EP-A-234-656 beschrieben. Verbindungen der Formel I, in denen X und L $X^2$ und $L^2$ bedeuten, sind Gegenstand der EP-A-137 868, während die Reste $X^3$

und $L^3$ aus EP-A-199 206 und die Reste $X^4$ und $L^4$ aus EP-A-373 bekannt sind.

Bezüglich der Definitionsbreite der Reste X und L sowie der bevorzugten Ausführungsformen und der Einzelverbindungen der Formel I wird ausdrücklich auf die vorstehend genannten Publikationen Bezug genommen.

Den Verbindungen der Formel I ist gemeinsam, dass sie am Stickstoffatom in der Position 1 des Imidazolsystems einen raumerfüllenden Substituenten tragen, welcher aufgrund sterischer Wechselwirkungen die Synthese des Imidazolsystems nach an sich bekanntem Verfahren (ausgehend von acyclischen Vorstufen, durch Imidazol-Ringschlussreaktionen) erschwert. Sterische Wechselwirkungen beeinflusen ebenfalls die Synthese von Verbindungen der Formel I durch Substitutionsreaktionen ausgehend von der Position 1 unsubstituierten Imidazolcarbonsäuren und geeigneten Verbindungen der Formel X - Y, in denen Y für eine Abgangsgruppe steht.

Die aus dem Stand der Technik bekannten Verfahren zur Synthese der Imidazole sind - sofern die Synthese über einen Imidazol-Ringschluss verläuft - Varianten der Paal-Knorr Synthese von Imidazolen. D.h. es werden 1,4-Dicarbonylverbindungen in welchen die Substituenten X und L bereits an den entsprechenden Positionen der acyclischen Edukte gebunden sind, mit Ammoniak oder Säureamiden zu den Imidazolen der Formel I cyclisiert. In vielen Fällen ist die Synthese der dazu benötigten offenkettigen 1,4-Dicarbonylverbindungen aufwendig. Diese Reaktionsführung nimmt ausserdem in Kauf, dass mit dem Substituenten L ein weiteres reaktionsfähiges Zentrum im dem acyclischen Edukt vorliegt, welches seinerseits zu Reaktionen mit dem zum Ringschluss benötigten Ammoniak bzw. Amin befähigt ist.

In den zuvor genannten Offenlegungschriften ist noch ein weiteres Verfahren zur Herstellung der anmeldungsgemässen Imidazole beschrieben, welches ausgehend von Aminen der Formel $X\text{-}NH_2$ diese durch Umsetzung mit Chloressigsäureestern zunächst in die entsprechenden Glycinderivate überführt. Diese werden dann mit Ameisen säureestern zweifach formiliert (am Stickstoffatom und an der Methylengruppe des Glycinfragments) und danach mit Rhodanwasserstoffsäure cyclisiert. Auf diese Weise entstehen zunächst in der Position 5 durch L und in Position 1 durch X substituierte 2-Mercaptoimidazole aus denen die Mercaptogruppe durch katalytische Verfahren abgespalten wird. Dieses Verfahren ist insbesondere dann ungeeignet, wenn die Gruppe X ihrerseits bezüglich der Entschwefelung labile Gruppen enthält, wie etwa in den Fällen der Verbindungen der Formel I, in denen X für einen schwefelhaltigen Substituenten steht.

Dieses Verfahren ist auch in J.Amer.Chem.Soc. 71 (1949) 644-647 und 2444-2448 beschrieben.

Die aus dem Stand der Technik bekannten Synthesen der Verbindungen der Formel I vermögen somit nicht im Hinblick auf Durchführbarkeit, Verfügbarkeit der Zwischenprodukte, generelle Durchführbarkeit sowie Reinheit und Ausbeute der Endprodukte zu befriedigen.

Der Erfindung liegt die Aufgabe zugrunde, ein gegenüber dem Bekannten verbessertes Verfahren zur Verfügung zu stellen.

Erfindungsgemäss wird die Aufgabe gelöst durch ein Verfahren zur Herstellung von Imidazolen der Formel I

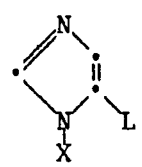

(I),

worin

| | |
|---|---|
| L und X | gemeinsam für $L^1$ und $X^1$; $L^2$ und $X^2$; $L^3$ und $X^3$; oder $L^4$ und $X^4$ stehen; und |
| $L^1$ | $COOR^1$; $CONR^2R^3$; $CONR^4NHR^3$ oder CN; |
| $R^1$ | Wasserstoff; $C_1$-$C_7$-Alkyl; $C_3$-$C_7$-Alkenyl; $C_3$-$C_7$-Alkinyl; $C_3$-$C_7$-Cycloalkyl; $C_1$-$C_7$-Alkoxy-$C_1$-$C_7$-alkyl; oder Aryl-$C_1$-$C_5$-alkyl; |
| | die Reste $R^2$, $R^3$ und $R^4$ unabhängig voneinander Wasserstoff; $C_1$-$C_5$-Alkyl; $C_3$-$C_5$-Alkenyl; $C_3$-$C_5$-Alkinyl; $C_3$-$C_7$-Cycloalkyl; Aryl; oder durch Aryl, $C_3$-$C_7$-Cycloalkyl, $C_3$-$C_7$-Cycloalkoxy, $C_1$-$C_5$-Alkoxy, Hydroxy, Carboxyl oder $C_1$-$C_5$-Alkyloxycarbonyl substituiertes $C_1$-$C_5$-Alkyl; oder |
| $R_2$ und $R_3$ | gemeinsam mit dem Stickstoffatom, an welches sie gebunden sind einen unsubstituierten oder mit 1 bis 3 $C_1$-$C_5$-Alkylgruppen substituierten Piperidinyl, Pyrrolidinyl, 2-Oxopiperidinyl, 2-Oxopyrrolidinyl, Morpholinyl, Thiomorpholinyl, Piperazinyl |

oder 4-($C_1$-$C_4$)-Alkylpiperazinylring;

$X^1$ 1-Indanyl, 1-Tetrahydronaphthalinyl, 5-Benzocycloheptanyl, 4-Tetrahydrobenzothienyl, 4-Tetrahydrobenzofuranyl, 5-Tetrahydrochinolinyl, 5-tetrahydroisochinolinyl, 8-Tetrahydrochinolinyl, 8-Tetrahydroisochinolinyl, 9,10-Dihydro-9-anthracenyl, 9H-Fluoren-9-yl, 5-Dibenzo[a,d]cycloheptenyl, 5-Dibenzo[a,d]cycloheptanyl oder 1-Dihydronaphthalinyl, welches unsubstituiert oder bis zu sechsfach gleich oder verschieden durch $C_1$-$C_5$-Alkyl, Aryl-$C_1$-$C_5$-alkyl, Di-Aryl-$C_1$-$C_5$-alkyl, $C_1$-$C_5$-Alkoxy, Halogen, $C_3$-$C_7$-Alkenyl, Amino, Nitro, $C_1$-$C_5$-Alkylcarbonylamino, Trifluormethyl oder Difluormethoxy substituiert ist oder in welchem zwei geminal gebundene Substituenten gemeinsam mit dem Kohlenstoffatom, an welches sie gebunden sind, eine $C_3$-$C_7$-Spirocycloalkylgruppe bilden oder in welchem zwei Substituenten gemeinsam für eine $C_1$-$C_5$-Alkylen- oder $C_5$-$C_7$-Cycloalkylengruppe stehen, wobei diese Alkylen- oder Cycloalkylengruppe ihrerseits gegebenenfalls bis zu zweifach unabhängig voneinander durch $C_1$-$C_5$-Alkyl, Aryl-$C_1$-$C_5$-alkyl, Di-Aryl-$C_1$-$C_5$-alkyl, $C_1$-$C_5$-Alkoxy, Halogen, $C_3$-$C_7$-Alkenyl, Trifluormethyl, Difluormethoxy oder Aryl substituiert sein kann; oder

$X^1$ die Gruppe

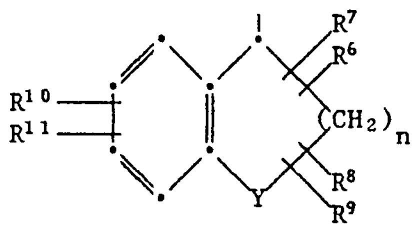

; oder

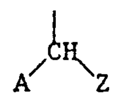

;

n null; eins; oder zwei;

Y die Gruppen $-CH_2S(O)_m-$, oder $-CH_2-N(R^{12})-$ in welchen das Heteroatom an das benzolische Ringkohlenstoffatom gebunden ist und worin m für 0, 1 oder 2 steht, bedeutet;

$R^6$, $R^7$, $R^8$ und $R^9$ unabhängig voneinander Wasserstoff; $C_1$-$C_5$-Alkyl; Aryl-$C_1$-$C_5$-alkyl; Di-Aryl-$C_1$-$C_5$-alkyl; $C_1$-$C_5$-Alkoxy; Halogen; $C_3$-$C_7$-Alkenyl; Trifluormethyl; Difluormethoxy; oder Aryl; oder

$R^6$ und $R^7$ gemeinsam eine annellierten Benzolrest, der gegebenenfalls bis zu zweifach durch $C_1$-$C_5$-Alkyl, $C_1$-$C_5$-Alkoxy, Halogen, ein- bis dreifach halogensubstituiertes $C_1$-$C_5$-Alkyl, ein- bis dreifach halogensubstituiertes $C_1$-$C_5$-Alkoxy, Nitro, Amino oder -NH-CO-M substituiert sein kann; oder

$R^6$ und $R^7$ zusammen mit dem Kohlenstoffatom, an welches sie geminal gebunden sind, einen spirocyclischen $C_3$-$C_7$-Ring; oder

$R^6$ und $R^7$ zusammen eine $C_1$-$C_5$-Alkylen- oder $C_5$-$C_7$-Cycloalkylengruppe, die gegebenenfalls bis zu zweifach unabhängig voneinander durch $C_1$-$C_5$-Alkyl, Aryl-$C_1$-$C_5$-alkyl, $C_1$-$C_5$-Alkoxy, Halogen, $C_3$-$C_7$-Alkenyl, Trifluormethyl, Difluormethoxy oder Aryl substituiert sein kann; und

$R^{10}$ und $R^{11}$ unabhängig voneinander Wasserstoff; $C_1$-$C_5$-Alkyl; $C_1$-$C_5$-Alkoxy; Halogen; Trifluormethyl; Difluormethoxy; Cyano; Nitro; Amino; Mono-$C_1$-$C_5$-alkylamino; Di-$C_1$-$C_5$-alkylamino; oder -NH-CO-M; und

$R^{12}$ Wasserstoff; $C_1$-$C_5$-Alkyl; $C_1$-$C_5$-Alkanoyl; oder 4-Methylphenylsulfonyl; und

A Wasserstoff; gegebenenfalls bis zu zweifach durch $C_1$-$C_5$-Alkyl substituiertes $C_3$-$C_7$-Cycloalkyl; gegebenenfalls durch $C_1$-$C_7$-Alkyloxy oder Aryl substituiertes $C_1$-$C_7$-Alkyl; durch ein $C_1$-$C_7$-Alkoxy und einen Arylrest substituiertes $C_1$-$C_7$-Alkyl; oder ein unsubstituiertes oder bis zu zweifach unabhängig voneinander durch $C_1$-$C_5$-Alkyl, $C_1$-$C_5$-Alkoxy, Halogen, Nitro, Amino, Mono-$C_1$-$C_5$-Alkylamino, Di-$C_1$-$C_5$-Alkylamino, -NH-CO-M, Cyano, Trifluormethyl oder Difluormethoxy substituiertes Pyridinyl, Pyrimidinyl, Naphthalinyl, Furanyl oder Thiophenyl;

wobei im Rahmen der Definition von A Aryl für Phenyl, Pyridinyl, Pyrimidinyl, Naphthalinyl, Furanyl oder Thiophenyl steht und in der dieser Rest bis zu

zweifach, im Falle von Aryl = Phenyl auch bis zu dreifach, unabhängig voneinander durch $C_1$-$C_5$-Alkyl, $C_1$-$C_5$-Alkoxy, Halogen, Nitro, Amino, Mono-$C_1$-$C_5$-alkylamino, Di-$C_1$-$C_5$-alkylamino, -NH-CO-M, Cyano, Trifluormethyl oder Difluormethoxy substituiert sein kann; und

Z unsubstituiertes oder bis zu dreifach unabhängig voneinander durch $C_1$-$C_5$-Alkyl, $C_1$-$C_5$-Alkoxy, Halogen, Cyano, Nitro, Amino, Mono-$C_1$-$C_5$-alkylamino, Di-$C_1$-$C_5$-alkylamino, -NH-CO-M, Trifluormethyl oder Difluormethoxy substituiertes Naphthalinyl, Thiophenyl, Furanyl, Pyrimidinyl, Phenyl oder Pyridinyl; und

M $C_1$-$C_5$-Alkyl; und

Aryl im Rahmen vorstehender Definitionen der Reste X und L ausserdem einen unsubstituierten oder bis zu dreifach gleich oder verschieden durch $C_1$-$C_5$-Alkyl, $C_1$-$C_5$-Alkyloxy oder Halogen substituierten Phenylrest; und

$L^2$ CO-D-$R^{13}$;

D O; oder $NR^{13}$;

$R^{13}$ Wasserstoff; Phenyl; $C_3$-$C_6$-Alkenyl; unsubstituiertes oder bis zu dreifach gleich oder unterschiedlich durch $C_1$-$C_6$-Alkoxy, $C_1$-$C_3$-Dialkylamino oder Halogen substituiertes $C_1$-$C_{12}$-Alkyl; und $R^{13}$ wenn D für 0 steht auch ein Kation eines Metalls der I., II. oder VII. Gruppe des Periodensystems oder Ammonium; und

$X^2$ einen Rest

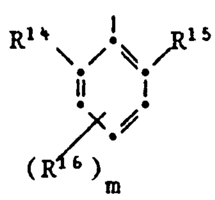

m die Zahlen 0, 1, 2 oder 3; und

$R^{14}$ und $R^{15}$ unabhängig voneinander $C_1$-$C_4$-Alkyl;

$R^{16}$ gleiche oder verschiedene Reste aus der Gruppe $C_1$-$C_4$-Alkyl, $C_1$-$C_4$-Alkoxy und Halogen;

$L^3$ CN; $COOR^{17}$; oder $CONR^{18}R^{19}$; und

$R^{17}$ unsubstituiertes oder durch Halogen substituiertes $C_1$-$C_7$-Alkyl, $C_3$-$C_7$-Alkenyl, $C_3$-$C_7$-Alkinyl, $C_1$-$C_7$-Alkoxy-$C_1$-$C_7$-alkyl, $C_3$-$C_7$-Cycloalkyl oder Aryl-$C_1$-$C_5$-alkyl; und

$R^{18}$ und $R^{19}$, die gleich oder verschieden sein können, Waserstoff; $C_1$-$C_4$-Alkyl; $C_3$-$C_7$-Alkenyl; $C_3$-$C_7$-Alkinyl; $C_1$-$C_7$-Alkoxy-$C_1$-$C_7$-alkyl; oder

$R^{18}$ und $R^{19}$ gemeinsam mit dem Stickstoffatom, an das sie gebunden sind, einen gesättigten oder ungesättigten drei- bis siebengliedrigen Ring, der bis zu drei Heteroatome aus der Gruppe O, N und S enthält und der unsubstituiert oder durch ($C_1$-$C_4$)-Alkyl oder Halogen substituiert ist und eine Carbonylgruppe enthalten kann; und

$X^3$ einen Rest

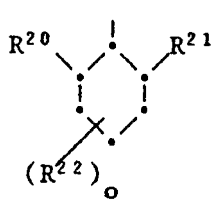

oder

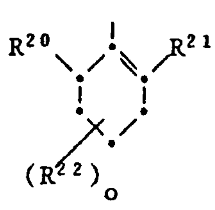

;

o 0 bis 6;

$R^{20}$ und $R^{21}$ unabhängig voneinander Wasserstoff oder $C_1$-$C_4$-Alkyl; und

$R^{22}$ jeweils unabhängig voneinander $C_1$-$C_4$-Alkyl; $C_1$-$C_4$-Alkoxy; $C_1$-$C_4$-Hydroxyalkyl; $C_1$-$C_4$-Alkoxy-$C_1$-$C_4$-alkyl; oder Halogen; und

$L^4$ CO-$R^{23}$; oder CN;

$R^{23}$ Hydroxy; $C_1$-$C_6$-Alkoxy; Hydroxy-($C_2$-$C_6$)-alkoxy; ($C_2$-$C_6$)-Alkoxyalkoxy; Amino; $C_1$-

$C_6$-Alkylamino; Di-($C_1$-$C_6$)-Alkylamino; Di-($C_1$-$C_6$)-Alkylamino-($C_1$-$C_3$)-alkylamino; $C_1$-$C_3$-Alkoxyamino; Anilino; N-Pyrrolidino; N-Piperidino; N-Morpholino; Hydrazino; N'-($C_1$-$C_3$)-Alkylhydrazino; N,N'-Dimethylhydrazino; oder N'-Phenylhydrazino; und

$X^4$ den Rest

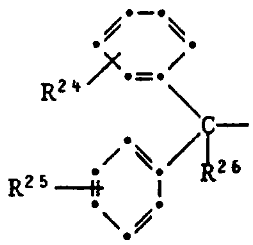

und

$R^{24}$ und $R^{25}$ die gleich oder verschiedene sein können Wasserstoff; Halogen; $C_1$-$C_3$-Alkyl; Trifluormethyl; Hydroxy; $C_1$-$C_3$-Alkoxy; Halogen-($C_1$-$C_3$)-alkoxy; $C_1$-$C_3$-Alkylthio; Cyano; Nitro; oder Acetamido; und

$R^{26}$ Wasserstoff; oder Phenyl;

bedeutet, dadurch gekennzeichnet, dass man N-Cyanoformamidine der Formel II

$$X - NH - CH = N - CN \quad (II),$$

worin X wie zuvor definiert ist, mit einer Verbindung der Formel III

$$Z - CH_2 - L \quad (III),$$

worin L wie zuvor definiert ist und Z für eine nucleofuge Gruppe steht zu einer Verbindung der Formel IV

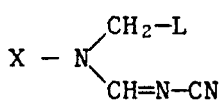

(IV)

N-alkyliert und IV unter Baseneinwirkung zu einem 4-Aminoimidazol der Formel V

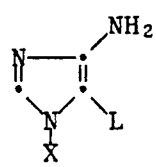

(V)

cyclisiert und das Aminoimidazol V zu einem Imidazol der Formel I reduziert.

Die 4-Aminoimidazole der Formel V werden vorzugsweise in einem zweistufigen Verfahren reduziert, indem man ein 4-Aminoimidazol der Formel V

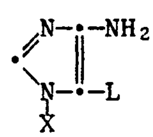

(V),

in dem X und L wie zuvor definiert sind in ein Diazoniumsalz der Formel VI

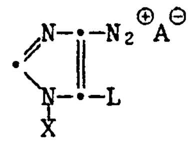

(VI),

in dem A für ein Anion steht, überführt und unter Stickstoffabspaltung zu Imidazolen der Formel I umsetzt. Diese Verfahren und auch die neuen Diazoniumsalze der Formel VI sind ebenfalls Gegenstand der Erfindung.

In der hier verwendeten Definition umfassen die angegebenen generischen Begriffe beispielsweise die folgenden spezifischen Einzelsubstituenten, wobei diese Aufzählung keine Einschränkung der Erfindung darstellt.

Alkyl umfasst die konstitutionsisomeren Alkyle im Umfang der jeweils angegebenen Zahl von Kohlenstoffatomen, wie Methyl, Ethyl, n-Propyl, iso-Propyl, n-Butyl, sek-Butyl, iso-Butyl, tert-Butyl, die isomeren Pentyle, wie etwa tert-Pentyl (1,1-dimethylpropyl), iso-Pentyl (1-ethylpropyl) usw, sowie der isomeren Hexyl- und Heptylgruppen.

Halogen ist Fluor, Chlor, Brom und Jod, vorzugsweise Fluor, Chlor und Brom, besonders Fluor und Chlor.

Mit Halogenalkyl sind ganz oder teilweise mit gleichen oder unterschiedlichen Halogenen substituierte Alkyl gemäss die jeweiligen Defintionsbreite gemeint, wie etwa 2,2,2-Trifluorethyl, Trifluormethyl, Difluormethyl, Chlordifluormethyl, Fluorchlormethyl, Dichlormethyl, Chlormethyl, Trichlormethyl u.s.w.

Alkenyl umfasst die Konstitutionsisomeren wie auch die cis-trans-Isomeren im Umfang der jeweils angegebenen Zahl von Kohlenstoffatomen, wie die Propenyl-, Butenyl-, Pentenyl-, Hexenyl- und Heptenylreste.

Alkinyl umfasst die konstitutionsisomeren Verbindungen ebenfalls im Umfang der jeweils angegebenen Zahl von Kohlenstoffatomen, wie etwa die Propinyl-, Butinyl-, Pentinyl, Hexinyl- und Heptinylreste.

Sofern Alkenyl- oder Alkinylreste an ein zu einer Ester- oder Amidgruppe gehörendes Sauerstoff- oder Stickstoffatom gebunden sind, sind diese Reste vorzugsweise über ein gesättigtes Kohlenstoffatom an das Heteroatom gebunden.

$C_3$-$C_7$-Cycloalkyl umfasst Cyclopropyl-, Cyclobutyl, Cyclopentyl, Cyclohexyl und Cycloheptyl.

Halogenalkoxy umfasst im Rahmen der jeweiligen Defintionsbreite vorzugsweise durch Fluor, Chlor und Brom substituierte Reste, wie etwa Trifluormethoxy, Trichlormethoxy, Dibrommethoxy, Difluorchlormethoxy, Dichlormethoxy, 2,2,1-Trichlorethoxy, 2,2,1-Trifluorethoxy usw.

In den weiteren Substituenten, welche aus mehreren Grundelementen zusammengesetzt sind, können die Teilelemente innerhalb der Definitionsbreite frei gewählt werden und unter anderem obige Bedeutung haben.

Die erfindungsgemässen Verbindungen können auch als optische Isomere vorliegen, welche ebenfalls Gegenstand dieser Erfindung sind.

In den Fällen, in denen der Substituent X durch ein unsymmetrisch substituiertes Kohlenstoffatom an den erfindungsgemässen Imidazolrest gebunden ist, sind sowohl die R- als auch die S-konfigurierten Verbindungen der Formel I mit umfasst.

Darüber hinaus können die anmeldungsgemässen Moleküle der Formel I noch weitere Asymmetriezentren aufweisen, welche in ihren einzelnen Interkombinationen ebenfalls mit umfasst sind. Die Erfindung umfasst somit sämtliche aus Formel I herleitbare Diastereomere und Enantiomere.

Insbesondere kann der in Nachbarstellung zu dem das Imidazolsystem tragenden Kohlenstoffatom stehende $\alpha$-Kohlenstoffatom oder das dazu benachbarte $\beta$-Kohlenstoffatom jeweils einfach substituiert sein, wobei diese Substituenten dann cis oder trans zueinander oder zu dem Imidazolsystem stehen können. Auch diese cis-, trans-Isomeren sind mit umfasst.

Insgesamt ist festzustellen, dass das erfindungsgemässe Verfahren zu Synthese chiraler Systeme der Formel I (sowie der dazugehörigen Zwischenprodukte II, IV, V und VI) bestens geeignet ist, da der Aufbau des Imidazolsystems ohne Konfigurationsänderung in den Molekülteilen X und L durchgeführt wird. Somit können aus den optisch aktiven Edukten der Formel VIII und/oder III die jeweiligen optisch aktiven Endprodukte der Formel I hergestellt werden, ohne dass im Verlauf der Reaktion die aus den Edukten herrührende optische Aktivität verlorengeht.

Die Definitionen der Endverbindungen der Formel I ist den europäischen Patentanmeldungen EP-A-373, EP-A-207 563, EP-A-240 050, EP-A-234 656, EP-A-137 868 und EP-A-199 206 noch weiter ausgeführt, worauf hier Bezug genommen wird.

Die Verbindungen der Formeln II, IV, V und VI sind neu. Sie stellen wertvolle Zwischenverbindungen für die Synthese der herbizid wirksamen Endverbindungen der Formel I dar. Die Erfindung betrifft somit auch die neuen Stoffe der Formel II, IV, V und VI sowie Verfahren zu deren Herstellung und deren Verwendung zur Herstellung der jeweiligen Folgeprodukte gemäss nachstehendem Formelschma:

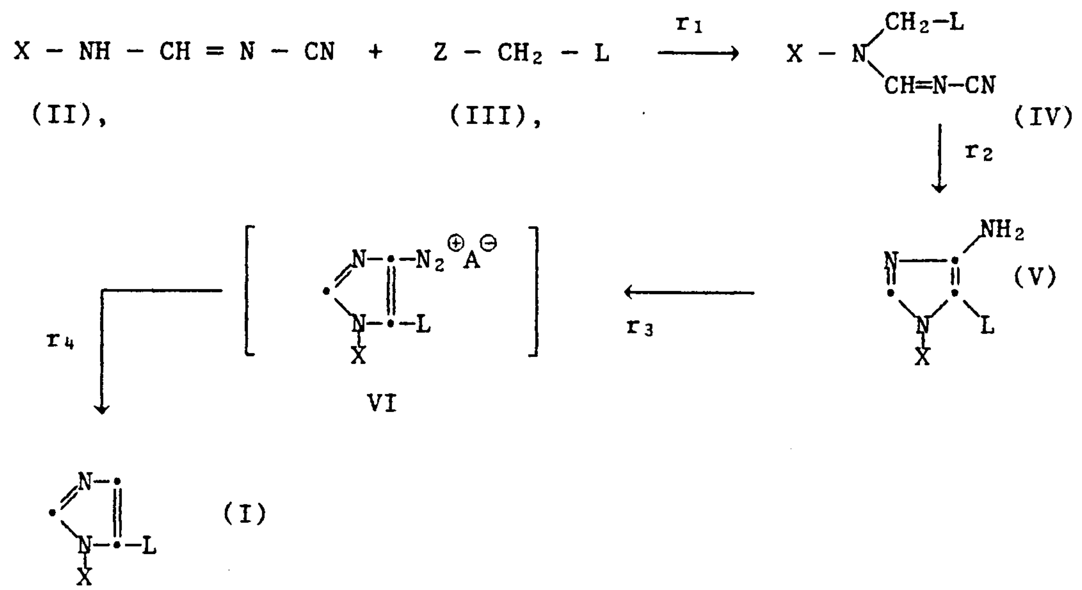

(II), (III), (IV), (V), VI, (I)

Die Alkylierungsreaktion $r_1$ wird vorzugsweise in zwei Schritten vollzogen, indem zunächst das N-Cyanoformamidin mittels einer Base zu einem Salz der Formel VII deprotoniert und dann mit einer Verbindung der Formel III gemäss nachstehender Reaktionsgleichung am Stickstoff alkyliert wird:

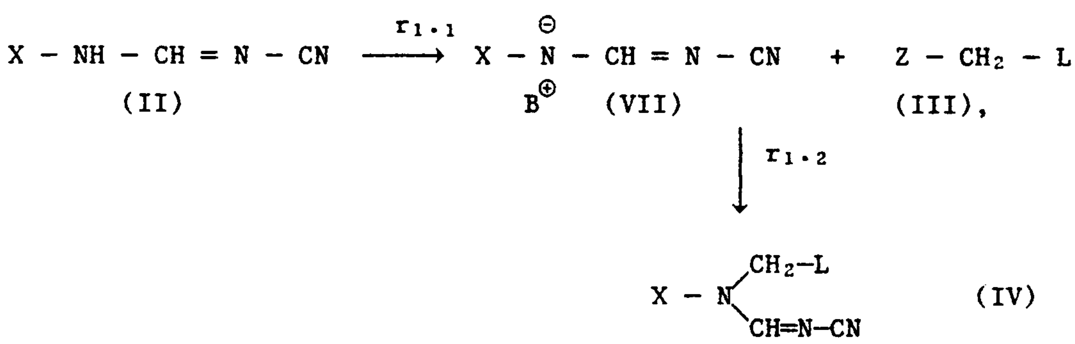

(II), (VII), (III), (IV)

Die Reaktionsschritte $r_{1.1}$ und $r_{1.2}$ können nacheinander, in zwei getrennten Stufen oder gleichzeitig als "Eintopfverfahren" durchgeführt werden.

Das erfindungsgemässe Verfahren kann vorzugsweise zur Herstellung derjenigen Verbindungen der Formel I angewendet werden, die in EP-A-373, EP-A-207 563, EP-A-240 050, EP-A-234 656, EP-A-137 868 und EP-A-100 206 als bevorzugt oder besonders bevorzugt hervorgehoben oder als Einzelverbindungen genannt sind.

Weiterhin ist das erfindungsgemässe Verfahren bevorzugt bei Verbindungen der Formel I

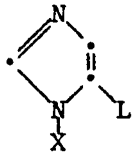

(I),

worin

| | |
|---|---|
| L | COOR[1]; CONR[2]R[3]; oder CN; und |
| $R^1$ | Wasserstoff; $C_1$-$C_7$-Alkyl; $C_3$-$C_7$-Alkenyl; $C_3$-$C_7$-Alkinyl; $C_3$-$C_7$-Cycloalkyl; $C_1$-$C_7$-Alkoxy-$C_1$-$C_7$-alkyl; oder Aryl-$C_1$-$C_5$-alkyl; |
| | die Reste $R^2$, $R^3$ und $R^4$ unabhängig voneinander Wasserstoff; $C_1$-$C_5$-Alkyl; $C_3$-$C_5$-Alkenyl; $C_3$-$C_5$-Alkinyl; $C_3$-$C_7$-cycloalkyl; Aryl; oder durch Aryl, $C_3$-$C_7$-Cycloalkyl, $C_3$-$C_7$-Cycloalkoxy, $C_1$-$C_5$-Alkoxy, Hydroxy, Carboxyl oder $C_1$-$C_5$-Alkyloxycarbonyl substituiertes $C_1$-$C_5$-Alkyl; oder |
| $R_2$ und $R_3$ | gemeinsam mit dem Stickstoffatom, an welches sie gebunden sind einen unsubstituierten oder mit 1 bis 3 $C_1$-$C_5$-Alkylgruppen substituierten Piperidinyl, Pyrrolidinyl, 2-Oxopiperidinyl, 2-Oxopyrrolidinyl, Morpholinyl, Thiomorpholinyl, Piperazinyl oder 4-($C_1$-$C_4$)-Alkylpiperazinylring; und |
| X | 1,2,3,4-Tetrahydronaphthalin-1-yl, Indan-1-yl, Phenyl, Diphenylmethyl, Benzocycloheptan-5-yl, 4,5,6,7-Tetrahydrobenzo[b]thiophen-4-yl, 4,5,6,7-Tetrahydrobenzo[b]furan-4-yl, 5,6,7,8-Tetrahydrochinolin-5-yl, 5,6,7,8-Tetrahydroisochinolin-5-yl, 5,6,7,8-Tetrahydrochinolin-8-yl, 5,6,7,8-Tetrahydroisochinolin-8-yl, 9,10-Dihydroanthracen-9-yl, 9H-Fluoren-9-yl, Dibenzo[a,d]cyclohepten-5-yl, Dibenzo[a,d]cycloheptan-5-yl, 1,2-Dihydronaphthalin-1-yl, Chroman-4-yl oder Thiochroman-4-yl, welches unsubstituiert oder bis zu dreifach durch $C_1$-$C_4$-Alkyl oder Halogen substituiert ist oder in welchem zwei geminal gebundene Substituenten gemeinsam mit dem Kohlenstoffatom, an welchem sie gebunden sind, eine $C_3$-$C_6$-Spiroalkylgruppe bilden; |

bedeutet.

Weiterhin bevorzugt sind Verbindungen der Formel I

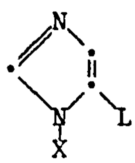

(I),

worin

| | |
|---|---|
| X | wie zuvor definiert ist und |
| L | COOR[1]; CN; oder CNR[2]R[3]; |
| $R^1$ | $C_1$-$C_4$-Alkyl; $C_3$-$C_6$-Cycloalkyl; oder $C_3$-$C_6$-Alkenyl; und |
| $R^2$ und $R^3$ | unabhängig voneinander Wasserstoff oder $C_1$-$C_4$-Alkyl |

bedeutet.

Besonders bevorzugt ist das erfindungsgemässe Verfahren zur Herstellung von Verbindungen der Formel I

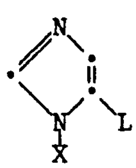

(I),

worin

L COOR[1]; CN; oder $CNR^2R^3$; und

$R^1$ $C_1$-$C_4$-Alkyl; $C_3$-$C_6$-Alkenyl; oder $C_3$-$C_6$-Cycloalkyl;

$R^2$ und $R^3$ unabhängig voneinander Wasserstoff oder $C_1$-$C_4$-Alkyl und

X 2,2-Dimethyl-1,2,3,4-tetrahydronaphthalin-1-yl, 2,2-Dimethyl-indan-1-yl, 2,6-Dimethylphenyl, Diphenylmethyl, Indan-1-yl, 2-Methyl-indan-1-yl, cis-2-Methyl-indan-1-yl, trans-2-Methyl-indan-1-yl, 1,2,3,4-Tetrahydronaphthalin-1-yl, 2-Methyl-1,2,3,4-tetrahydronaphthalin-1-yl, cis-2-Methyl-1,2,3,4-tetrahydronaphthalin-1-yl, trans-2-Methyl-1,2,3,4-tetrahydronaphthalin-1-yl, Benzocycloheptan-5-yl, 4,5,6,7-Tetrahydrobenzo[b]thiophen-4-yl, 4,5,6,7-Tetrahydrobenzo[b]furan-4-yl, 5,6,7,8-Tetrahydrochinolin-5-yl, 5,6,7,8-Tetrahydroisochinolin-5-yl, 5,6,7,8-Tetrahydrochinolin-8-yl, 5,6,7,8-Tetrahydroisochinolin-8-yl, 9,10-Dihydroanthracen-9-yl, 9H-Fluoren-9-yl, dibenzo[a,d]cyclohepten-5-yl, Dibenzo-[a,d]cycloheptan-5-yl, 1,2-Dihydronaphthalin-1-yl, (4-chlorphenyl)-(phenyl)-methyl, 2-Ethyl-6-methylphenyl, 2,6-diethyl-phenyl, 2,6-Di-isopropyl-phenyl, 2,4,6-Trimethyl, phenyl, 4-Chlor-2,6-dimethyl-phenyl, 2-Ethyl-indan-1-yl, cis-2-Ethyl-indan-1-yl, trans-2-Ethyl-indan-1-yl, 2-Propyl-indan-1-yl, cis-Propyl-indan-1-yl, trans-Propyl-indan-1-yl, 2-Isopropyl-indan-1-yl, 2-Ethyl-2-methyl-indan-1-yl, 2-Methyl-2-propyl-indan-1-yl, 2,2-Diethyl-indan-1-yl, 2,2-Dipropyl-indan-1-yl, Spiro-[cyclopropan-1,2′-indan]-1′-yl, Spiro-[cyclopentan-1,2′-indan]-1′-yl, Spiro-[cyclohexan-1,2′-indan]-1′-yl, 2-Ethyl-1,2,3,4-tetrahydronaphthalin-1-yl, cis-2-Ethyl-1,2,3,4-tetrahydronaphthalin-1-yl, trans-2-Ethyl-1,2,3,4-tetrahydronaphthalin-1-yl, 2-Propyl-1,2,3,4-tetrahydronaphthalin-1-yl, cis-2-Propyl-1,2,3,4-tetrahydronaphthalin-1-yl, trans-2-Propyl-1,2,3,4-tetrahydronaphthalin-1-yl, 2-Ethyl-2-methyl-1,2,3,4-tetrahydronaphthalin-1-yl, 2-Methyl-2-propyl-1,2,3,4-tetrahydronaphthalin-1-yl, 2,2-Diethyl-1,2,3,4-tetrahydronaphthalin-1-yl, 2,2-Dipropyl-1,2,3,4-tetrahydronaphthalin-1-yl, Spiro-[cyclopropan-1,2′-(1′,2′,3′,4′-tetrahydro-naphthalin)]-1′-yl, Spiro[cyclopentan-1,2′-(1′,2′,3′,4′-tetrahydronaphthalin)]-1′-yl, Spiro[cyclohexan-1,2′-(1′,2′,3,′4′-tetrahydro-naphthalin)]-1′-yl, Chroman-4-yl, 3-Methylchroman-4-yl, cis-3-Methylchroman-4-yl, trans-3-Methylchroman-4-yl, 3-Ethylchroman-4-yl, cis-3-Ethylchroman-4-yl, trans-3-Ethylchroman-4-yl, 3-Propylchroman-4-yl, cis-3-Propylchroman-4-yl, trans-3-Propylchroman-4-yl, 3-Ethyl-3-methyl-chroman-4-yl, 3-Methyl-3-propyl-chroman-4-yl, 3-Isopropylchroman-4-yl, cis 3-Isopropylchroman-4-yl, trans-3-Isopropylchroman-4-yl, 3,3-Dimethylchroman-4-yl, 3,3-Dimethylchroman-4-yl, 3,3-Dipropylchroman-4-yl, Spiro[cyclopropan-1,3′-chroman]-4′-yl, Spiro[cyclopentan-1,3′-chroman]-4′yl, Spiro[cyclohexan-1,3′-chroman]-4′-yl, 2-Methylchroman-4-yl, cis-2-Methylchroman-4-yl, trans-2-Methylchroman-4-yl, 2-Ethylchroman-4-yl, cis-2-Ethylchroman-4-yl, trans-2-Ethylchroman-4-yl, 2-Propylchroman-4-yl, cis-2-Propylchroman-4-yl, trans-2-Propylchroman-4-yl, 2-Ethyl-2-methylchroman-4-yl, 2-Methyl-2-propyl-chroman-4-yl, 2-Isopropylchroman-4-yl, cis 2-Isopropylchroman-4-yl, trans-2-Isopropylchroman-4-yl, 2,2-Dimethylchroman-4-yl, 2,2-Diethylchroman-4-yl, 2,2-Dipropylchroman-4-yl, Spiro[cyclopropan-1,2′-chroman]-4′-yl, Spiro[cyclopentan-1,2′-chroman]4′-yl, Spiro-[cyclohexan-1,2′-chroman]-4′-yl, 2,2-Dimethyl-1,2,3,4-tetrahydronaphthalin-1-yl, Thiochroman-4-yl, 2,2-Dimethylthiochroman-4-yl, 3,3-Dimethylthiochroman-4-yl, 2,3-Dimethylthiochroman-4-yl, [cis-2,3-Dimethyl]thio-chroman-4-yl, [trans-2,3-Dimethyl]thio-chroman-4-yl, 2-Methylthiochroman-4-yl, 2-Ethylthiochroman-4-yl, 2-Methyl-2-ethyl-thiochroman-4-yl, 2,2-Diethyl-thiochroman-4-yl, 3,3-Diethyl-thiochroman-4-yl, 3-Methyl-3-ethyl-thiochroman-4 yl, 2,3-Dimethylindan-1-yl, [cis-2,3-Dimethyl]indan-1-yl, [trans-2,3-Dimethyl]indan-1-yl, (+)-2,2-Dimethylindan-1-yl, (-)-2,2-Dimethylindan-1-yl, (+)-2,2-Dimethyl-1,2,3,4-tetrahydronaphthalin-1-yl, (-)-2,2-Dimethyl-1,2,3,4-tetrahydronaphthalin-1-yl,

bedeutet.

Die Erfindung betrifft auch die neuen Zwischenprodukte der Formel II, VII, IV, V und VI sowie Verfahren zu deren Herstellung und deren Verwendung zur Herstellung von Verbindungen der Formel I im Umfang der als bevorzugt bzw. besonders bevorzugt genannten Endprodukte der Formel I.

Im folgenden soll das erfindungsgemässe Verfahren näher beschrieben werden, ohne dass die hier gemachten Ausführungen eine Beschränkung auf die genannten speziellen Mittel und Beispiele darstellen.

Die N-Cyanoformamidine der Formel II können in einfacher Weise analog zu literaturbekannten Verfahren etwa durch Kondensation von Aminen (VIII) mit Cyanamid (IX) und Orthoameisensäureestern der Formel X hergestellt werden.

$$X - NH_2 \quad + \quad CH(OD)_3 \quad + \quad H_2N\text{-}CN \longrightarrow X - NH - CH = N - CN$$

(VIII) (X) (IX) (II)

(Lit: H. Schäfer und R. Gewald; J. prakt. Chem. 318 (1976), 347-349). In obiger Gleichung steht D vorzugsweise für eine $C_1$-$C_4$-Alkylgruppe.

Diese Kondensationsreaktion kann in einem reaktionsinerten Lösungsmittel oder ohne Lösungsmittel vorzugsweise in einem Ueberschuss des Orthoesters X durchgeführt werden.

Die Verbindungen der Formel II sind neu. Die Erfindung betrifft somit auch ein Verfahren zur Herstellung von Verbindungen der Formel II

X - NH - CH = N - CN (II)

worin X wie zuvor definiert ist, dadurch gekennzeichnet, dass man

a) ein Amin der Formel VIII

$X - NH_2$ (VIII)

mit einem Orthoester der Formel X

$CH(OD)_3$ (X)

worin D vorzugsweise für $C_1$-$C_4$-Alkyl steht und Cyanamid der Formel IX

$H_2NCN$ (IX)

kondensiert, oder

b) ein Amin der Formel VIII

$X - NH_2$ (VIII)

mit einem Imidoester der Formel XI

DO - CH = N - CN (XI),

in dem D für eine $C_1$-$C_4$-Alkylgruppe oder für Phenyl steht, kondensiert oder

c) einen Imidoester der Formel XII

X - N = CH - OD (XII),

in dem D für eine $C_1$-$C_4$-Alkylgruppe oder für Phenyl steht, mit Cyanamid der Formel IX

$H_2NCN$ (IX)

kondensiert.

Die Reaktion wird vorzugsweise bei Temperaturen zwischen Raumtemperatur und der Siedetemperatur des Reaktionsgemisches bei Normaldruck durchgeführt. Als bevorzugt ist der Temperaturbereich von 20-200 °C, insbesondere von 70-150 °C anzusehen. Besonders vorteilhaft arbeitet man unter gleichzeitigem Abdestillieren des bei der Reaktion freiwerdenden Alkohols. Durch Zusatz katalytischer Mengen an Mineralsäure oder organischer Säuren kann die Reaktion ebenfalls beschleunigt werden.

Die als Ausgangsverbindung benötigten Amine der Formel VIII sind entweder bekannt oder können nach an sich bekanntem Verfahren hergestellt werden.

Wenn die Aminogruppe in VIII an ein gesättigtes Kohlenstoffatom gebunden ist, können die Verbindungen etwa durch Reduktion des entsprechenden Oxims (etwa analog zu Levine J. Org. Chem. 9 [1944] 380; Woods et al., J. Org. Chem. 19 (1954) 1290; Kipling, J. chem. Soc. 75 152) oder durch Reduktion der entsprechenden Nitroverbindung (Tranverso., Ann. Chimica 45 (1955) 706) hergestellt werden. Des weiteren

sind beispielsweise die partiell hydrierten Ringsysteme in den Aminen der Formel VIII durch partielle Reduktion der ungesättigten Ringsysteme zugänglich.

Die Alkylierung der N-Cyanoformamidine II mit III (Reaktion $r_1$) wird vorzugsweise in Gegenwart geeigneter Basen durchgeführt, wobei in einem ersten Reaktionsschritt die Base des N-Cyanoformamidins II zu einem Salz der Formel VII deprotoniert, welches

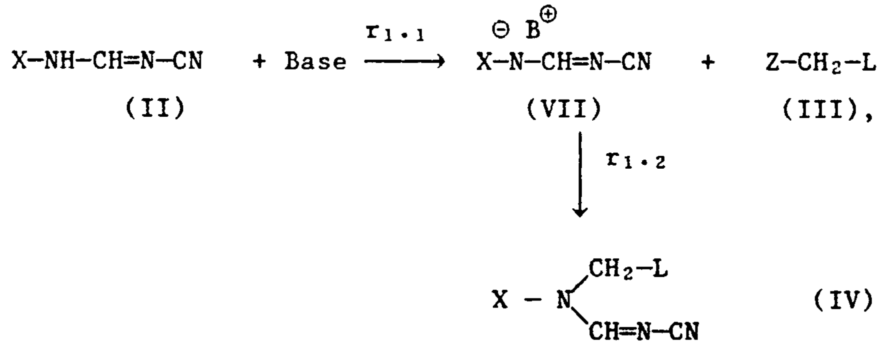


(II) (VII) (III), (IV)

danach mit einer Verbindung der Formel III zu IV alkyliert wird. Das Verfahren kann in zwei Stufen, d.h. zunächst Bildung des Salzes und anschliessende Zugabe von III oder auch ein "Eintopfverfahren", d.h. Salzbildung in Gegenwart von III durchgeführt werden.

In Formel VII hat der Substituent X die zuvor genannte Bedeutung, während $B^{\oplus}$ für ein Aequivalent eines Kations, vorzugsweise für ein Alkali; Erdalkali; Ammonium- oder organisches Ammoniumion, steht.

Die Reaktion kann bei Temperaturen zwischen 0 °C und der Siedetemperatur des Reaktionsgemisches in einem reaktionsinerten Lösungsmittel durchgeführt werden, vorzugsweise wird bei Temperaturen zwischen 0 °C und 50 °C insbesondere bei Raumtemperatur gearbeitet. Für die Reaktion geeignete Basen sind:

Alkali- und Erdalkalialkoholate, wie z.B. Kalium- oder Natriummethanolat, -ethanolat oder -tert.butylat; anorganische Basen, wie NaOH, KOH, Natriumcarbonat etc.; Alkali- bzw. Erdalkalihydride, wie Natriumhydrid; Alkaliamide, wie Natriumamid; stickstoffhaltige organische Basen, wie DABCO (Diazabicyclooctan) etc.

Vorteilhaft kann die Reaktion auch unter Phasentransferbedingungen in einem zweiphasigen System durchgeführt werden. Geeignete Systeme von Basen, Lösungsmitteln und Phasentransferkatalysatoren sind literaturbekannt (z.B. Dehmlow und Dehmlow, "Phase Transfer Catalysis", Verlag Chemie Weinheim, 1983; W.E. Keller "Phase Transfer Reactions", G. Thieme Verlag Stuttgart Vol. 1 1986 und Vol. 2 1987).

Zur Durchführung der Reaktion $r_1$ wird vorzugsweise eine äquimolare Menge an Base verwendet. Sofern mit einer mehr als äquimolaren Menge an Base gearbeitet wird, kann man die Verbindung der Formel IV gemäss Reaktionsschritt $r_2$ direkt zu V cycliseren. Wenn eine Isolierung des Produktes IV gewünscht wird, sind diejenigen Basen geeignet, deren Basenstärke zur Deprotonierung von II jedoch nicht zur Cyclisierung von V ausreichend ist. Wenn als Reaktionsprodukt gleich ein Aminoimidazol der Formel V gewünscht wird, kann dementsprechend eine Base verwendet werden, deren Basenstärke zur Deprotonierung von IV ausreicht. Es ist dann auch eine mindestens äquimolare Menge an Base (bezogen auf II) zu verwenden. Des weiteren wird die Cyclisierungsreaktion $r_2$ vorzugsweise bei erhöhten Temperaturen (vorzugsweise zwischen 50 und 150 °C) durchgeführt.

In der Verbindung der Formel III steht Z für eine unter den Reaktionsbedingungen abspaltbare nucleofuge Gruppe, wie z.B. Halogen (insbesondere Chlor, Brom oder Jod) oder eine aromatische oder aliphatische Sulfonylgruppe, wie p-Toluolsulfonyl, Mesityl oder Methylsulfonyl.

Als Einzelverbindungen der Formel III sind zu nennen: Chlor- bzw. Bromessigsäuremethylester, -ethylester, -allylester, -iso-propylester, -propylester und -cyclopropylester; N-Methyl-bromessigsäureamid, N,N-Dimethylbromessigsäureamid, N-Methyl-Chloressigsäureamid und N,N-Dimethylchloressigsäureamid; Bromacetonitril und Chloracetonitril.

Die gemäss vorstehendem Verfahren herstellbaren Verbindungen der Formel IV können durch Fällung, Kristallisation, Extraktion, chromatographischen oder destillativen Trenn- und Reinigungsverfahren isoliert werden. Sie können aber auch ohne Isolierung des reinen Stoffes für den nachfolgenden Reaktionsschritt $r_2$ direkt weiterverwendet werden.

Die Cyclisierung der N,N-disubstituierten N'-Cyanoformamidine IV gemäss Reaktionsschritt $r_2$ erfolgt unter Baseneinwirkung in einem reaktionsinerten Lösungsmittel analog zu literaturbekannten Verfahren (DD-118,640; K. Gewald und G. Heinhold, Monatsh. für Chemie, 107 (1976) 1413; Comprehensive Heterocyclic Chemistry, Ed. K. T. Potts, Pergamon Press Oxford 1984, Bd. 5, S. 466, 467).

Als geeignete Basen für die Cyclisierung $r_2$ sind diejenigen anzugeben, deren Basenstärke ausreichend ist, um die Ausgangsverbindung der Formel IV zu deprotonieren. Es können äquimolare aber auch geringere als äquimolere (katalytische) Mengen an Basen verwendet werden.

Für die Reaktion geeignete Basen sind:

Alkali- und Erdalkalialkoholate, wie z.B. Kalium- oder Natriummethanolat, -ethanolat oder -tert.butylat; anorganische Basen, wie NaOH, KOH, Natriumcarbonat etc.; Alkali- bzw. Erdalkalihydride, wie Natriumhydrid; Alkaliamide, wie Natriumamid; stickstoffhaltige organische Basen, wie DABCO (Diazabicyclooctan) etc.

Vorteilhaft kann die Reaktion auch unter Phasentransferbedingungen in einem zweiphasigen System durchgeführt werden. Geeignete Systeme von Basen, Lösungsmitteln und Phasentransferkatalysatoren sind literaturbekannt.

Die Cyclisierung $r_2$ kann bei Temperaturen zwischen 0 °C und der Siedetemperatur des Reaktionsgemisches, vorzugweise jedoch bei Temperaturen etwa zwischen 50 und 150 °C durchgeführt werden.

Das Aminoimidazol V kann nach üblichen Methoden durch Fällung, Kristallisation, Extraktion oder durch chromatographische oder destillative Verfahren isoliert und gereinigt werden.

In den Verfahrensschritten $r_3$ und $r_4$ wird das 4-Aminoimidazol der Formel V zum gewünschten Endprodukt reduziert.

Dazu wird Verbindung V zunächst nach an sich bekannten Verfahren diazotiert. Diazotierungen heterocyclischer Amine sind allgemein bekannt (R. N. Butler, Chem. Rev. 75 (1975) 241).

Als Diazotierungsmittel für Amine der Formel II sind generell alle für die Diazotierung aromatischer Amine geeignete Mittel in Betracht zu ziehen.

Als geeignete Mittel zur Diazotierung sind zu nennen:

Nitritsalze in Kombination mit Säuren; $NO^{\oplus}BF_4^{\ominus}$; sowie
Alkylnitrite der Formel XIII

Alk—O—NO (XIII),

worin Alk für $C_1$-$C_8$-Alkyl steht, insbesondere Isoamylnitrit und tert.-Butylnitrit.

Bevorzugt ist das System Nitritsalz/Säure. Als Nitritsalze sind vor allem geeignet Natriumnitrit und Kaliumnitrit.

Als Säuren sind geeignet Mineralsäuren; organische Säuren sowie die Kombination von Mineralsäuren mit organischen Säuren.

Als Mineralsäuren kommen unter anderem in Frage:

Halogenwasserstoffsäuren, wie Fluorwasserstoffsäure, Salzsäure, Bromwasserstoffsäure oder Jodwasserstoffsäure; Perhalogensäuren wie Perchlorsäure, Perbrom- und Perjodsäure; Phosphorsäure; unterphosphorige Säure; Schwefelsäure; Salpertersäure; Tetrafluorborsäure u.a.

Organische Säuren sind etwa die Trifluoressigsäure, Ameisensäure, Essigsäure, Propionsäure, Benzol- oder Toluolsulfonsäure, Sulfonsäuren, wie Methansulfonsäure etc.

Die zuvor genannten Mineralsäuren und organischen Säuren können in wässriger Lösung oder als Mischung von Mineralsäure und organische Säure gewünschtenfalls auch unter Zusatz von Wasser oder einem Cosolvens verwendet werden.

Durch Wahl der Säure und des Nitritsalzes sowie gewünschtenfalls des Lösungsmittels kann die erfindungsgemässe Reaktion als zweistufiges Verfahren, d.h. unter Isolierung des Diazoniumsalzes der Formel VI, oder als einstufiges Verfahren, d.h. ohne Isolierung des Diazoniumsalzes VI durchgeführt werden.

Sofern eine Isolierung des Diazoniumsalzes VI gewünscht wird, muss bei niedrigen Tempeaturen und vorzugsweise unter Verwendung folgender Mineralsäuren gearbeitet werden: Salzsäure, Perchlorsäure, Tetrafluorborsäure, Perbromsäure, Perjodsäure, Hexafluorphosphorsäure.

Ausserdem kann bevorzugt unter Verwendung von $NO^{\oplus}BF_4^{\ominus}$ oder Isoamylnitrit/Perchlorat bzw. $HBF_4$ gearbeitet werden.

Generell kann das Diazoniumsalz der Formel VI aber auch durch Arbeiten bei Temperaturen unter 0 °C aus den jeweiligen Aminoimidazolen und den oben angegebenen Diazotierungsmitteln hergestellt werden.

Die Reduktion von Diazoniumsalzen ist literaturbekannt (Organ. Reactions 2, (1944), 262; Org. Synth. Coll. Vol. 3 (1955) 295; Org. Synth. Coll. Vol. 4 (1963) 947; Angew. Chem. 70 (1958) 211; J. Org. Chem. 28 (1963), 568; J. Amer. Chem. Soc. 83 (1961) 1251; Tetrahedron 26 (1970) 4609; J. Org. Chem. 36 (1971) 1725; Chem. Lett. 1979 1051).

Als geeignete Reduktionsmittel sind unter anderem zu nennen: Unterphosphorige Säure ($H_3PO_2$); Alkohole, wie Methanol, Ethanol, n-Propanol, iso-Propanol Butanol; Hydrazin; Hydride, wie $(C_4H_9)_3$-SnH, $NaBH_4$; Komplexe, wie $RhCl[P(C_6H_3)_3]_3$; Thiophenol; Dioxan; Tetrahydrofuran; Dimethylformamid oder Formaldehydderivate, insbesondere unter alkalischen Bedingungen.

Eine Zusammenstellung geeigneter Reduktionsmittel ist in oben genannter Literaturstelle "Organic Reactions" angegeben, worauf ausdrücklich Bezug genommen wird.

Oft ist es vorteilhaft das Diazoniumsalz VI nicht zu isolieren sondern in situ zum gewünschten Produkt I zu reduzieren. In diesen Fällen hat es sich als vorteilhaft erweisen die Diazotierung mit $NaNO_2$/Mineralsäure gegebenenfalls unter Zusatz von Eisessig und/oder Propionsäure durchzuführen und nach der Diazotierung das Reaktionsgemisch direkt mit unterphosphoriger Säure zu versetzen oder aber die Diazotierungsreaktion in Gegenwart eines der oben genannten Reduktionsmittel als Cosolvens zu arbeiten.

Die Deaminierung der Aminoimidazole V kann auch bevorzugt durch Umsetzung mit Alkylnitriten der Formel XIII

Alk—O—NO (XIII),

worin Alk für einen $C_4$-$C_8$-Alkylrest steht, durchgeführt werden, wobei die Diazotierung vorzugsweise, im Sinne einer einstufigen Reaktion, ohne Isolierung des Diazoniumsalzes VI ($A^{\ominus}$ = Alk-$O^{\ominus}$) direkt unter Stickstoffabspaltung zum Endprodukt I führt. Dabei wird die Diazotierung von V mit XIII vorteilhaft in einem Lösungsmittel durchgeführt, welches zur Reduktion der intermediär entstehenden Diazoniumsalzes befähigt ist, wie Dimethylformamid, Dioxan, Tetrahydrofuran oder $C_1$-$C_8$-Alkohole (J.C.S. Perkin I, 1973, 541; J. Org. Chem. 42 (1977) 3494 sowie die vorstehend genannten Literaturzitate).

In besonders vorteilhafter Weise können die Verbindungen der Formel I in einem "Eintopf-Verfahren", d.h. ohne Isolierung der Zwischenstufen II, IV und V ausgehend von Aminen der Formel VIII gemäss nachfolgendem Schema hergestellt werden:

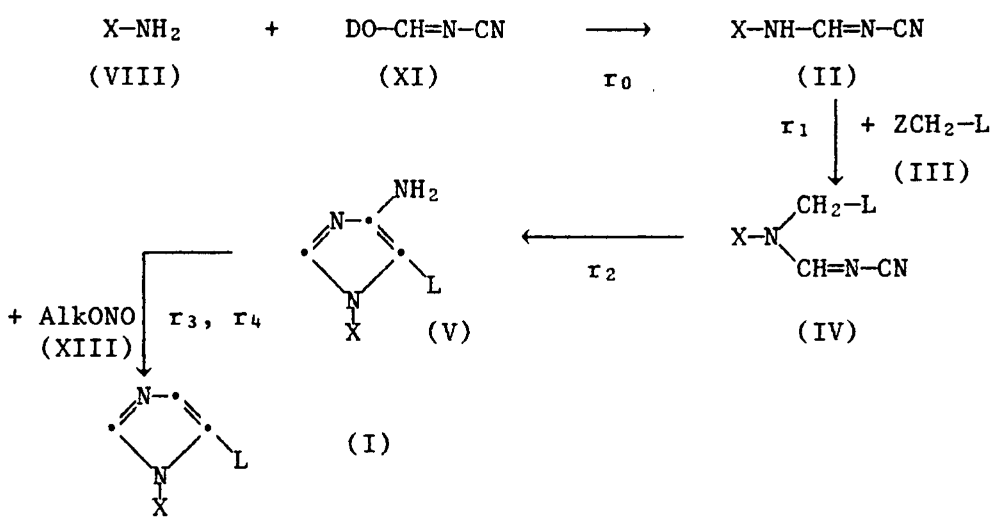

(VIII) (XI) (II) (IV) (V) (I)

Die vorstehend beschriebenen Umsetzung wird dabei vorzugsweise in einem Lösungsmittel oder einem Lösungmittelgemisch durchgeführt, welches ein für die reduktive Diazotierung geeignetes Lösungsmittel (bzw. Cosolvens) enthält, wie etwa Dimethylformamid.

Mit dem Begriff reaktionsinerte Lösungsmittel, welcher bei der Beschreibung der jeweiligen Verfahren $r_1$, $r_2$, $r_3$ und $r_4$ verwendet wird, sind in erster Linie Lösungsmittel gemeint, in welchen die Edukte oder die Reaktionszwischenstufen löslich, emulgier- oder suspendierbar sind, ohne dass das Lösungsmittel seinerseits mit dem Edukt, der Zwischenstufe oder mit dem Produkt reagiert. Protolysereaktionen sind mit diesem Begriff aber nicht ausgeschlossen.

Geeignete Lösungsmittel sind:

Alkohole, wie Methanol, Ethanol, Propanol, iso-Propanol, Butanol, Ethylenglycol etc.

Ether, wie Diethylether, Tetrahydrofuran, Furan, Ethylenglycol-, mono- oder di-Methyl oder -Ethylether, Diglyme; Amide, wie Dimethylformamid, N-Methylpyrrolidon, Dimethylacetamid etc;

Sulfone und Sulfoxide, wie Sulfolan, Dimethylsulfoxid etc; Ketone, wie Aceton, Methylethylketon etc;

Ester, wie Essigsäurethylester, Essigsäurebutylester etc;
Halogenierte Kohlenwasserstoffe, wie Di-, Tri- und Tetrachlormethan, Tetrachlorethylen etc;
Nitrile, wie Acetonitril, Propionitril; Isocyclische und acyclische Kohlenwasserstoffe, wie Benzin, Benzol, Toluol, Xylol, Chlorbenzol, Nitrobenzol, Pentan, Hexan, Heptan etc; aber auch Wasser und gegebenenfalls wässrige Basen oder Säuren.

Im Falle der Diazotierung ($r_3$) und der Reduktion ($r_4$) kommen auch wässrige Systeme von Mineralsäuren sowie organischen Säuren in Betracht.

Die folgenden Beispiele erläutern die Erfindung:

Herstellungsbeispiel 1: Aminomethylencyanamide der Formel II

Beispiel 1.01: N-(2,2-Dimethyl-1,2,3,4-tetrahydronaphth-1-yl) aminomethylencyanamid

19,3 g 1-Amino-2,2-dimethyl-1,2,3,4-tetrahydro-naphthalin (0,11 Mol) werden in 50 ml Ethanol abs. vorgelegt und 10,8 g Ethoxymethylencyanamid (0,11 Mol) zugegeben. Die Lösung wird 90 Min. unter Rückfluss gerührt und dann eingeengt. Der zunächst ölige Rückstand kristallisiert beim Anreiben.

Man isoliert 16,3 g (65 % der Theorie) der Titelverbindung der Formel

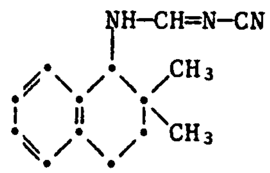

als farblose Kristalle vom Fp. 140-142 °C (Verb. No. 1.01)

Beispiel 1.02: N-(2,2-Dimethylindan-1-yl)-aminomethylencyanamid

Ausgehend von 1-Amino-2,2-dimethylindan und Ethoxymethylencyanamid erhält man analog zu Beispiel 1.01 in 96 % Ausbeute die Titelverbindung der Formel

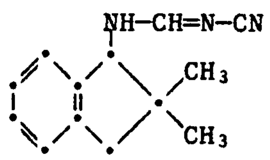

als farblose Kristalle vom Fp. 164-167 °C (Verb. No. 1.02).

Beispiel 1.03: N-(2,6-Dimethylphenyl)-aminomethylen-cyanamid

Ausgehend von 2,6-Dimethylanilin und Ethoxymethylencyanamid erhält man analog zu Beispiel 1.1 in 56 % Ausbeute die Titelverbindung der Formel

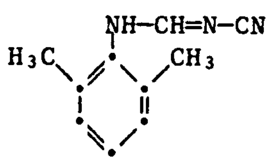

als farblose Kristalle vom Fp. 162-165 °C (Verb. No. 1.03).

Beispiel 1.04: N-(Diphenylmethyl)-aminomethylen-cyanamid

Ausgehend von Diphenylmethylamin und Ethoxymethylencyanamid erhält man analog zu Beispiel 1.1 in 83 % Ausbeute die Titelverbindung der Formel

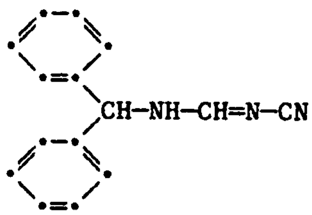

als farblose Kristalle vom Fp. 158-160 °C (Verb. No. 1.04).

Beispiel 1.05: (+)-N-(2,2-Dimethylindan-1-yl)-aminomethylencyanamid

Analog zu Beispiel 1.01 erhält man aus 45,2 g (-)-1-Amino-2,2-dimethylindan (ee ~ 90 %) und 27,5 g Ethoxymethylencyanamid in 60 ml Ethanol 54,7 g (92 %) der Titelverbindung der Formel

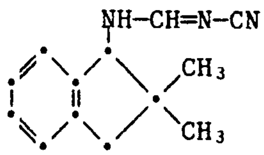

(+)

mit einem $[\alpha]_D^{20}$: +144,0±3° ($CHCl_3$) und einem Fp.: 164-167 °C (Verb. No. 1.123).

Analog zu den vorstehenden Beispielen können die Verbindungen der Tabelle 1 erhalten werden.

Tabelle 1: Verbindungen der Formel

X − NH − CH = N − CN

| Verb. No. | X | | phys. Daten |
|---|---|---|---|
| 1.01 | 2,2-Dimethyl-1,2,3,4-tetrahydronaphthalin-1-yl | | Fp.140-142°C |
| 1.02 | 2,2-Dimethyl-indan-1-yl | | Fp.164-167°C |
| 1.03 | 2,6-Dimethylphenyl | | Fp.162-165°C |
| 1.04 | Diphenylmethyl | | Fp.158-160°C |
| 1.05 | Indan-1-yl | | |
| 1.06 | 2-Methyl-indan-1-yl | | |
| 1.07 | cis-2-Methyl-indan-1-yl | | |
| 1.08 | trans-2-Methyl-indan-1-yl | | |
| 1.09 | 1,2,3,4-Tetrahydronaphthalin-1-yl | | |
| 1.10 | 2-Methyl-1,2,3,4-tetrahydronaphthalin-1-yl | | |
| 1.11 | cis-2-Methyl-1,2,3,4-tetrahydronaphthalin-1-yl | | |
| 1.12 | trans-2-Methyl-1,2,3,4-tetrahydro-naphthalin-1-yl | | |
| 1.13 | Benzocycloheptan-5-yl | | |
| 1.14 | 4,5,6,7-Tetrahydrobenzo[b]-thiophen-4-yl | | |
| 1.15 | 4,5,6,7-Tetrahydrobenzo[b]-furan-4-yl | | |
| 1.16 | 5,6,7,8-Tetrahydrochinolin-5-yl | | |
| 1.17 | 5,6,7,8-Tetrahydroisochinolin-5-yl | | |
| 1.18 | 5,6,7,8-Tetrahydrochinolin-8-yl | | |
| 1.19 | 5,6,7,8-Tetrahydroisochinolin-8-yl | | |

| Verb. No. | X | | phys. Daten |
|---|---|---|---|
| 1.20 | 9,10-Dihydroanthracen-9-yl | | |
| 1.21 | 9H-Fluoren-9-yl | | |
| 1.22 | Dibenzo[a,d]cyclohepten-5-yl | | |
| 1.23 | Dibenzo[a,d]cycloheptan-5-yl | | |
| 1.24 | 1,2-Dihydronaphthalin-1-yl | | |
| 1.25 | (4-chlorphenyl)-(phenyl)-methyl | | |
| 1.26 | 2-Ethyl-6-methyl-phenyl | | Oel |
| 1.27 | 2,6-Diethyl-phenyl | | Oel |
| 1.28 | 2,6-Di-isopropyl-phenyl | | Fp.131-135°C |
| 1.29 | 2,4,6-Trimethyl-phenyl | | |
| 1.30 | 4-Chlor-2,6-dimethyl-phenyl | | |
| 1.31 | 2-Ethyl-indan-1-yl | | |
| 1.32 | cis-2-Ethyl-indan-1-yl | | |
| 1.33 | trans-2-Ethyl-indan-1-yl | | |
| 1.34 | 2-Propyl-indan-1-yl | | |
| 1.35 | cis-Propyl-indan-1-yl | | |
| 1.36 | trans-Propyl-indan-1-yl | | |
| 1.37 | 2-Isopropyl-indan-1-yl | | |
| 1.38 | 2-Ethyl-2-methyl-indan-1-yl | | |
| 1.39 | 2-Methyl-2-propyl-indan-1-yl | | |
| 1.40 | 2,2-Diethyl-indan-1-yl | | |
| 1.41 | 2,2-Dipropyl-indan-1-yl | | |
| 1.42 | Spiro-[cyclopropan-1,2'-indan]-1'-yl | | |
| 1.43 | Spiro-[cyclopentan-1,2'-indan]-1'-yl | | |
| 1.44 | Spiro-[cyclohexan-1,2'-indan]-1'-yl | | |
| 1.45 | 2-Ethyl-1,2,3,4-tetrahydro-naphthalin-1-yl | | |
| 1.46 | cis-2-Ethyl-1,2,3,4-tetra-hydronaphthalin-1-yl | | |

| Verb. No. | X | | phys. Daten |
|---|---|---|---|
| 1.47 | trans-2-Ethyl-1,2,3,4-tetrahydronaphthalin-1-yl | | |
| 1.48 | 2-Propyl-1,2,3,4-tetrahydronaphthalin-1-yl | | |
| 1.49 | cis-2-Propyl-1,2,3,4-tetrahydronaphthalin-1-yl | | |
| 1.50 | trans-2-Propyl-1,2,3,4-tetrahydronaphthalin-1-yl | | |
| 1.51 | 2-Ethyl-2-methyl-1,2,3,4-tetrahydronaphthalin-1-yl | | |
| 1.52 | 2-Methyl-2-propyl-1,2,3,4-tetrahydronaphthalin-1-yl | | |
| 1.53 | 2,2-Diethyl-1,2,3,4-tetrahydronaphthalin-1-yl | | |
| 1.54 | 2,2-Dipropyl-1,2,3,4-tetrahydronaphthalin-1-yl | | |
| 1.55 | Spiro[cyclopropan-1,2'-(1',2',3',4'-tetrahydronaphthalin)]-1'-yl | | |
| 1.56 | Spiro[cyclopentan-1,2'-(1',2',3',4'-tetrahydronaphthalin)]-1'-yl | | |
| 1.57 | Spiro[cyclohexan-1,2'-(1',2',3',4'-tetrahydronaphthalin)]-1'-yl | | |
| 1.58 | Chroman-4-yl | | |
| 1.59 | 3-Methylchroman-4-yl | | |
| 1.60 | cis-3-Methylchroman-4-yl | | |
| 1.61 | trans-3-Methylchroman-4-yl | | |
| 1.62 | 3-Ethylchroman-4-yl | | |
| 1.63 | cis-3-Ethylchroman-4-yl | | |
| 1.64 | trans-3-Ethylchroman-4-yl | | |
| 1.65 | 3-Propylchroman-4-yl | | |
| 1.66 | cis-3-Propylchroman-4-yl | | |
| 1.67 | trans-3-Propylchroman-4-yl | | |
| 1.68 | 3-Ethyl-3-methyl-chroman-4-yl | | |

| Verb. No. | X | | phys. Daten |
|---|---|---|---|
| 1.69 | 3-Methyl-3-propyl-chroman-4-yl | | |
| 1.70 | 3-Isopropylchroman-4-yl | | |
| 1.71 | cis 3-Isopropylchroman-4-yl | | |
| 1.72 | trans-3-Isopropylchroman-4-yl | | |
| 1.73 | 3,3-Dimethylchroman-4-yl | | |
| 1.74 | 3,3-Diethylchroman-4-yl | | |
| 1.75 | 3,3-Dipropylchroman-4-yl | | |
| 1.76 | Spiro[cyclopropan-1,3'-chroman]-4'-yl | | |
| 1.77 | Spiro[cyclopentan-1,3'-chroman]-4'-yl | | |
| 1.78 | Spiro[cyclohexan-1,3'-chroman]-4'-yl | | |
| 1.79 | 2-Methylchroman-4-yl | | |
| 1.80 | cis-2-Methylchroman-4-yl | | |
| 1.81 | trans-2-Methylchroman-4-yl | | |
| 1.82 | 2-Ethylchroman-4-yl | | |
| 1.83 | cis-2-Ethylchroman-4-yl | | |
| 1.84 | trans-2-Ethylchroman-4-yl | | |
| 1.85 | 2-Propylchroman-4-yl | | |
| 1.86 | cis-2-Propylchroman-4-yl | | |
| 1.87 | trans-2-Propylchroman-4-yl | | |
| 1.88 | 2-Ethyl-2-methylchroman-4-yl | | |
| 1.89 | 2-Methyl-2-propylchroman-4-yl | | |
| 1.90 | 2-Isopropylchroman-4-yl | | |
| 1.91 | cis-2-Isopropylchroman-4-yl | | |
| 1.92 | trans-2-Isopropylchroman-4-yl | | |
| 1.93 | 2,2-Dimethylchroman-4-yl | | |
| 1.94 | 2,2-Diethylchroman-4-yl | | |
| 1.95 | 2,2-Dipropylchroman-4-yl | | |
| 1.96 | Spiro[cyclopropan-1,2'-chroman]-4'-yl | | |

| Verb. No. | X | | phys. Daten |
|---|---|---|---|
| 1.97 | Spiro[cyclopentan-1,2'-chroman]-4'-yl | | |
| 1.98 | Spiro[cyclohexan-1,2'-chroman]-4'-yl | | |
| 1.99 | 2,3-Dimethyl-chroman-4-yl | | |
| 1.100 | [cis-2,3-Dimethyl]-chroman-4-yl | | |
| 1.101 | [trans-2,3-Dimethyl]-chroman-4-yl | | |
| 1.102 | Thiochroman-4-yl | | |
| 1.103 | 2,2-Dimethylthiochroman-4-yl | | |
| 1.104 | 3,3-Dimethylthiochroman-4-yl | | |
| 1.105 | 2,3-Dimethylthiochroman-4-yl | | |
| 1.106 | [cis-2,3-Dimethyl]thio-chroman-4-yl | | |
| 1.107 | [trans-2,3-Dimethyl]thio-chroman-4-yl | | |
| 1.108 | 2-Methyl-thiochroman-4-yl | | |
| 1.109 | 2-Ethyl-thiochroman-4-yl | | |
| 1.110 | 2-Ethyl-2-methyl-thio-chroman-4-yl | | |
| 1.111 | 2,2-Diethyl-thiochroman-4-yl | | |
| 1.112 | 3,3-Diethyl-thiochroman-4-yl | | |
| 1.113 | 3-Ethyl-3-methyl-thio-chroman-4-yl | | |
| 1.120 | 2,3-Dimethylindan-1-yl | | |
| 1.121 | [cis-2,3-Dimethyl]indan-1-yl | | |
| 1.122 | [trans-2,3-Dimethyl]indan-1-yl | | |
| 1.123 | (+)-2,2-Dimethylindan-1-yl | | Fp.164-167°C |
| 1.124 | (-)-2,2-Dimethylindan-1-yl | | |
| 1.125 | (+)-2,2-Dimethylindan-1-yl | | |
| 1.126 | (-)-2,2-Dimethyl-1,2,3,4-tetrahydronaphthalin-1-yl | | |
| 1.127 | (+)-2,2-Dimethyl-1,2,3,4-tetrahydronaphthalin-1-yl | | |
| 1.128 | 3,3-Dimethylindan-1-yl | | |
| 1.129 | 3-Methylindan-1-yl | | |

Herstellungsbeispiel 2: Cyanamide der Formel IV

Beispiel 2.01: N-(Methoxycarbonylmethyl)-N-(2,2-dimethyl-1,2,3,4-tetrahydro-naphth-1-yl)-aminomethylen-cyanamid

120,7 g der Verbindung 1.01 (0,531 Mol) werden in 200 ml Methanol suspendiert und 95,6 g Natriummethylat/Methanol 30 % (0,531 Mol) zugegeben. Dann wird bei Raumtemperatur gerührt bis alles gelöst ist. Der grösste Teil des Lösungsmittels wird abdestilliert und der Rückstand in 400 ml DMSO aufgenommen. Danach wird das restliche Methanol durch 5malige Zugabe von je 300 ml Hexan und anschliessendes Abdestillieren entfernt. Unter Kühlen werden dann bei 20 °C 92,1 g Bromessigsäuremethylester (0,584 Mol) zugetropft. Nach kurzer Zeit fällt NaBr aus. Die Suspension wird 2 Std. bei 30° gerührt und dann mit 2 l Wasser verdünnt. Das abgeschiedene Oel wird in Essigester aufgenommen und über $Na_2SO_4$/Kohle getrocknet. Nach Abdestillieren des Lösungsmittels erhält man ein gelbes Oel, welches beim Verrühren mit Aceton/Ether kristallisert.

Man isoliert 87 g (55 %) der Titelverbindung der Formel

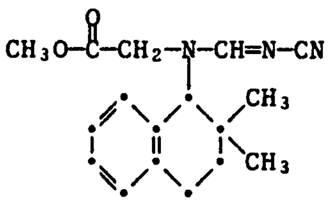

als farblose Kristalle vom Fp. 125-127 °C (Verb. No. 2.01).

Beispiel 2.02: N-(Methoxycarbonylmethyl)-N-(2,2-dimethyl-indan-1-yl)-aminomethylen-cyanamid

32,0 g der Verbindung 2.02 (0,15 Mol) werden in 160 ml Methanol suspendiert und 17,3 g K-tert.Butylat (0,15 Mol) zugegeben. Die klare Lösung wird zur Trockne eingedampft und das zurückbleibende K-Salz durch 3malige Zugabe von je 250 ml Toluol und anschliessendes Abdestillieren getrocknet und danach in 80 ml DMSO gelöst. Nach Zugabe von 0,5 g NaJ wird unter Kühlen bei 20-25 °C 23,6 g Bromessigsäuremethylester (0,15 Mol) zugetropft. Der Ansatz wird noch 2 Std. bei 90° gerührt, nach dem Erkalten in Wasser gegossen und das abgeschiedene Oel in Essigester aufgenommen. Nach Trocknen über $Na_2SO_4$//Kohle wird das Lösungsmittel abdestilliert. Das zurückbleibende Oel kristallisiert bei Verrühren mit wenig Methanol.

Man isoliert 19,3 g (46 %) der Titelverbindung der Formel

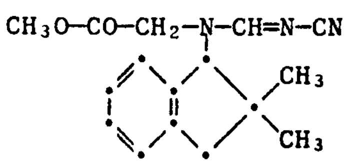

als farblose Kristalle vom Fp. 120-123 °C (Verb. No. 2.02).

Beispiel 2.03: N-(Methoxycarbonylmethyl)-N-(2,6-Dimethyl-phenyl)-aminomethylen-cyanamid

17,3 g der Verbindung No. 1.03 (0,1 Mol), 10,8 g Natriumcarbonat (0,1 Mol) 15,8 g Bromessigsäuremethylester (0,1 Mol), 0,5 g NaJ und 0,5 g 18-Krone-6 in 100 ml DMSO werden 5 Std. bei Raumtemperatur und danach 2 Std. bei 100° angerührt. Nach Erkalten wird in 1 l Wasser eingerührt und mit Essigsäure auf pH-6 gestellt ($CO_2$↑). Dabei scheidet sich das Produkt kristallin ab.

Man isoliert 16,0 g (65 %) der Titelverbindung der Formel

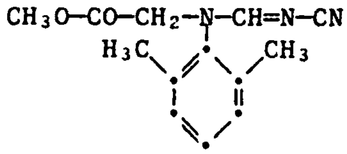

als Kristalle vom Fp. 153-156 °C (Verb. No. 2.03).

Beispiel 2.04: N-(Methoxycarbonylmethyl)-N-(diphenylmethyl)-aminomethylencyanamid

17, 3 g der Verb. No. 1.04 (0,073 Mol) werden in 100 ml DMSO gelöst und 9,0 g K-tert.Butylat (0,078 Mol) zugegeben. Die klare, rote Lösung wird 1 Std. bei Raumtemperatur gerührt und dann 12,3 g Bromessigsäuremethylester (0,078 Mol) zugetropft wobei sich die Lösung gelb färbt. Es wird noch 2 Std. bei Raumtemperatur gerührt und dann mit Wasser verdünnt.

Das abgeschiedene Oel wird in Essigester aufgenommen und über $Na_2SO_4$/Kohle getrocknet. Nach Abdestillieren des Lösungsmittels bleibt das Produkt als gelbes Oel zurück.

Man isoliert 17,1 g (76 %) der Titelverbindung der Formel

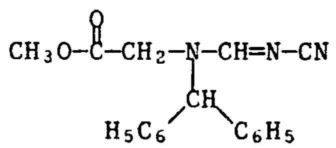

als gelbes Oel (Verb. No. 2.04), welches nach längerem Stehen kristallisiert (Smp. 108-110 °C).

Beispiel 2.05: (+)-N-(2,2-Dimethylindan-1-yl)-N-(methoxycarbonylmethyl)-aminomethy len-cyanamid

Analog zu Beispiel 2.04 erhält man aus 50,3 g (+)-N-(2,2-Dimethylindan-1-yl)-aminomethylencyanamid, 26,5 g K-tert.-Butylat und 36,1 g Bromessigsäuremethylester in 100 ml Dimethylsulfoxid 34,8 g (52 %) der Titelverbindung der Formel

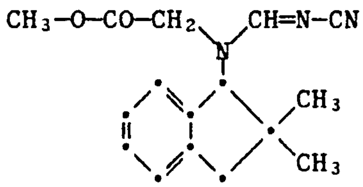

(+)

als Kristalle vom Fp. 116-119 °C und einem Drehwert von $[\alpha]_D^{20}$ : +75,1±0,4 ($CHCl_3$) (Verb. No. 2.143).

Analog zu den vorstehenden Beispielen 2.01 bis 2.05 können die Verbindungen der Tabelle 2 erhalten werden.

Tabelle 2: Verbindungen der Formel

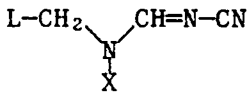

| Verb. No. | X | L | phys. Daten |
|---|---|---|---|
| 2.01 | 2,2-Dimethyl-1,2,3,4-tetrahydronaphthalin-1-yl | $COOCH_3$ | Fp.125-127°C |
| 2.02 | 2,2-Dimethyl-indan-1-yl | $COOCH_3$ | Fp.120-123°C |
| 2.03 | 2,6-Dimethylphenyl | $COOCH_3$ | Fp.153-156°C |
| 2.04 | Diphenylmethyl | $COOCH_3$ | Fp.108-110°C |
| 2.05 | Indan-1-yl | $COOCH_3$ | |
| 2.06 | 2-Methyl-indan-1-yl | $COOCH_3$ | |
| 2.07 | cis-2-Methyl-indan-1-yl | $COOCH_3$ | |
| 2.08 | trans-2-Methyl-indan-1-yl | $COOCH_3$ | |
| 2.09 | 1,2,3,4-Tetrahydronaphthalin-1-yl | $COOCH_3$ | |
| 2.10 | 2-Methyl-1,2,3,4-tetrahydronaphthalin-1-yl | $COOCH_3$ | |
| 2.11 | cis-2-Methyl-1,2,3,4-tetrahydronaphthalin-1-yl | $COOCH_3$ | |
| 2.12 | trans-2-Methyl-1,2,3,4-tetrahydro-naphthalin-1-yl | $COOCH_3$ | |
| 2.13 | Benzocycloheptan-5-yl | $COOCH_3$ | |
| 2.14 | 4,5,6,7-Tetrahydrobenzo[b]-thiophen-4-yl | $COOCH_3$ | |
| 2.15 | 4,5,6,7-Tetrahydrobenzo[b]-furan-4-yl | $COOCH_3$ | |
| 2.16 | 5,6,7,8-Tetrahydrochinolin-5-yl | $COOCH_3$ | |
| 2.17 | 5,6,7,8-Tetrahydroisochinolin-5-yl | $COOCH_3$ | |
| 2.18 | 5,6,7,8-Tetrahydrochinolin-8-yl | $COOCH_3$ | |

| Verb. No. | X | L | phys. Daten |
|---|---|---|---|
| 2.19 | 5,6,7,8-Tetrahydroiso-chinolin-8-yl | $COOCH_3$ | |
| 2.20 | 9,10-Dihydroanthracen-9-yl | $COOCH_3$ | |
| 2.21 | 9H-Fluoren-9-yl | $COOCH_3$ | |
| 2.22 | Dibenzo[a,d]cyclohepten-5-yl | $COOCH_3$ | |
| 2.23 | Dibenzo[a,d]cycloheptan-5-yl | $COOCH_3$ | |
| 2.24 | 1,2-Dihydronaphthalin-1-yl | $COOCH_3$ | |
| 2.25 | (4-chlorphenyl)-(phenyl)-methyl | $COOCH_3$ | |
| 2.26 | 2-Ethyl-6-methyl-phenyl | $COOCH_3$ | Fp.113-116°C |
| 2.27 | 2,6-Diethyl-phenyl | $COOCH_3$ | Oel |
| 2.28 | 2,6-Di-isopropyl-phenyl | $COOCH_3$ | Fp.141-143°C |
| 2.29 | 2,4,6-Trimethyl-phenyl | $COOCH_3$ | |
| 2.30 | 4-Chlor-2,6-dimethyl-phenyl | $COOCH_3$ | |
| 2.31 | 2-Ethyl-indan-1-yl | $COOCH_3$ | |
| 2.32 | cis-2-Ethyl-indan-1-yl | $COOCH_3$ | |
| 2.33 | trans-2-Ethyl-indan-1-yl | $COOCH_3$ | |
| 2.34 | 2-Propyl-indan-1-yl | $COOCH_3$ | |
| 2.35 | cis-Propyl-indan-1-yl | $COOCH_3$ | |
| 2.36 | trans-Propyl-indan-1-yl | $COOCH_3$ | |
| 2.37 | 2-Isopropyl-indan-1-yl | $COOCH_3$ | |
| 2.38 | 2-Ethyl-2-methyl-indan-1-yl | $COOCH_3$ | |
| 2.39 | 2-Methyl-2-propyl-indan-1-yl | $COOCH_3$ | |
| 2.40 | 2,2-Diethyl-indan-1-yl | $COOCH_3$ | |
| 2.41 | 2,2-Dipropyl-indan-1-yl | $COOCH_3$ | |
| 2.42 | Spiro-[cyclopropan-1,2'-indan]-1'-yl | $COOCH_3$ | |
| 2.43 | Spiro-[cyclopentan-1,2'-indan]-1'-yl | $COOCH_3$ | |
| 2.44 | Spiro-[cyclohexan-1,2'-indan]-1'-yl | $COOCH_3$ | |
| 2.45 | 2-Ethyl-1,2,3,4-tetrahydro-naphthalin-1-yl | $COOCH_3$ | |

| Verb. No. | X | L | phys. Daten |
|---|---|---|---|
| 2.46 | cis-2-Ethyl-1,2,3,4-tetrahydronaphthalin-1-yl | $COOCH_3$ | |
| 2.47 | trans-2-Ethyl-1,2,3,4-tetrahydronaphthalin-1-yl | $COOCH_3$ | |
| 2.48 | 2-Propyl-1,2,3,4-tetrahydronaphthalin-1-yl | $COOCH_3$ | |
| 2.49 | cis-2-Propyl-1,2,3,4-tetrahydronaphthalin-1-yl | $COOCH_3$ | |
| 2.50 | trans-2-Propyl-1,2,3,4-tetrahydronaphthalin-1-yl | $COOCH_3$ | |
| 2.51 | 2-Ethyl-2-methyl-1,2,3,4-tetrahydronaphthalin-1-yl | $COOCH_3$ | |
| 2.52 | 2-Methyl-2-propyl-1,2,3,4-tetrahydronaphthalin-1-yl | $COOCH_3$ | |
| 2.53 | 2,2-Diethyl-1,2,3,4-tetrahydronaphthalin-1-yl | $COOCH_3$ | |
| 2.54 | 2,2-Dipropyl-1,2,3,4-tetrahydronaphthalin-1-yl | $COOCH_3$ | |
| 2.55 | Spiro[cyclopropan-1,2'-(1',2',3',4'-tetrahydronaphthalin)]-1'-yl | $COOCH_3$ | |
| 2.56 | Spiro[cyclopentan-1,2'-(1',2',3',4'-tetrahydronaphthalin)]-1'-yl | $COOCH_3$ | |
| 2.57 | Spiro[cyclohexan-1,2'-(1',2',3',4'-tetrahydronaphthalin)]-1'-yl | $COOCH_3$ | |
| 2.58 | Chroman-4-yl | $COOCH_3$ | |
| 2.59 | 3-Methylchroman-4-yl | $COOCH_3$ | |
| 2.60 | cis-3-Methylchroman-4-yl | $COOCH_3$ | |
| 2.61 | trans-3-Methylchroman-4-yl | $COOCH_3$ | |
| 2.62 | 3-Ethylchroman-4-yl | $COOCH_3$ | |
| 2.63 | cis-3-Ethylchroman-4-yl | $COOCH_3$ | |
| 2.64 | trans-3-Ethylchroman-4-yl | $COOCH_3$ | |
| 2.65 | 3-Propylchroman-4-yl | $COOCH_3$ | |
| 2.66 | cis-3-Propylchroman-4-yl | $COOCH_3$ | |
| 2.67 | trans-3-Propylchroman-4-yl | $COOCH_3$ | |

| Verb. No. | X | L | phys. Daten |
|---|---|---|---|
| 2.68 | 3-Ethyl-3-methyl-chroman-4-yl | $COOCH_3$ | |
| 2.69 | 3-Methyl-3-propyl-chroman-4-yl | $COOCH_3$ | |
| 2.70 | 3-Isopropylchroman-4-yl | $COOCH_3$ | |
| 2.71 | cis 3-Isopropylchroman-4-yl | $COOCH_3$ | |
| 2.72 | trans-3-Isopropylchroman-4-yl | $COOCH_3$ | |
| 2.73 | 3,3-Dimethylchroman-4-yl | $COOCH_3$ | |
| 2.74 | 3,3-Diethylchroman-4-yl | $COOCH_3$ | |
| 2.75 | 3,3-Dipropylchroman-4-yl | $COOCH_3$ | |
| 2.76 | Spiro[cyclopropan-1,3'-chroman]-4'-yl | $COOCH_3$ | |
| 2.77 | Spiro[cyclopentan-1,3'-chroman]-4'-yl | $COOCH_3$ | |
| 2.78 | Spiro[cyclohexan-1,3'-chroman]-4'-yl | $COOCH_3$ | |
| 2.79 | 2,2-Dimethylindan-1-yl | $COOC_2H_5$ | |
| 2.80 | 2,2-Dimethylindan-1-yl | $COO-i-C_3H_7$ | |
| 2.81 | 2,2-Dimethylindan-1-yl | $COO-CH_2-CH=CH_2$ | |
| 2.82 | 2,2-Dimethylindan-1-yl | COO— 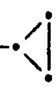 | |
| 2.83 | 2,2-Dimethylindan-1-yl | CO-NH $CH_3$ | |
| 2.84 | 2,2-Dimethylindan-1-yl | $CO-N(CH_3)_2$ | |
| 2.85 | 2,2-Dimethylindan-1-yl | CN | |
| 2.86 | 2,2-Dimethyl-1,2,3,4-tetrahydronaphthalin-1-yl | $COOC_2H_5$ | |
| 2.87 | 2,2-Dimethyl-1,2,3,4-tetrahydronaphthalin-1-yl | $COO-i-C_3H_7$ | |
| 2.88 | 2,2-Dimethyl-1,2,3,4-tetrahydronaphthalin-1-yl | $COO-CH_2-CH=CH_2$ | |
| 2.89 | 2,2-Dimethyl-1,2,3,4-tetrahydronaphthalin-1-yl | COO— 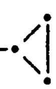 | |
| 2.90 | 2,2-Dimethyl-1,2,3,4-tetrahydronaphthalin-1-yl | CO-NH $CH_3$ | |

| Verb. No. | X | L | phys. Daten |
|---|---|---|---|
| 2.91 | 2,2-Dimethyl-1,2,3,4-tetra-hydronaphthalin-1-yl | $CO-N(CH_3)_2$ | |
| 2.92 | 2,2-Dimethyl-1,2,3,4-tetra-hydronaphthalin-1-yl | CN | |
| 2.93 | 2,6-Dimethylphenyl | $COOC_2H_5$ | |
| 2.94 | 2,6-Dimethylphenyl | $COO-i-C_3H_7$ | |
| 2.95 | 2,6-Dimethylphenyl | $COO-CH_2-CH=CH_2$ | |
| 2.96 | 2,6-Dimethylphenyl | COO— 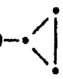 | |
| 2.97 | 2,6-Dimethylphenyl | $CO-NH\ CH_3$ | |
| 2.98 | 2,6-Dimethylphenyl | $CO-N(CH_3)_2$ | |
| 2.99 | 2,6-Dimethylphenyl | CN | |
| 2.100 | Diphenylmethyl | $COOC_2H_5$ | |
| 2.101 | Diphenylmethyl | $COO-i-C_3H_7$ | |
| 2.102 | Diphenylmethyl | $COO-CH_2-CH=CH_2$ | |
| 2.103 | Diphenylmethyl | COO— 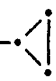 | |
| 2.104 | Diphenylmethyl | $CO-NH\ CH_3$ | |
| 2.105 | Diphenylmethyl | $CO-N(CH_3)_2$ | |
| 2.106 | Diphenylmethyl | CN | |
| 2.107 | 2-Methylchroman-4-yl | $COOCH_3$ | |
| 2.108 | cis-2-Methylchroman-4-yl | $COOCH_3$ | |
| 2.109 | trans-2-Methylchroman-4-yl | $COOCH_3$ | |
| 2.110 | 2-Ethylchroman-4-yl | $COOCH_3$ | |
| 2.111 | cis-2-Ethylchroman-4-yl | $COOCH_3$ | |
| 2.112 | trans-2-Ethylchroman-4-yl | $COOCH_3$ | |
| 2.113 | 2-Propylchroman-4-yl | $COOCH_3$ | |
| 2.114 | cis-2-Propylchroman-4-yl | $COOCH_3$ | |
| 2.115 | trans-2-Propylchroman-4-yl | $COOCH_3$ | |
| 2.116 | 2-Ethyl-2-methyl-chroman-4-yl | $COOCH_3$ | |
| 2.117 | 2-Methyl-2-propyl-chroman-4-yl | $COOCH_3$ | |
| 2.118 | 2-Isopropylchroman-4-yl | $COOCH_3$ | |
| 2.119 | cis 2-Isopropylchroman-4-yl | $COOCH_3$ | |

| Verb. No. | X | L | phys. Daten |
|---|---|---|---|
| 2.120 | trans-2-Isopropylchroman-4-yl | $COOCH_3$ | |
| 2.121 | 2,2-Dimethylchroman-4-yl | $COOCH_3$ | |
| 2.122 | 2,2-Diethylchroman-4-yl | $COOCH_3$ | |
| 2.123 | 2,2-Dipropylchroman-4-yl | $COOCH_3$ | |
| 2.124 | Spiro[cyclopropan-1,2'-chroman]-4'-yl | $COOCH_3$ | |
| 2.125 | Spiro[cyclopentan-1,2'-chroman]-4'-yl | $COOCH_3$ | |
| 2.126 | Spiro[cyclohexan-1,2'-chroman]-4'-yl | $COOCH_3$ | |
| 2.127 | 2,2-Dimethyl-1,2,3,4-tetrahydronaphthalin-1-yl | $CO-NH-i-C_3H_7$ | |
| 2.128 | Thiochroman-4-yl | $COOCH_3$ | |
| 2.129 | 2,2-Dimethylthiochroman-4-yl | $COOCH_3$ | |
| 2.130 | 3,3-Dimethylthiochroman-4-yl | $COOCH_3$ | |
| 2.131 | 2,3-Dimethylthiochroman-4-yl | $COOCH_3$ | |
| 2.132 | [cis-2,3-Dimethyl]thiochroman-4-yl | $COOCH_3$ | |
| 2.133 | [trans-2,3-Dimethyl]thiochroman-4-yl | $COOCH_3$ | |
| 2.134 | 2-Methyl-thiochroman-4-yl | $COOCH_3$ | |
| 2.135 | 2-Ethyl-thiochroman-4-yl | $COOCH_3$ | |
| 2.136 | 2-Methyl-2-ethyl-thiochroman-4-yl | $COOCH_3$ | |
| 2.137 | 2,2-Diethyl-thiochroman-4-yl | $COOCH_3$ | |
| 2.138 | 3,3-Diethyl-thiochroman-4-yl | $COOCH_3$ | |
| 2.139 | 3-Methyl-3-ethyl-thiochroman-4-yl | $COOCH_3$ | |
| 2.140 | 2,3-Dimethylindan-1-yl | $COOCH_3$ | |
| 2.141 | [cis-2,3-Dimethyl]indan-1-yl | $COOCH_3$ | |
| 2.142 | [trans-2,3-Dimethyl]indan-1-yl | $COOCH_3$ | |
| 2.143 | (+)-2,2-Dimethylindan-1-yl | $COOCH_3$ | Fp.116-119°C |
| 2.144 | (-)-2,2-Dimethylindan-1-yl | $COOCH_3$ | |
| 2.145 | (+)-2,2-Dimethyl-1,2,3,4-tetrahydronaphthalin-1-yl | $COOCH_3$ | |
| 2.146 | (-)-2,2-Dimethyl-1,2,3,4-tetrahydronaphthalin-1-yl | $COOCH_3$ | |

| Verb. No. | X | L | phys. Daten |
|---|---|---|---|
| 2.147 | 2,2-Dimethyl-1,2,3,4-tetrahydronaphthalin-1-yl | $CONH-C_3H_7(i)$ | Fp.186-188°C |

Herstellungsbeispiel 3 4-Aminoimidazole der Formel V

Beispiel 3.01: 4-Amino-1-(2,2-dimethyl-1,2,3,4-tetrahydro-naphth-1-yl)-imidazol-5-carbonssäure-methylester

10,0 g der Verbindung 2.01 werden in 30 ml Methanol suspendiert und nach Zugabe von 1,0 g Na-methylat/Methanol 30 % (0,006 Mol) 2 Std. zum Sieden erwärmt. Die entstandene Lösung wird nach Erkalten mit Wasser verdünnt. Dabei kristallisert das Produkt.

Man isoliert 8,4 g (84 %) der Titelverbindung der Formel

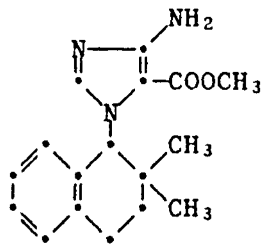

als farblose Kristalle mit Fp. 193-195 ° C (Verb. Nr. 3.01).

Beispiel 3.02: 4-Amino-1-(2,2-dimethyl-indan-1-yl)-imidazol-5-carbonsäuremethylester

12,2 g der Verbindung 2.02 (0.043 Mol) werden in 30 ml Methanol suspendiert und nach Zugeben von 1,6 g Na-methylat/Methanol 30 % (0,009 Mol) 1 Std. unter Rückfluss gerührt. Nach Erkalten wird die erhaltene Lösung mit Wasser verdünnt und mit Essigsäure auf pH 7 gestellt. Das kristalline Produkt wird filtriert und getrocknet.

Man isoliert 11,8 g (98 %) der Titelverbindung der Formel

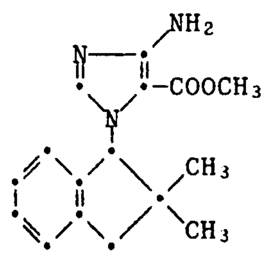

als farblose Kristalle mit Fp. 167-168 ° C (Verb. Nr. 3.02).

Beispiel 3.03: 4-Amino-1-(2,6-Dimethyl-phenyl)-imidazol-5-carbonsäuremethylester

2,5 g der Verbindung No 2.03 (0,01 Mol) werden in 30 ml Methanol suspendiert und nach Zugabe von 0,2 g Na-methylat/Methanol 30 % (0,001 Mol) 1 Std. bei 60 ° C gerührt. Die entstandene klare Lösung wird nach Erkalten mit Wasser verdünnt. Dabei kristallisiert das Produkt.

Man isoliert 2,1 g (88 %) der Titelverbindung der Formel

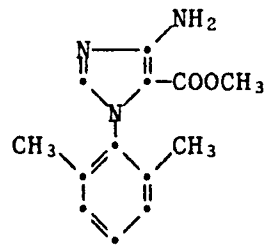

als farblose Kristalle mit Fp. 154-156 °C (Verb. Nr. 3.03).

Beispiel 3.04: 4-Amino-1-(diphenylmethyl)-imidazol-5-carbonsäuremethylester

15,2 g der Verbindung No. 2.04 (0,049 Mol) werden in 50 ml Methanol gelöst. Nach Zugabe von 0,45 g Natriumethylat/Methanol 30 % (0,002 Mol) wird 1 Std. bei 60° gerührt. Dabei fällt das Produkt kristallin aus. Nach Erkalten wird mit Wasser verdünnt und abgekühlt.

Man isoliert 12,5 g (83 %) der Titelverbindung der Formel

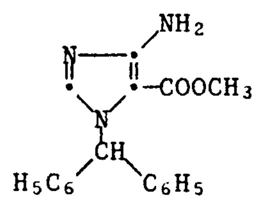

als farblose Kristalle mit Fp. 199-201 °C (Verb. Nr. 3.04).

Beispiel 3.05: Verfahren zur Herstellung von 4-Aminoimidazolen der Formel V aus Aminen der Formel VIII, ohne Isolierung der Zwischenprodukte: 4-Amino-1-(2,2-dimethylindan-1-yl)-imidazol-5-carbonsäuremethylester

Zu einer Lösung von 6,4 g 1-Amino-2,2-dimethylindan in 50 ml Dimethylformamid werden 3,9 g Ethoxymethylencyanamid gegeben. Dabei steigt die Temperatur auf 40 °C an. Das Reaktionsgemisch wird 90 Min. bei 80 °C gerührt, abgekühlt und bei Raumtemperatur am Hochvakuum das im Reaktionsverlauf entstandene Ethanol abdestilliert.

Danach werden 13,8 g gemahlenes Kaliumcarbonat und eine Spatelspitze 18-crown-6 zugegeben und 30 Min. bei 80 °C gerührt. Danach wird mit 6,7 g Bromessigsäuremethylester versetzt. Die Temperatur steigt dabei auf 87 °C an. Nach einstündigem Rühren bei 80 °C wird das Reaktionsgemisch 15 Stunden bei 100 °C belassen. Nach dem Erkalten wird von dem Ungelösten abfiltriert, das Filtrat in Wasser gegossen und mit Essigester extrahiert. Die vereinigten Extrakte werden getrocknet, eingeengt und an Kieselgel mit Essigester/Hexan (1:1) gereinigt.

Man isoliert 6,4 g (56 %) der Titelverbindung der Formel

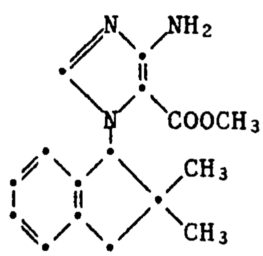

als Kristalle vom Fp. 167-168 °C (Verb. Nr. 3.02).

Beispiel 3.06: (+)-4-Amino-1-(2,2-dimethyl-1,2,3,4-tetrahydronaphthalin-1-yl)-imidazol-5-carbonsäuremethylester

10,5 g (-)-1-Amino-2,2-dimethyl-1,2,3,4-tetrahydronaphthalin (0,06 Mol) werden in 50 ml N,N-Dimethylformamid gelöst und mit 5,9 g Ethoxymethylencyanamid (0,06 Mol) versetzt. Dabei steigt die Temperatur auf 35 °C. Die Lösung wird 2 Stunden bei 80 °C gerührt, abgekühlt und bei Raumtemperatur am Hochvakuum das entstandene Ethanol abdestilliert. Danach werden 20,7 g pulverisierter Kaliumcarbonat (0,15 Mol) und nach 30 Min. Rühren bei 80 °C zu dieser Suspension 10,1 g Bromessigsäuremethylester (0,066 Mol) zugegeben. Die Temperatur steigt dabei auf 100 °C.
Nach 15 Stunden bei 100 °C Rühren wird die abgekühlte Suspension auf 500 ml Wasser ausgegossen und mit Essigsäure ethylester extrahiert. Die vereinigten organischen Phasen werden über $Na_2SO_4$ getrocknet und filtriert. Nach Abdestillieren des Lösungsmittels erhält man 15,8 g eines braunen Oels (Rohprodukt), welches über eine Drucksäule (Kieselgel) mit Essigsäure-ethylester/Hexan 1:1 gereinigt wird. Die so erhaltenen 5,3 g Oel werden in 20 ml Ethanol warm gelöst, mit Aktivkohle behandelt und filtriert. Beim Erkalten beginnt das Produkt auszukristallisieren.

Man erhält 4,1 g (22,9 %) der Titelverbindung der Formel

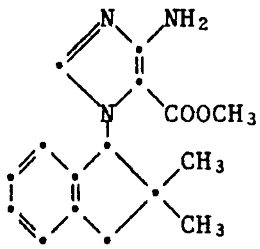

(+)

als braungelbes Harz mit einem Drehwert von $[\alpha]_D^{20}$ : 54,18 ($CHCl_3$) (Verb. Nr. 3.143).

Beispiel 3.07: (-)-4-Amino-1-(2,2-dimethylindan-1-yl)-imidazol-5-carbonsäuremethylester

Analog Beispiel 3.01 erhält man aus 29,7 g (+)-N-(2,2-Dimethylindan-1-yl)-N-(methoxycarbonylmethyl)-aminomethylencyanamid und 3,8 g Natriummethylat (30 % in Methanol) in 100 ml Methanol 26,1 g (88 %) der Titelverbindung der Formel

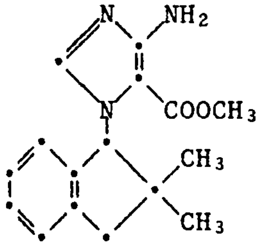

(-)

als Kristalle vom Fp. 100-105 °C mit einem Drehwert $[\alpha]_D^{20}$ : -106,6 ° ±0,4 ° ($CHCl_3$) (Verb. Nr. 3.144).

Analog zu den vorstehenden Beispielen 3.01 bis 3.07 können die Verbindungen der Tabelle 3 erhalten werden.

Tabelle 3: Verbindungen der Formel

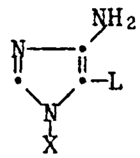

| Verb. No. | X | L | phys. Daten |
|---|---|---|---|
| 3.01 | 2,2-Dimethyl-1,2,3,4-tetrahydronaphthalin-1-yl | $COOCH_3$ | Fp.193-195°C |
| 3.02 | 2,2-Dimethyl-indan-1-yl | $COOCH_3$ | Fp.167-168°C |
| 3.03 | 2,6-Dimethylphenyl | $COOCH_3$ | Fp.154-156°C |
| 3.04 | Diphenylmethyl | $COOCH_3$ | Fp.199-201°C |
| 3.05 | Indan-1-yl | $COOCH_3$ | |
| 3.06 | 2-Methyl-indan-1-yl | $COOCH_3$ | |
| 3.07 | cis-2-Methyl-indan-1-yl | $COOCH_3$ | |
| 3.08 | trans-2-Methyl-indan-1-yl | $COOCH_3$ | |
| 3.09 | 1,2,3,4-Tetrahydronaphthalin-1-yl | $COOCH_3$ | |
| 3.10 | 2-Methyl-1,2,3,4-tetrahydronaphthalin-1-yl | $COOCH_3$ | |
| 3.11 | cis-2-Methyl-1,2,3,4-tetrahydronaphthalin-1-yl | $COOCH_3$ | |
| 3.12 | trans-2-Methyl-1,2,3,4-tetrahydro-naphthalin-1-yl | $COOCH_3$ | |
| 3.13 | Benzocycloheptan-5-yl | $COOCH_3$ | |
| 3.14 | 4,5,6,7-Tetrahydrobenzo[b]-thiophen-4-yl | $COOCH_3$ | |
| 3.15 | 4,5,6,7-Tetrahydrobenzo[b]-furan-4-yl | $COOCH_3$ | |
| 3.16 | 5,6,7,8-Tetrahydrochinolin-5-yl | $COOCH_3$ | |
| 3.17 | 5,6,7,8-Tetrahydroiso-chinolin-5-yl | $COOCH_3$ | |
| 3.18 | 5,6,7,8-Tetrahydrochinolin-8-yl | $COOCH_3$ | |

| Verb. No. | X | L | phys. Daten |
|---|---|---|---|
| 3.19 | 5,6,7,8-Tetrahydroiso-chinolin-8-yl | $COOCH_3$ | |
| 3.20 | 9,10-Dihydroanthracen-9-yl | $COOCH_3$ | |
| 3.21 | 9H-Fluoren-9-yl | $COOCH_3$ | |
| 3.22 | Dibenzo[a,d]cyclohepten-5-yl | $COOCH_3$ | |
| 3.23 | Dibenzo[a,d]cycloheptan-5-yl | $COOCH_3$ | |
| 3.24 | 1,2-Dihydronaphthalin-1-yl | $COOCH_3$ | |
| 3.25 | (4-chlorphenyl)-(phenyl)-methyl | $COOCH_3$ | |
| 3.26 | 2-Ethyl-6-methyl-phenyl | $COOCH_3$ | Fp.123-127°C |
| 3.27 | 2,6-Diethyl-phenyl | $COOCH_3$ | Fp.103-105°C |
| 3.28 | 2,6-Di-isopropyl-phenyl | $COOCH_3$ | Fp.127-129°C |
| 3.29 | 2,4,6-Trimethyl-phenyl | $COOCH_3$ | |
| 3.30 | 4-Chlor-2,6-dimethyl-phenyl | $COOCH_3$ | |
| 3.31 | 2-Ethyl-indan-1-yl | $COOCH_3$ | |
| 3.32 | cis-2-Ethyl-indan-1-yl | $COOCH_3$ | |
| 3.33 | trans-2-Ethyl-indan-1-yl | $COOCH_3$ | |
| 3.34 | 2-Propyl-indan-1-yl | $COOCH_3$ | |
| 3.35 | cis-Propyl-indan-1-yl | $COOCH_3$ | |
| 3.36 | trans-Propyl-indan-1-yl | $COOCH_3$ | |
| 3.37 | 2-Isopropyl-indan-1-yl | $COOCH_3$ | |
| 3.38 | 2-Ethyl-2-methyl-indan-1-yl | $COOCH_3$ | |
| 3.39 | 2-Methyl-2-propyl-indan-1-yl | $COOCH_3$ | |
| 3.40 | 2,2-Diethyl-indan-1-yl | $COOCH_3$ | |
| 3.41 | 2,2-Dipropyl-indan-1-yl | $COOCH_3$ | |
| 3.42 | Spiro-[cyclopropan-1,2'-indan]-1'-yl | $COOCH_3$ | |
| 3.43 | Spiro-[cyclopentan-1,2'-indan]-1'-yl | $COOCH_3$ | |
| 3.44 | Spiro-[cyclohexan-1,2'-indan]-1'-yl | $COOCH_3$ | |
| 3.45 | 2-Ethyl-1,2,3,4-tetrahydro-naphthalin-1-yl | $COOCH_3$ | |

| Verb. No. | X | L | phys. Daten |
|---|---|---|---|
| 3.46 | cis-2-Ethyl-1,2,3,4-tetra-hydronaphthalin-1-yl | $COOCH_3$ | |
| 3.47 | trans-2-Ethyl-1,2,3,4-tetra-hydronaphthalin-1-yl | $COOCH_3$ | |
| 3.48 | 2-Propyl-1,2,3,4-tetra-hydronaphthalin-1-yl | $COOCH_3$ | |
| 3.49 | cis-2-Propyl-1,2,3,4-tetra-hydronaphthalin-1-yl | $COOCH_3$ | |
| 3.50 | trans-2-Propyl-1,2,3,4-tetrahydronaphthalin-1-yl | $COOCH_3$ | |
| 3.51 | 2-Ethyl-2-methyl-1,2,3,4-tetrahydronaphthalin-1-yl | $COOCH_3$ | |
| 3.52 | 2-Methyl-2-propyl-1,2,3,4-tetrahydronaphthalin-1-yl | $COOCH_3$ | |
| 3.53 | 2,2-Diethyl-1,2,3,4-tetra-hydronaphthalin-1-yl | $COOCH_3$ | |
| 3.54 | 2,2-Dipropyl-1,2,3,4-tetra-hydronaphthalin-1-yl | $COOCH_3$ | |
| 3.55 | Spiro[cyclopropan-1,2'-(1',2',3',4'-tetrahydro-naphthalin)]-1'-yl | $COOCH_3$ | |
| 3.56 | Spiro[cyclopentan-1,2'-(1',2',3',4'-tetrahydro-naphthalin)]-1'-yl | $COOCH_3$ | |
| 3.57 | Spiro[cyclohexan-1,2'-(1',2',3',4'-tetrahydro-naphthalin)]-1'-yl | $COOCH_3$ | |
| 3.58 | Chroman-4-yl | $COOCH_3$ | |
| 3.59 | 3-Methylchroman-4-yl | $COOCH_3$ | |
| 3.60 | cis-3-Methylchroman-4-yl | $COOCH_3$ | |
| 3.61 | trans-3-Methylchroman-4-yl | $COOCH_3$ | |
| 3.62 | 3-Ethylchroman-4-yl | $COOCH_3$ | |
| 3.63 | cis-3-Ethylchroman-4-yl | $COOCH_3$ | |
| 3.64 | trans-3-Ethylchroman-4-yl | $COOCH_3$ | |
| 3.65 | 3-Propylchroman-4-yl | $COOCH_3$ | |
| 3.66 | cis-3-Propylchroman-4-yl | $COOCH_3$ | |
| 3.67 | trans-3-Propylchroman-4-yl | $COOCH_3$ | |

| Verb. No. | X | L | phys. Daten |
|---|---|---|---|
| 3.68 | 3-Ethyl-3-methyl-chroman-4-yl | $COOCH_3$ | |
| 3.69 | 3-Methyl-3-propyl-chroman-4-yl | $COOCH_3$ | |
| 3.70 | 3-Isopropylchroman-4-yl | $COOCH_3$ | |
| 3.71 | cis 3-Isopropylchroman-4-yl | $COOCH_3$ | |
| 3.72 | trans-3-Isopropylchroman-4-yl | $COOCH_3$ | |
| 3.73 | 3,3-Dimethylchroman-4-yl | $COOCH_3$ | |
| 3.74 | 3,3-Diethylchroman-4-yl | $COOCH_3$ | |
| 3.75 | 3,3-Dipropylchroman-4-yl | $COOCH_3$ | |
| 3.76 | Spiro[cyclopropan-1,3'-chroman]-4'-yl | $COOCH_3$ | |
| 3.77 | Spiro[cyclopentan-1,3'-chroman]-4'-yl | $COOCH_3$ | |
| 3.78 | Spiro[cyclohexan-1,3'-chroman]-4'-yl | $COOCH_3$ | |
| 3.79 | 2,2-Dimethylindan-1-yl | $COOC_2H_5$ | |
| 3.80 | 2,2-Dimethylindan-1-yl | $COO\text{-}i\text{-}C_3H_7$ | |
| 3.81 | 2,2-Dimethylindan-1-yl | $COO\text{-}CH_2\text{-}CH{=}CH_2$ | |
| 3.82 | 2,2-Dimethylindan-1-yl | COO— 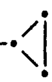 | |
| 3.83 | 2,2-Dimethylindan-1-yl | $CO\text{-}NH\ CH_3$ | |
| 3.84 | 2,2-Dimethylindan-1-yl | $CO\text{-}N(CH_3)_2$ | |
| 3.85 | 2,2-Dimethylindan-1-yl | CN | |
| 3.86 | 2,2-Dimethyl-1,2,3,4-tetrahydronaphthalin-1-yl | $COOC_2H_5$ | |
| 3.87 | 2,2-Dimethyl-1,2,3,4-tetrahydronaphthalin-1-yl | $COO\text{-}i\text{-}C_3H_7$ | |
| 3.88 | 2,2-Dimethyl-1,2,3,4-tetrahydronaphthalin-1-yl | $COO\text{-}CH_2\text{-}CH{=}CH_2$ | |
| 3.89 | 2,2-Dimethyl-1,2,3,4-tetrahydronaphthalin-1-yl | COO— 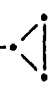 | |
| 3.90 | 2,2-Dimethyl-1,2,3,4-tetrahydronaphthalin-1-yl | $CO\text{-}NH\ CH_3$ | |
| 3.91 | 2,2-Dimethyl-1,2,3,4-tetrahydronaphthalin-1-yl | $CO\text{-}N(CH_3)_2$ | |

| Verb. No. | X | L | phys. Daten |
|---|---|---|---|
| 3.92 | 2,2-Dimethyl-1,2,3,4-tetra-hydronaphthalin-1-yl | CN | |
| 3.93 | 2,6-Dimethylphenyl | $COOC_2H_5$ | |
| 3.94 | 2,6-Dimethylphenyl | $COO-i-C_3H_7$ | |
| 3.95 | 2,6-Dimethylphenyl | $COO-CH_2-CH{=}CH_2$ | |
| 3.96 | 2,6-Dimethylphenyl | COO– 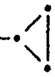 | |
| 3.97 | 2,6-Dimethylphenyl | $CO-NH\ CH_3$ | |
| 3.98 | 2,6-Dimethylphenyl | $CO-N(CH_3)_2$ | |
| 3.99 | 2,6-Dimethylphenyl | CN | |
| 3.100 | Diphenylmethyl | $COOC_2H_5$ | |
| 3.101 | Diphenylmethyl | $COO-i-C_3H_7$ | |
| 3.102 | Diphenylmethyl | $COO-CH_2-CH{=}CH_2$ | |
| 3.103 | Diphenylmethyl | COO– 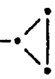 | |
| 3.104 | Diphenylmethyl | $CO-NH\ CH_3$ | |
| 3.105 | Diphenylmethyl | $CO-N(CH_3)_2$ | |
| 3.106 | Diphenylmethyl | CN | |
| 3.107 | 2-Methylchroman-4-yl | $COOCH_3$ | |
| 3.108 | cis-2-Methylchroman-4-yl | $COOCH_3$ | |
| 3.109 | trans-2-Methylchroman-4-yl | $COOCH_3$ | |
| 3.110 | 2-Ethylchroman-4-yl | $COOCH_3$ | |
| 3.111 | cis-2-Ethylchroman-4-yl | $COOCH_3$ | |
| 3.112 | trans-2-Ethylchroman-4-yl | $COOCH_3$ | |
| 3.113 | 2-Propylchroman-4-yl | $COOCH_3$ | |
| 3.114 | cis-2-Propylchroman-4-yl | $COOCH_3$ | |
| 3.115 | trans-2-Propylchroman-4-yl | $COOCH_3$ | |
| 3.116 | 2-Ethyl-2-methyl-chroman-4-yl | $COOCH_3$ | |
| 3.117 | 2-Methyl-2-propyl-chroman-4-yl | $COOCH_3$ | |
| 3.118 | 2-Isopropylchroman-4-yl | $COOCH_3$ | |
| 3.119 | cis 2-Isopropylchroman-4-yl | $COOCH_3$ | |
| 3.120 | trans-2-Isopropylchroman-4-yl | $COOCH_3$ | |
| 3.121 | 2,2-Dimethylchroman-4-yl | $COOCH_3$ | |

| Verb. No. | X | L | phys. Daten |
|---|---|---|---|
| 3.122 | 2,2-Diethylchroman-4-yl | $COOCH_3$ | |
| 3.123 | 2,2-Dipropylchroman-4-yl | $COOCH_3$ | |
| 3.124 | Spiro[cyclopropan-1,2'-chroman]-4'-yl | $COOCH_3$ | |
| 3.125 | Spiro[cyclopentan-1,2'-chroman]-4'-yl | $COOCH_3$ | |
| 3.126 | Spiro[cyclohexan-1,2'-chroman]-4'-yl | $COOCH_3$ | |
| 3.127 | 2,2-Dimethyl-1,2,3,4-tetrahydronaphthalin-1-yl | $CO-NH-i-C_3H_7$ | |
| 3.128 | Thiochroman-4-yl | $COOCH_3$ | |
| 3.129 | 2,2-Dimethylthiochroman-4-yl | $COOCH_3$ | |
| 3.130 | 3,3-Dimethylthiochroman-4-yl | $COOCH_3$ | |
| 3.131 | 2,3-Dimethylthiochroman-4-yl | $COOCH_3$ | |
| 3.132 | [cis-2,3-Dimethyl]thiochroman-4-yl | $COOCH_3$ | |
| 3.133 | [trans-2,3-Dimethyl]thiochroman-4-yl | $COOCH_3$ | |
| 3.134 | 2-Methylthiochroman-4-yl | $COOCH_3$ | |
| 3.135 | 2-Ethylthiochroman-4-yl | $COOCH_3$ | |
| 3.136 | 2-Methyl-2-ethylthiochroman-4-yl | $COOCH_3$ | |
| 3.137 | 2,2-Diethylthiochroman-4-yl | $COOCH_3$ | |
| 3.138 | 3,3-Diethylthiochroman-4-yl | $COOCH_3$ | |
| 3.139 | 3-Methyl-3-ethyl-thiochroman-4-yl | $COOCH_3$ | |
| 3.140 | 2,3-Dimethylindan-1-yl | $COOCH_3$ | |
| 3.141 | [cis-2,3-Dimethyl]indan-1-yl | $COOCH_3$ | |
| 3.142 | [trans-2,3-Dimethyl]indan 1-yl | $COOCH_3$ | |
| 3.143 | (+)-2,2-Dimethylindan-1-yl | $COOCH_3$ | $[\alpha]_D^{20}$:+54,18 ($CHCl_3$) |
| 3.144 | (-)-2,2-Dimethylindan-1-yl | $COOCH_3$ | Fp.100-105°C |
| 3.145 | (+)-2,2-Dimethyl-1,2,3,4-tetrahydronaphthalin-1-yl | $COOCH_3$ | |
| 3.146 | (-)-2,2-Dimethyl-1,2,3,4-tetrahydronaphthalin-1-yl | $COOCH_3$ | |

Herstellungsbeispiel 4 Diazoniumsalze der Formel VI

Beispiel 4.01: 5-Methoxycarbonyl-1-(2,2-dimethyl-1,2,3,4-tetrahydronaphth-1-yl)-imidazol-4-diazonium-tetrafluorborat

8,3 g der Verbindung No. 3.01 (0,028 Mol) werden in 100 ml Essigsäure/Propionsäure 1:1 gelöst und 24,6 g Borfluorwasserstoffsäure/($H_2O$ 50 % (0,14 Mol) zugetropft. Dann werden bei 0° 4,7 g $NaNO_2$ (0,067 Mol) eingetragen und weitere 5 Std. bei 0° gerührt. Dabei fällt das Produkt kristallin aus. Die Suspension wird über Nacht bei Raumtemperatur gelassen, mit Eiswasser verdünnt, das Produkt abgenutscht und über KOH getrocknet.

Man isoliert 8,9 g (97 %) der Titelverbindung der Formel

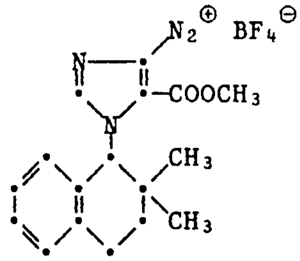

als farblose Kristalle mit Fp. 178°C (Zers.) (Verb. No. 4.01).

Beispiel 4.02: 1-(2,2-Dimethyl-indan-1-yl)-5-methoxycarbonyl-imidazol-4-diazonium-hexafluorophosphat

Analog zu Beispiel 4.01 erhält man aus 4-Amino-1-(2,2-dimethylindan-1-yl)-imidazol-5-carbonsäuremethylester und Hexafluorphosphorsäure die Titelverbindung der Formel

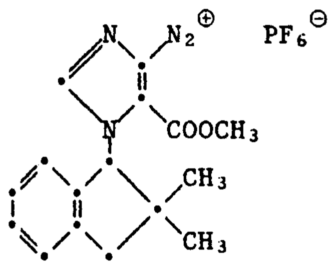

als Kristalle vom Smp. 172-174°C (Zers.).

Analog zu dem vorstehenden Beispiel können die Salze der Tabelle 4 erhalten werden.

Tabelle 4: Salze der Formel

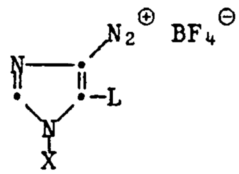

| Verb. No. | X | L | phys. Daten |
|---|---|---|---|
| 4.01 | 2,2-Dimethyl-1,2,3,4-tetrahydronaphthalin-1-yl | $COOCH_3$ | Fp.178°C (Zers.) |
| 4.02 | 2,2-Dimethyl-indan-1-yl | $COOCH_3$ | Fp.154-156°C (Zers.) |
| 4.03 | 2,6-Dimethylphenyl | $COOCH_3$ | |
| 4.04 | Diphenylmethyl | $COOCH_3$ | Fp.159-161°C |
| 4.05 | Indan-1-yl | $COOCH_3$ | |
| 4.06 | 2-Methyl-indan-1-yl | $COOCH_3$ | |
| 4.07 | cis-2-Methyl-indan-1-yl | $COOCH_3$ | |
| 4.08 | trans-2-Methyl-indan-1-yl | $COOCH_3$ | |
| 4.09 | 1,2,3,4-Tetrahydronaphthalin-1-yl | $COOCH_3$ | |
| 4.10 | 2-Methyl-1,2,3,4-tetrahydronaphthalin-1-yl | $COOCH_3$ | |
| 4.11 | cis-2-Methyl-1,2,3,4-tetrahydronaphthalin-1-yl | $COOCH_3$ | |
| 4.12 | trans-2-Methyl-1,2,3,4-tetrahydro-naphthalin-1-yl | $COOCH_3$ | |
| 4.13 | Benzocycloheptan-5-yl | $COOCH_3$ | |
| 4.14 | 4,5,6,7-Tetrahydrobenzo[b]-thiophen-4-yl | $COOCH_3$ | |
| 4.15 | 4,5,6,7-Tetrahydrobenzo[b]-furan-4-yl | $COOCH_3$ | |
| 4.16 | 5,6,7,8-Tetrahydrochinolin-5-yl | $COOCH_3$ | |
| 4.17 | 5,6,7,8-Tetrahydroisochinolin-5-yl | $COOCH_3$ | |
| 4.18 | 5,6,7,8-Tetrahydrochinolin-8-yl | $COOCH_3$ | |

| Verb. No. | X | L | phys. Daten |
|---|---|---|---|
| 4.19 | 5,6,7,8-Tetrahydroiso-chinolin-8-yl | $COOCH_3$ | |
| 4.20 | 9,10-Dihydroanthracen-9-yl | $COOCH_3$ | |
| 4.21 | 9H-Fluoren-9-yl | $COOCH_3$ | |
| 4.22 | Dibenzo[a,d]cyclohepten-5-yl | $COOCH_3$ | |
| 4.23 | Dibenzo[a,d]cycloheptan-5-yl | $COOCH_3$ | |
| 4.24 | 1,2-Dihydronaphthalin-1-yl | $COOCH_3$ | |
| 4.25 | (4-chlorphenyl)-(phenyl)-methyl | $COOCH_3$ | |
| 4.26 | 2-Ethyl-6-methyl-phenyl | $COOCH_3$ | |
| 4.27 | 2,6-Diethyl-phenyl | $COOCH_3$ | |
| 4.28 | 2,6-Di-isopropyl-phenyl | $COOCH_3$ | |
| 4.29 | 2,4,6-Trimethyl-phenyl | $COOCH_3$ | |
| 4.30 | 4-Chlor-2,6-dimethyl-phenyl | $COOCH_3$ | |
| 4.31 | 2-Ethyl-indan-1-yl | $COOCH_3$ | |
| 4.32 | cis-2-Ethyl-indan-1-yl | $COOCH_3$ | |
| 4.33 | trans-2-Ethyl-indan-1-yl | $COOCH_3$ | |
| 4.34 | 2-Propyl-indan-1-yl | $COOCH_3$ | |
| 4.35 | cis-Propyl-indan-1-yl | $COOCH_3$ | |
| 4.36 | trans-Propyl-indan-1-yl | $COOCH_3$ | |
| 4.37 | 2-Isopropyl-indan-1-yl | $COOCH_3$ | |
| 4.38 | 2-Ethyl-2-methyl-indan-1-yl | $COOCH_3$ | |
| 4.39 | 2-Methyl-2-propyl-indan-1-yl | $COOCH_3$ | |
| 4.40 | 2,2-Diethyl-indan-1-yl | $COOCH_3$ | |
| 4.41 | 2,2-Dipropyl-indan-1-yl | $COOCH_3$ | |
| 4.42 | Spiro-[cyclopropan-1,2'-indan]-1'-yl | $COOCH_3$ | |
| 4.43 | Spiro-[cyclopentan-1,2'-indan]-1'-yl | $COOCH_3$ | |
| 4.44 | Spiro-[cyclohexan-1,2'-indan]-1'-yl | $COOCH_3$ | |
| 4.45 | 2-Ethyl-1,2,3,4-tetrahydro-naphthalin-1-yl | $COOCH_3$ | |
| 4.46 | cis-2-Ethyl-1,2,3,4-tetra-hydronaphthalin-1-yl | $COOCH_3$ | |

| Verb. No. | X | L | phys. Daten |
|---|---|---|---|
| 4.47 | trans-2-Ethyl-1,2,3,4-tetrahydronaphthalin-1-yl | $COOCH_3$ | |
| 4.48 | 2-Propyl-1,2,3,4-tetrahydronaphthalin-1-yl | $COOCH_3$ | |
| 4.49 | cis-2-Propyl-1,2,3,4-tetrahydronaphthalin-1-yl | $COOCH_3$ | |
| 4.50 | trans-2-Propyl-1,2,3,4-tetrahydronaphthalin-1-yl | $COOCH_3$ | |
| 4.51 | 2-Ethyl-2-methyl-1,2,3,4-tetrahydronaphthalin-1-yl | $COOCH_3$ | |
| 4.52 | 2-Methyl-2-propyl-1,2,3,4-tetrahydronaphthalin-1-yl | $COOCH_3$ | |
| 4.53 | 2,2-Diethyl-1,2,3,4-tetrahydronaphthalin-1-yl | $COOCH_3$ | |
| 4.54 | 2,2-Dipropyl-1,2,3,4-tetrahydronaphthalin-1-yl | $COOCH_3$ | |
| 4.55 | Spiro[cyclopropan-1,2'-(1',2',3',4'-tetrahydronaphthalin)]-1'-yl | $COOCH_3$ | |
| 4.56 | Spiro[cyclopentan-1,2'-(1',2',3',4'-tetrahydronaphthalin)]-1'-yl | $COOCH_3$ | |
| 4.57 | Spiro[cyclohexan-1,2'-(1',2',3',4'-tetrahydronaphthalin)]-1'-yl | $COOCH_3$ | |
| 4.58 | Chroman-4-yl | $COOCH_3$ | |
| 4.59 | 3-Methylchroman-4-yl | $COOCH_3$ | |
| 4.60 | cis-3-Methylchroman-4-yl | $COOCH_3$ | |
| 4.61 | trans-3-Methylchroman-4-yl | $COOCH_3$ | |
| 4.62 | 3-Ethylchroman-4-yl | $COOCH_3$ | |
| 4.63 | cis-3-Ethylchroman-4-yl | $COOCH_3$ | |
| 4.64 | trans-3-Ethylchroman-4-yl | $COOCH_3$ | |
| 4.65 | 3-Propylchroman-4-yl | $COOCH_3$ | |
| 4.66 | cis-3-Propylchroman-4-yl | $COOCH_3$ | |
| 4.67 | trans-3-Propylchroman-4-yl | $COOCH_3$ | |
| 4.68 | 3-Ethyl-3-methyl-chroman-4-yl | $COOCH_3$ | |
| 4.69 | 3-Methyl-3-propyl-chroman-4-yl | $COOCH_3$ | |

| Verb. No. | X | L | phys. Daten |
|---|---|---|---|
| 4.70 | 3-Isopropylchroman-4-yl | $COOCH_3$ | |
| 4.71 | cis 3-Isopropylchroman-4-yl | $COOCH_3$ | |
| 4.72 | trans-3-Isopropylchroman-4-yl | $COOCH_3$ | |
| 4.73 | 3,3-Dimethylchroman-4-yl | $COOCH_3$ | |
| 4.74 | 3,3-Diethylchroman-4-yl | $COOCH_3$ | |
| 4.75 | 3,3-Dipropylchroman-4-yl | $COOCH_3$ | |
| 4.76 | Spiro[cyclopropan-1,3'-chroman]-4'-yl | $COOCH_3$ | |
| 4.77 | Spiro[cyclopentan-1,3'-chroman]-4'-yl | $COOCH_3$ | |
| 4.78 | Spiro[cyclohexan-1,3'-chroman]-4'-yl | $COOCH_3$ | |
| 4.79 | 2,2-Dimethylindan-1-yl | $COOC_2H_5$ | |
| 4.80 | 2,2-Dimethylindan-1-yl | $COO-i-C_3H_7$ | |
| 4.81 | 2,2-Dimethylindan-1-yl | $COO-CH_2-CH=CH_2$ | |
| 4.82 | 2,2-Dimethylindan-1-yl | COO— 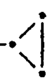 | |
| 4.83 | 2,2-Dimethylindan-1-yl | $CO-NH\ CH_3$ | |
| 4.84 | 2,2-Dimethylindan-1-yl | $CO-N(CH_3)_2$ | |
| 4.85 | 2,2-Dimethylindan-1-yl | CN | |
| 4.86 | 2,2-Dimethyl-1,2,3,4-tetrahydronaphthalin-1-yl | $COOC_2H_5$ | |
| 4.87 | 2,2-Dimethyl-1,2,3,4-tetrahydronaphthalin-1-yl | $COO-i-C_3H_7$ | |
| 4.88 | 2,2-Dimethyl-1,2,3,4-tetrahydronaphthalin-1-yl | $COO-CH_2-CH=CH_2$ | |
| 4.89 | 2,2-Dimethyl-1,2,3,4-tetrahydronaphthalin-1-yl | COO— 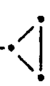 | |
| 4.90 | 2,2-Dimethyl-1,2,3,4-tetrahydronaphthalin-1-yl | $CO-NH\ CH_3$ | |
| 4.91 | 2,2-Dimethyl-1,2,3,4-tetrahydronaphthalin-1-yl | $CO-N(CH_3)_2$ | |
| 4.92 | 2,2-Dimethyl-1,2,3,4-tetrahydronaphthalin-1-yl | CN | |
| 4.93 | 2,6-Dimethylphenyl | $COOC_2H_5$ | |
| 4.94 | 2,6-Dimethylphenyl | $COO-i-C_3H_7$ | |

| Verb. No. | X | L | phys. Daten |
|---|---|---|---|
| 4.95 | 2,6-Dimethylphenyl | $COO-CH_2-CH=CH_2$ | |
| 4.96 | 2,6-Dimethylphenyl | COO— 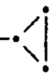 | |
| 4.97 | 2,6-Dimethylphenyl | $CO-NH\ CH_3$ | |
| 4.98 | 2,6-Dimethylphenyl | $CO-N(CH_3)_2$ | |
| 4.99 | 2,6-Dimethylphenyl | CN | |
| 4.100 | Diphenylmethyl | $COOC_2H_5$ | |
| 4.101 | Diphenylmethyl | $COO-i-C_3H_7$ | |
| 4.102 | Diphenylmethyl | $COO-CH_2-CH=CH_2$ | |
| 4.103 | Diphenylmethyl | COO— 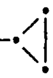 | |
| 4.104 | Diphenylmethyl | $CO-NH\ CH_3$ | |
| 4.105 | Diphenylmethyl | $CO-N(CH_3)_2$ | |
| 4.106 | Diphenylmethyl | CN | |
| 4.107 | 2-Methylchroman-4-yl | $COOCH_3$ | |
| 4.108 | cis-2-Methylchroman-4-yl | $COOCH_3$ | |
| 4.109 | trans-2-Methylchroman-4-yl | $COOCH_3$ | |
| 4.110 | 2-Ethylchroman-4-yl | $COOCH_3$ | |
| 4.111 | cis-2-Ethylchroman-4-yl | $COOCH_3$ | |
| 4.112 | trans-2-Ethylchroman-4-yl | $COOCH_3$ | |
| 4.113 | 2-Propylchroman-4-yl | $COOCH_3$ | |
| 4.114 | cis-2-Propylchroman-4-yl | $COOCH_3$ | |
| 4.115 | trans-2-Propylchroman-4-yl | $COOCH_3$ | |
| 4.116 | 2-Ethyl-2-methyl-chroman-4-yl | $COOCH_3$ | |
| 4.117 | 2-Methyl-2-propyl-chroman-4-yl | $COOCH_3$ | |
| 4.118 | 2-Isopropylchroman-4-yl | $COOCH_3$ | |
| 4.119 | cis 2-Isopropylchroman-4-yl | $COOCH_3$ | |
| 4.120 | trans-2-Isopropylchroman-4-yl | $COOCH_3$ | |
| 4.121 | 2,2-Dimethylchroman-4-yl | $COOCH_3$ | |
| 4.122 | 2,2-Diethylchroman-4-yl | $COOCH_3$ | |
| 4.123 | 2,2-Dipropylchroman-4-yl | $COOCH_3$ | |
| 4.124 | Spiro[cyclopropan-1,2'-chroman]-4'-yl | $COOCH_3$ | |

| Verb. No. | X | L | phys. Daten |
|---|---|---|---|
| 4.125 | Spiro[cyclopentan-1,2'-chroman]-4'-yl | $COOCH_3$ | |
| 4.126 | Spiro[cyclohexan-1,2'-chroman]-4'-yl | $COOCH_3$ | |
| 4.127 | 2,2-Dimethyl-1,2,3,4-tetrahydronaphthalin-1-yl | $CO-NH-i-C_3H_7$ | |
| 4.128 | Thiochroman-4-yl | $COOCH_3$ | |
| 4.129 | 2,2-Dimethylthiochroman-4-yl | $COOCH_3$ | |
| 4.130 | 3,3-Dimethylthiochroman-4-yl | $COOCH_3$ | |
| 4.131 | 2,3-Dimethylthiochroman-4-yl | $COOCH_3$ | |
| 4.132 | [cis-2,3-Dimethyl]thiochroman-4-yl | $COOCH_3$ | |
| 4.133 | [trans-2,3-Dimethyl]thiochroman-4-yl | $COOCH_3$ | |
| 4.134 | 2-Methylthiochroman-4-yl | $COOCH_3$ | |
| 4.135 | 2-Ethylthiochroman-4-yl | $COOCH_3$ | |
| 4.136 | 2-Methyl-2-ethyl-thiochroman-4-yl | $COOCH_3$ | |
| 4.137 | 2,2-Diethylthiochroman-4-yl | $COOCH_3$ | |
| 4.138 | 3,3-Diethylthiochroman-4-yl | $COOCH_3$ | |
| 4.139 | 3-Methyl-3-ethyl-thiochroman-4-yl | $COOCH_3$ | |
| 4.140 | 2,3-Dimethylindan-1-yl | $COOCH_3$ | |
| 4.141 | [cis-2,3-Dimethyl]indan-1-yl | $COOCH_3$ | |
| 4.142 | [trans-2,3-Dimethyl]indan-1-yl | $COOCH_3$ | |
| 4.143 | (+)-2,2-Dimethylindan-1-yl | $COOCH_3$ | |
| 4.144 | (-)-2,2-Dimethylindan-1-yl | $COOCH_3$ | |
| 4.145 | (+)-2,2-Dimethyl-1,2,3,4-tetrahydronaphthalin-1-yl | $COOCH_3$ | |
| 4.146 | (-)-2,2-Dimethyl-1,2,3,4-tetrahydronaphthalin-1-yl | $COOCH_3$ | |

Herstellungsbeispiel 5 Imidazole der Formel I

Beispiel 5.01: 1-(2,2-Dimethyl-1,2,3,4-tetrahydro-naphthalin-1-yl)-imidazol-5-carbonsäure-methylester

6,0 g der Verbindung 3.01 (0,02 Mol) werden in 30 ml Essigsäure/Propionsäure 1:1 gelöst und 11,5 g Phosphorsäure 85 % (0,1 Mol) zugetropft. Die Lösung wird auf 0° gekühlt und bei dieser Temperatur 1,4 g $NaNO_2$ (0,02 Mol) eingetragen, 6 Std. bei 0° gehalten und dann 13,2 g $H_3PO_2/H_2O$ 50 % (0,1 Mol) zugetropft. Die dabei entstandene gelbe Suspension wird 15 Std. bei 0° gehalten. Dabei bildet sich eine

gelbrote, klare Lösung. Der Ansatz wird auf 1 l Eiswasser gegeben, mit NaOH auf pH 7 gestellt und mit Essigester ausgeschüttelt. Die organische Phase wird über $Na_2SO_4$/Kohle getrocknet und das Lösungsmittel abdestilliert. Das zurückbleibende rote Oel wird mit Essigester/Hexan 1:2 über Kieselgel chromatografiert.

Man isoliert 3,3 g (59 %) der Titelverbindung der Formel

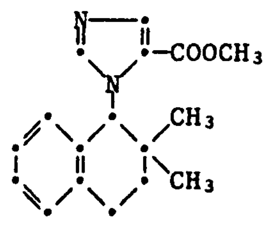

als farblose Kristalle mit Fp. 101-104 °C (Verb. No. 5.01).

Beispiel 5.02: 1-Diphenylmethyl-imidazol-5-carbonsäure-methylester

2,0 g der Verbindung 3,04 (6,5 mMol) werden in 20 ml Essigsäure/Propionsäure 1:1 gelöst und mit 4,9 g $HBF_5/H_2O$ 50 % (19,5 mMol) versetzt. Danach werden bei 0 °C 0,9 g $NaNO_2/H_2O$ 50 % (6,5 mMol) zugetropft. Die orangerote Lösung wird 3 Std. bei 0 °C gehalten und dann 4,3 g $H_3PO_2/H_2O$ 50 % (32,5 mMol) zugetropft. Die Lösung wird noch 48 Std. bei 0 ° gehalten, dann auf Eis/Wasser gegeben, mit NaOH auf pH 7 gestellt und mit Essigester ausgeschüttelt. Die organische Phase wird über $Na_2SO_4$/Kohle getrocknet, das Lösungsmittel abdestilliert und der Rückstand mit Essigester/Hexan 1:2 über Kieselgel chromatographiert.

Man isoliert 1,8 g (48 %) der Titelverbindung der Formel

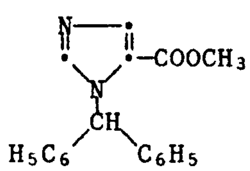

als farblose Kristalle mit Fp. 126-129 °C (Verb. No. 5.04).

Beispiel 5.03: Verfahren zur Herstellung von Verbindungen der Formel I aus Aminen der Formel VIII ohne Isolierung der Zwischenprodukte: 1-(2,2-Dimethylindan-1-yl)-imidazol-5-carbonsäuremethylester

Zu einer Lösung von 6,4 g 1-Amino-2,2-dimethylindan in 50 ml Dimethylformamid werden 3,9 g Ethoxymethylencyanamid gegeben. Dabei steigt die Temperatur auf 40 °C. Das Reaktionsgemisch wird 1 1/2 Stunden bei 80 °C gerührt, abgekühlt und bei Raumtemperatur am Hochvakuum das entstandene Ethanol abgesogen.
Dnn werden 13,8 g gemahlenes Kaliumcarbonat und eine Spatelspitze 18-crown-6 zugegeben und 30 Min. bei 80 °C gerührt, dann wird mit 6,7 g Bromessigsäuremethylester versetzt. Die Temperatur steigt dabei auf 87 °C. Nach 1-stündigem Rühren bei 80 °C wird das Reaktionsgemisch 15 Stunden bei 100 °C gehalten. Nach Erkalten auf Raumtemperatur werden die Salze abfiltriert und mit 10 ml DMF nachgewaschen. Diese Lösung wird in 30 Min zu einer Lösung von 6,8 g tert.Butylnitrit in 30 ml Dimethylformamid bei 60-62 °C zugetropft und 1 Stunde bei dieser Temperatur gehalten bis kein Stickstoff mehr entweicht. Die Lösung wird auf Eis gegossen, mit Essigester extrahiert. Die vereinigten Extrakte werden mit Sole gewaschen, über Natriumsulfat getrocknet, mit Aktivkohle entfärbt, über Hyflo filtriert und eingedampft. Der Rückstand wird Essigester/Hexan 1:1, an Kieselgel (150 g) gereinigt. Dabei erhält man 7 g eines gelben Oels, woraus durch Verreiben mt Hexan ein kristallines Produkt erhalten wird.

Man erhält 5,2 g der Titelverbindung der Formel

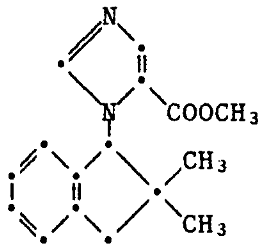

(Verb. Nr. 5.2)

als Kristalle vom Fp. 100-102 °C.

Beispiel 5.04: (+)-1-(2,2-Dimethyl-1,2,3,4-tetrahydronaphthalin-1-yl)-imidazol-5-carbonsäuremethylester

1,7 g tert.-Butylnitrit 90%-ig (0,015 Mol) werden in 20 ml N,N-Dimethyl-formamid gelöst. Dazu wird innerhalb von 30 Min. eine Lösung aus 2,6 g (+)-4-Amino-1-(2,2-dimethyl-1,2,3,4-tetrahydro-naphthalin-1-yl)-imidazol-5-carbonsäure-methylester (0,0087 Mol) in 20 ml N,N-Dimethyl-formamid bei 60-62 °C getropft und noch 70 Min. bei dieser Temperatur gehalten bis kein Stickstoff mehr entweicht. Die abgekühlte Lösung wird auf Eis gegossen und mit Essigsäure-ethylester extrahiert. Der Essigsäure-ethylester wird mit Sole gewaschen, über $Na_2SO_4$ getrocknet und filtriert. Nach dem Abdestillieren des Lösungsmittels erhält man 2,4 g eines braunen Oels (Rohprodukt), welches über eine Drucksäule (Kieselgel) mit Essigsäure-ethylester/Hexan 1:1 gereinigt wird.

Man isoliert 1,5 g (60 %) der Titelverbindung der Formel

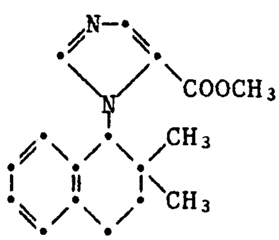

(+)

als Kristalle vom Fp. 66-67,5 °C und einem Drehwert von $[\alpha]_D^{20}$ : 64,8° (Verb. Nr. 5.145).

Beispiel 5.05: (-)-1-(2,2-Dimethylindan-1-yl)-imidazol-5-carbonsäuremethylester

Aus zwei getrennten Tropftrichtern werden gleichzeitig eine Lösung aus 11,0 g (-)-4-Amino-1-(2,2-dimethylindan-1-yl)-imidazol-5-carbonsäuremethylester in 17,2 g unterphosphoriger Säure (50 %) und 40 ml Dimethylformamid und eine Lösung von 2,7 g Natriumnitrit in 10 ml Wasser an einer Vorlage von 4,4 g Phosphorsäure (85 %) in 100 ml Wasser getropft. Es wird noch 2 Stunden bei 0 °C nachgerührt, in Wasser eingegossen und mit 30%-iger Natronlauge auf pH 7 gestellt. Die wässrige Phase wird mit Essigester extrahiert. Aus der organischen Phase isoliert man nach Trocknen, Einengen und Reinigen an Kieselgel mit Essigester/Hexan 10,5 g eines braunen Oels, welches beim Trocknen im Hochvakuum kristallisiert.

Man isoliert 7.1 g der Titelverbindung der Formel

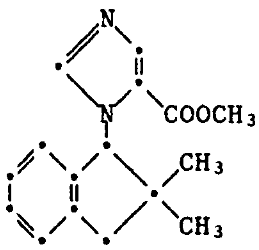

(–)

als rosa Kristalle vom Fp. 74-76 °C mit einem Drehwert: $[\alpha]_D^{20}$ : -66,5±0,4 °C ($CHCl_3$) (Verb. Nr. 5.144).

Analog zu den vorstehenden Beispielen 5.01 bis 5.05 können die Verbindungen der Tabelle 5 erhalten werden.

Tabelle 5: Verbindungen der Formel

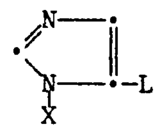

| Verb. No. | X | L | phys. Daten |
|---|---|---|---|
| 5.01 | 2,2-Dimethyl-1,2,3,4-tetrahydronaphthalin-1-yl | $COOCH_3$ | Fp.101-104°C |
| 5.02 | 2,2-Dimethyl-indan-1-yl | $COOCH_3$ | Fp.100-102°C |
| 5.03 | 2,6-Dimethylphenyl | $COOCH_3$ | Fp. 88- 91°C |
| 5.04 | Diphenylmethyl | $COOCH_3$ | Fp.126-129°C |
| 5.05 | Indan-1-yl | $COOCH_3$ | Fp. 140°C[1] |
| 5.06 | 2-Methyl-indan-1-yl | $COOCH_3$ | Fp.137-138°C[1] |
| 5.07 | cis-2-Methyl-indan-1-yl | $COOCH_3$ | Harz |
| 5.08 | trans-2-Methyl-indan-1-yl | $COOCH_3$ | Harz |
| 5.09 | 1,2,3,4-Tetrahydronaphthalin-1-yl | $COOCH_3$ | Fp. 63°C |
| 5.10 | 2-Methyl-1,2,3,4-tetrahydronaphthalin-1-yl | $COOCH_3$ | Fp. 90- 92°C |
| 5.11 | cis-2-Methyl-1,2,3,4-tetrahydronaphthalin-1-yl | $COOCH_3$ | Fp.101-102°C |
| 5.12 | trans-2-Methyl-1,2,3,4-tetrahydro-naphthalin-1-yl | $COOCH_3$ | Fp.110 - 110,5°C |
| 5.13 | Benzocycloheptan-5-yl | $COOCH_3$ | |
| 5.14 | 4,5,6,7-Tetrahydrobenzo[b]-thiophen-4-yl | $COOCH_3$ | Fp. 223,5°C[2] |
| 5.15 | 4,5,6,7-Tetrahydrobenzo[b]-furan-4-yl | $COOCH_3$ | |
| 5.16 | 5,6,7,8-Tetrahydrochinolin-5-yl | $COOCH_3$ | |
| 5.17 | 5,6,7,8-Tetrahydroisochinolin-5-yl | $COOCH_3$ | |

1 Die Verbindung wird als $HNO_3$-Salz (1:1) charakterisiert
2 Die Verbindung wird als HCl-Salz (1:1) charakterisiert

| Verb. No. | X | L | phys. Daten |
|---|---|---|---|
| 5.18 | 5,6,7,8-Tetrahydrochinolin-8-yl | $COOCH_3$ | |
| 5.19 | 5,6,7,8-Tetrahydroiso-chinolin-8-yl | $COOCH_3$ | |
| 5.20 | 9,10-Dihydroanthracen-9-yl | $COOCH_3$ | |
| 5.21 | 9H-Fluoren-9-yl | $COOCH_3$ | Fp. 146°C |
| 5.22 | Dibenzo[a,d]cyclohepten-5-yl | $COOCH_3$ | |
| 5.23 | Dibenzo[a,d]cycloheptan-5-yl | $COOCH_3$ | |
| 5.24 | 1,2-Dihydronaphthalin-1-yl | $COOCH_3$ | |
| 5.25 | (4-chlorphenyl)-(phenyl)-methyl | $COOCH_3$ | |
| 5.26 | 2-Ethyl-6-methyl-phenyl | $COOCH_3$ | Oel |
| 5.27 | 2,6-Diethyl-phenyl | $COOCH_3$ | |
| 5.28 | 2,6-Di-isopropyl-phenyl | $COOCH_3$ | |
| 5.29 | 2,4,6-Trimethyl-phenyl | $COOCH_3$ | |
| 5.30 | 4-Chlor-2,6-dimethyl-phenyl | $COOCH_3$ | |
| 5.31 | 2-Ethyl-indan-1-yl | $COOCH_3$ | |
| 5.32 | cis-2-Ethyl-indan-1-yl | $COOCH_3$ | |
| 5.33 | trans-2-Ethyl-indan-1-yl | $COOCH_3$ | |
| 5.34 | 2-Propyl-indan-1-yl | $COOCH_3$ | |
| 5.35 | cis-Propyl-indan-1-yl | $COOCH_3$ | |
| 5.36 | trans-Propyl-indan-1-yl | $COOCH_3$ | |
| 5.37 | 2-Isopropyl-indan-1-yl | $COOCH_3$ | |
| 5.38 | 2-Ethyl-2-methyl-indan-1-yl | $COOCH_3$ | Harz |
| 5.39 | 2-Methyl-2-propyl-indan-1-yl | $COOCH_3$ | |
| 5.40 | 2,2-Diethyl-indan-1-yl | $COOCH_3$ | Fp.83,5-85°C |
| 5.41 | 2,2-Dipropyl-indan-1-yl | $COOCH_3$ | |
| 5.42 | Spiro-[cyclopropan-1,2'-indan]-1'-yl | $COOCH_3$ | |
| 5.43 | Spiro-[cyclopentan-1,2'-indan]-1'-yl | $COOCH_3$ | Harz |
| 5.44 | Spiro-[cyclohexan-1,2'-indan]-1'-yl | $COOCH_3$ | |
| 5.45 | 2-Ethyl-1,2,3,4-tetrahydro-naphthalin-1-yl | $COOCH_3$ | |

| Verb. No. | X | L | phys. Daten |
|---|---|---|---|
| 5.46 | cis-2-Ethyl-1,2,3,4-tetrahydronaphthalin-1-yl | $COOCH_3$ | |
| 5.47 | trans-2-Ethyl-1,2,3,4-tetrahydronaphthalin-1-yl | $COOCH_3$ | |
| 5.48 | 2-Propyl-1,2,3,4-tetrahydronaphthalin-1-yl | $COOCH_3$ | Harz |
| 5.49 | cis-2-Propyl-1,2,3,4-tetrahydronaphthalin-1-yl | $COOCH_3$ | |
| 5.50 | trans-2-Propyl-1,2,3,4-tetrahydronaphthalin-1-yl | $COOCH_3$ | |
| 5.51 | 2-Ethyl-2-methyl-1,2,3,4-tetrahydronaphthalin-1-yl | $COOCH_3$ | |
| 5.52 | 2-Methyl-2-propyl-1,2,3,4-tetrahydronaphthalin-1-yl | $COOCH_3$ | |
| 5.53 | 2,2-Diethyl-1,2,3,4-tetrahydronaphthalin-1-yl | $COOCH_3$ | Fp.109-110°C |
| 5.54 | 2,2-Dipropyl-1,2,3,4-tetrahydronaphthalin-1-yl | $COOCH_3$ | |
| 5.55 | Spiro[cyclopropan-1,2'-(1',2',3',4'-tetrahydronaphthalin)]-1'-yl | $COOCH_3$ | |
| 5.56 | Spiro[cyclopentan-1,2'-(1',2',3',4'-tetrahydronaphthalin)]-1'-yl | $COOCH_3$ | Fp.153-154°C |
| 5.57 | Spiro[cyclohexan-1,2'-(1',2',3',4'-tetrahydronaphthalin)]-1'-yl | $COOCH_3$ | Fp.177-178°C |
| 5.58 | Chroman-4-yl | $COOCH_3$ | |
| 5.59 | 3-Methylchroman-4-yl | $COOCH_3$ | |
| 5.60 | cis-3-Methylchroman-4-yl | $COOCH_3$ | |
| 5.61 | trans-3-Methylchroman-4-yl | $COOCH_3$ | |
| 5.62 | 3-Ethylchroman-4-yl | $COOCH_3$ | |
| 5.63 | cis-3-Ethylchroman-4-yl | $COOCH_3$ | |
| 5.64 | trans-3-Ethylchroman-4-yl | $COOCH_3$ | |
| 5.65 | 3-Propylchroman-4-yl | $COOCH_3$ | |
| 5.66 | cis-3-Propylchroman-4-yl | $COOCH_3$ | |
| 5.67 | trans-3-Propylchroman-4-yl | $COOCH_3$ | |
| 5.68 | 3-Ethyl-3-methyl-chroman-4-yl | $COOCH_3$ | |

| Verb. No. | X | L | phys. Daten |
|---|---|---|---|
| 5.69 | 3-Methyl-3-propyl-chroman-4-yl | $COOCH_3$ | |
| 5.70 | 3-Isopropylchroman-4-yl | $COOCH_3$ | |
| 5.71 | cis 3-Isopropylchroman-4-yl | $COOCH_3$ | |
| 5.72 | trans-3-Isopropylchroman-4-yl | $COOCH_3$ | |
| 5.73 | 3,3-Dimethylchroman-4-yl | $COOCH_3$ | |
| 5.74 | 3,3-Diethylchroman-4-yl | $COOCH_3$ | |
| 5.75 | 3,3-Dipropylchroman-4-yl | $COOCH_3$ | |
| 5.76 | Spiro[cyclopropan-1,3'-chroman]-4'-yl | $COOCH_3$ | |
| 5.77 | Spiro[cyclopentan-1,3'-chroman]-4'-yl | $COOCH_3$ | |
| 5.78 | Spiro[cyclohexan-1,3'-chroman]-4'-yl | $COOCH_3$ | |
| 5.79 | 2,2-Dimethylindan-1-yl | $COOC_2H_5$ | Fp.124-125°C |
| 5.80 | 2,2-Dimethylindan-1-yl | $COO\text{-}i\text{-}C_3H_7$ | Fp.140-141°C |
| 5.81 | 2,2-Dimethylindan-1-yl | $COO\text{-}CH_2\text{-}CH{=}CH_2$ | |
| 5.82 | 2,2-Dimethylindan-1-yl | COO 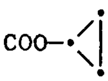 | |
| 5.83 | 2,2-Dimethylindan-1-yl | $CO\text{-}NH\ CH_3$ | |
| 5.84 | 2,2-Dimethylindan-1-yl | $CO\text{-}N(CH_3)_2$ | |
| 5.85 | 2,2-Dimethylindan-1-yl | CN | |
| 5.86 | 2,2-Dimethyl-1,2,3,4-tetrahydronaphthalin-1-yl | $COOC_2H_5$ | Fp. 74- 75°C |
| 5.87 | 2,2-Dimethyl-1,2,3,4-tetrahydronaphthalin-1-yl | $COO\text{-}i\text{-}C_3H_7$ | |
| 5.88 | 2,2-Dimethyl-1,2,3,4-tetrahydronaphthalin-1-yl | $COO\text{-}CH_2\text{-}CH{=}CH_2$ | |
| 5.89 | 2,2-Dimethyl-1,2,3,4-tetrahydronaphthalin-1-yl | COO 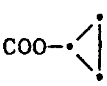 | |
| 5.90 | 2,2-Dimethyl-1,2,3,4-tetrahydronaphthalin-1-yl | $CO\text{-}NH\ CH_3$ | |
| 5.91 | 2,2-Dimethyl-1,2,3,4-tetrahydronaphthalin-1-yl | $CO\text{-}N(CH_3)_2$ | |
| 5.92 | 2,2-Dimethyl-1,2,3,4-tetrahydronaphthalin-1-yl | CN | |

| Verb. No. | X | L | phys. Daten |
|---|---|---|---|
| 5.93 | 2,6-Dimethylphenyl | $COOC_2H_5$ | |
| 5.94 | 2,6-Dimethylphenyl | $COO-i-C_3H_7$ | |
| 5.95 | 2,6-Dimethylphenyl | $COO-CH_2-CH=CH_2$ | |
| 5.96 | 2,6-Dimethylphenyl | COO— 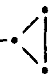 | |
| 5.97 | 2,6-Dimethylphenyl | $CO-NH\ CH_3$ | |
| 5.98 | 2,6-Dimethylphenyl | $CO-N(CH_3)_2$ | |
| 5.99 | 2,6-Dimethylphenyl | CN | |
| 5.100 | Diphenylmethyl | $COOC_2H_5$ | |
| 5.101 | Diphenylmethyl | $COO-i-C_3H_7$ | |
| 5.102 | Diphenylmethyl | $COO-CH_2-CH=CH_2$ | |
| 5.103 | Diphenylmethyl | COO— 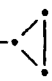 | |
| 5.104 | Diphenylmethyl | $CO-NH\ CH_3$ | |
| 5.105 | Diphenylmethyl | $CO-N(CH_3)_2$ | |
| 5.106 | Diphenylmethyl | CN | |
| 5.107 | 2-Methylchroman-4-yl | $COOCH_3$ | |
| 5.108 | cis-2-Methylchroman-4-yl | $COOCH_3$ | |
| 5.109 | trans-2-Methylchroman-4-yl | $COOCH_3$ | |
| 5.110 | 2-Ethylchroman-4-yl | $COOCH_3$ | |
| 5.111 | cis-2-Ethylchroman-4-yl | $COOCH_3$ | |
| 5.112 | trans-2-Ethylchroman-4-yl | $COOCH_3$ | |
| 5.113 | 2-Propylchroman-4-yl | $COOCH_3$ | |
| 5.114 | cis-2-Propylchroman-4-yl | $COOCH_3$ | |
| 5.115 | trans-2-Propylchroman-4-yl | $COOCH_3$ | |
| 5.116 | 2-Ethyl-2-methyl-chroman-4-yl | $COOCH_3$ | |
| 5.117 | 2-Methyl-2-propyl-chroman-4-yl | $COOCH_3$ | |
| 5.118 | 2-Isopropylchroman-4-yl | $COOCH_3$ | |
| 5.119 | cis 2-Isopropylchroman-4-yl | $COOCH_3$ | |
| 5.120 | trans-2-Isopropylchroman-4-yl | $COOCH_3$ | |
| 5.121 | 2,2-Dimethylchroman-4-yl | $COOCH_3$ | |
| 5.122 | 2,2-Diethylchroman-4-yl | $COOCH_3$ | |

| Verb. No. | X | L | phys. Daten |
|---|---|---|---|
| 5.123 | 2,2-Dipropylchroman-4-yl | $COOCH_3$ | |
| 5.124 | Spiro[cyclopropan-1,2'-chroman]-4'-yl | $COOCH_3$ | |
| 5.125 | Spiro[cyclopentan-1,2'-chroman]-4'-yl | $COOCH_3$ | |
| 5.126 | Spiro[cyclohexan-1,2'-chroman]-4'-yl | $COOCH_3$ | |
| 5.127 | 2,2-Dimethyl-1,2,3,4-tetrahydronaphthalin-1-yl | $CO-NH-i-C_3H_7$ | |
| 5.128 | Thiochroman-4-yl | $COOCH_3$ | |
| 5.129 | 2,2-Dimethylthiochroman-4-yl | $COOCH_3$ | |
| 5.130 | 3,3-Dimethylthiochroman-4-yl | $COOCH_3$ | |
| 5.131 | 2,3-Dimethylthiochroman-4-yl | $COOCH_3$ | |
| 5.132 | [cis-2,3-Dimethyl]thiochroman-4-yl | $COOCH_3$ | |
| 5.133 | [trans-2,3-Dimethyl]thiochroman-4-yl | $COOCH_3$ | |
| 5.134 | 2-Methylthiochroman-4-yl | $COOCH_3$ | |
| 5.135 | 2-Ethylthiochroman-4-yl | $COOCH_3$ | |
| 5.136 | 2-Methyl-2-ethyl-thiochroman-4-yl | $COOCH_3$ | |
| 5.137 | 2,2-Diethyl-thiochroman-4-yl | $COOCH_3$ | |
| 5.138 | 3,3-Diethyl-thiochroman-4-yl | $COOCH_3$ | |
| 5.139 | 3-Methyl-3-ethyl-thiochroman-4-yl | $COOCH_3$ | |
| 5.140 | 2,3-Dimethylindan-1-yl | $COOCH_3$ | |
| 5.141 | [cis-2,3-Dimethyl]indan-1-yl | $COOCH_3$ | |
| 5.142 | [trans-2,3-Dimethyl]indan-1-yl | $COOCH_3$ | |
| 5.143 | (+)-2,2-Dimethylindan-1-yl | $COOCH_3$ | |
| 5.144 | (-)-2,2-Dimethylindan-1-yl | $COOCH_3$ | Fp. 74- 76°C |
| 5.145 | (+)-2,2-Dimethyl-1,2,3,4-tetrahydronaphthalin-1-yl | $COOCH_3$ | Fp.66-67,5°C |
| 5.146 | (-)-2,2-Dimethyl-1,2,3,4-tetrahydronaphthalin-1-yl | $COOCH_3$ | |

## Patentansprüche

1. Verfahren zur Herstellung von Imidazolen der Formel I

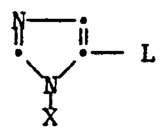

(I),

worin

| | |
|---|---|
| L und X | gemeinsam für $L^1$ und $X^1$; $L^2$ und $X^2$; $L^3$ und $X^3$; oder $L^4$ und $X^4$ stehen; und |
| $L^1$ | $COOR^1$; $CONR^2R^3$; $CONR^4NHR^3$; oder CN; |
| $R^1$ | Wasserstoff; $C_1$-$C_7$-Alkyl; $C_3$-$C_7$-Alkenyl; $C_3$-$C_7$-Alkinyl; $C_3$-$C_7$-Cycloalkyl; $C_1$-$C_7$-Alkoxy-$C_1$-$C_7$-alkyl; oder Aryl-$C_1$-$C_5$-alkyl; |
| | die Reste $R^2$, $R^3$ und $R^4$ unabhängig voneinander Wasserstoff; $C_1$-$C_5$-Alkyl; $C_3$-$C_5$-Alkenyl; $C_3$-$C_5$-Alkinyl; $C_3$-$C_7$-Cycloalkoxy; Aryl; oder durch Aryl, $C_3$-$C_7$-Cycloalkyl, $C_3$-$C_7$-Cycloalkoxy; $C_1$-$C_5$-Alkoxy, Hydroxy, Carboxyl oder $C_1$-$C_5$-Alkyloxycarbonyl substituiertes $C_1$-$C_5$-Alkyl; oder |
| $R_2$ und $R_3$ | gemeinsam mit dem Stickstoffatom, an welches sie gebunden sind einem unsubstituierten oder mit 1 bis 3 $C_1$-$C_5$-Alkylgruppen substituierten Piperidinyl, Pyrrolidinyl, 2-Oxopiperidinyl, 2-Oxopyrrolidinyl, Morpholinyl, Thiomorpholinyl, Piperazinyl oder 4-($C_1$-$C_4$)-Alkylpiperazinylring; |
| $X^1$ | 1-Indanyl, 1-Tetrahydronaphthalinyl, 5-Benzocycloheptanyl, 4-Tetrahydrobenzothienyl, 4-Tetrahydrobenzofuranyl, 5-Tetrahydrochinolinyl, 5-Tetrahydroisochinolinyl, 8-Tetrahydrochinolinyl, 8-Tetrahydroisochinolinyl, 9,10-Dihydro-9-anthracenyl, 9H-Fluoren-9-yl, 5-Dibenzo[a,d]cycloheptenyl, 5-Dibenzo[a,d]-cycloheptanyl oder 1-Dihydronaphthalinyl, welches unsubstituiert oder bis zu sechsfach gleich oder verschieden durch $C_1$-$C_5$-Alkyl, Aryl-$C_1$-$C_5$-alkyl, Di-Aryl-$C_1$-$C_5$-alkyl, $C_1$-$C_5$-Alkoxy, Halogen, $C_3$-$C_7$-Alkenyl, Amino, Nitro, $C_1$-$C_5$-Alkylcarbonylamino, Trifluormethyl oder Difluormethoxy substituiert ist oder in welchem zwei geminal gebundene Substituenten gemeinsam mit dem Kohlenstoffatom, an welches sie gebunden sind, eine $C_3$-$C_7$-Spirocycloalkylgruppe bilden oder in welchem zwei Substituenten gemeinsam für eine $C_1$-$C_5$-Alkylen- oder $C_5$-$C_7$-Cycloalkylengruppe stehen, wobei diese Alkylen- oder Cycloalkylengruppe ihrerseits gegebenenfalls bis zu zweifach unabhängig voneinander durch $C_1$-$C_5$-Alkyl, Aryl-$C_1$-$C_5$-alkyl, Di-Aryl-$C_1$-$C_5$-alkyl, $C_1$-$C_5$-Alkoxy, Halogen, $C_3$-$C_7$-Alkenyl, Trifluormethyl, Difluormethoxy oder Aryl substituiert sein kann; oder |
| $X^1$ | die Gruppe |

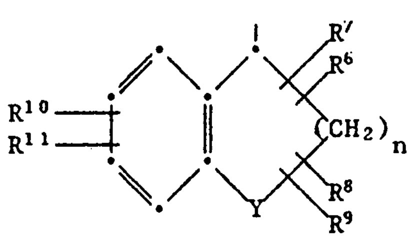

; oder

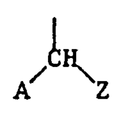

;

| | |
|---|---|
| n | null; eins; oder zwei; |
| Y | die Gruppen $-CH_2S(O)_m-$, oder $-CH_2-N(R^{12})-$ in welchen das Heteroatom an das benzolische Ringkohlenstoffatom gebunden ist und worin m für 0, 1 oder 2 steht, bedeutet; |
| $R^6$, $R^7$, $R^8$ und $R^9$ | unabhängig voneinander Wasserstoff; $C_1$-$C_5$-Alkyl; Aryl-$C_1$-$C_5$-alkyl; Di-Aryl-$C_1$-$C_5$-alkyl; $C_1$-$C_5$-Alkoxy; Halogen; $C_3$-$C_7$-Alkenyl; Trifluormethyl; Difluorme- |

thoxy; oder Aryl; oder

$R^6$ und $R^7$ gemeinsam einen annellierten Benzolrest, der gegebenenfalls bis zu zweifach durch $C_1$-$C_5$-Alkyl, $C_1$-$C_5$-Alkoxy, Halogen, ein- bis dreifach halogensubstituiertes $C_1$-$C_5$-Alkyl, ein- bis dreifach halogensubstituiertes $C_1$-$C_5$-Alkoxy, Nitro, Amino oder -NH-CO-M substituiert sein kann; oder

$R^6$ und $R^7$ zusammen mit dem Kohlenstoffatom, an welches sie geminal gebunden sind, einen spirocyclischen $C_3$-$C_7$-Ring; oder

$R^6$ und $R^7$ zusammen eine $C_1$-$C_5$-Alkylen- oder $C_5$-$C_7$-Cycloalkylengruppe, die gegebenenfalls bis zu zweifach unabhängig voneinander durch $C_1$-$C_5$-Alkyl, Aryl-$C_1$-$C_5$-alkyl, $C_1$-$C_5$-Alkoxy, Halogen, $C_3$-$C_7$-Alkenyl, Trifluormethyl, Difluormethoxy oder Aryl substituiert sein kann; und

$R^{10}$ und $R^{11}$ unabhängig voneinander Wasserstoff; $C_1$-$C_5$-Alkyl; $C_1$-$C_5$-Alkoxy; Halogen; Trifluormethyl; Difluormethoxy; Cyano; Nitro; Amino; Mono-$C_1$-$C_5$-alkylamino; Di-$C_1$-$C_5$-alkylamino; oder -NH-CO-M; und

$R^{12}$ Wasserstoff; $C_1$-$C_5$-Alkyl; $C_1$-$C_5$-Alkanoyl; oder 4-Methylphenylsulfonyl; und

A Wasserstoff; gegebenenfalls bis zu zweifach durch $C_1$-$C_5$-Alkyl substituiertes $C_3$-$C_7$-Cycloalkyl; gegebenenfalls durch $C_1$-$C_7$-Alkyloxy oder Aryl substituiertes $C_1$-$C_7$-Alkyl; durch ein $C_1$-$C_7$-Alkoxy und einen Arylrest substituiertes $C_1$-$C_7$-Alkyl; oder ein unsubstituiertes oder bis zu zweifach unabhängig voneinander durch $C_1$-$C_5$-Alkyl, $C_1$-$C_5$-Alkoxy, Halogen, Nitro, Amino, Mono-$C_1$-$C_5$-Alkylamino, Di-$C_1$-$C_5$-Alkylamino, -NH-CO-M, Cyano, Trifluormethyl oder Difluormethoxy substituiertes Pyridinyl, Pyrimidinyl, Naphthalinyl, Furanyl oder Thiophenyl;

wobei im Rahmen der Definition von A Aryl für Phenyl, Pyridinyl, Pyrimidinyl, Naphthalinyl, Furanyl oder Thiophenyl steht und in der dieser Rest bis zu zweifach, im Falle von Aryl = Phenyl auch bis zu dreifach, unabhängig voneinander durch $C_1$-$C_5$-Alkyl, $C_1$-$C_5$-Alkoxy, Halogen, Nitro, Amino, Mono-$C_1$-$C_5$-alkylamino, Di-$C_1$-$C_5$-alkylamino, -NH-CO-M, Cyano, Trifluormethyl oder Difluormethoxy substituiert sein kann; und

Z unsubstituiertes oder bis zu dreifach unabhängig voneinander durch $C_1$-$C_5$-Alkyl, $C_1$-$C_5$-Alkoxy, Halogen, Cyano, Nitro, Amino, Mono-$C_1$-$C_5$-alkylamino, Di-$C_1$-$C_5$-alkylamino, -NH-CO-M, Trifluormethyl oder Difluormethoxy substituiertes Naphthalinyl, Thiophenyl, Furanyl, Pyrimidinyl, Phenyl oder Pyridinyl; und

M $C_1$-$C_5$-Alkyl; und

Aryl im Rahmen vorstehender Definitionen der Reste X und L ausserdem einen unsubstituierten oder bis zu dreifach gleich oder verschieden durch $C_1$-$C_5$-Alkyl, $C_1$-$C_5$-Alkyloxy oder Halogen substituierten Phenylrest; und

$L^2$ CO-D-$R^{13}$;

D O; oder $NR^{13}$;

$R^{13}$ Wasserstoff; Phenyl; $C_3$-$C_6$-Alkenyl; unsubstituiertes oder bis zu dreifach gleich oder unterschiedlich durch $C_1$-$C_6$-Alkoxy, $C_1$-$C_3$-Dialkylamino oder Halogen substituiertes $C_1$-$C_{12}$-Alkyl; und

$R^{13}$ wenn D für 0 steht auch ein Kation eines Metalls der I., II. oder VII. Gruppe des Periodensystems oder Ammonium; und

$X^2$ einen Rest

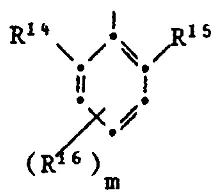

m die Zahlen 0, 1, 2 oder 3; und

$R^{14}$ und $R^{15}$ unabhängig voneinander $C_1$-$C_4$-Alkyl;

$R^{16}$ gleiche oder verschiedene Reste aus der Gruppe $C_1$-$C_4$-Alkyl, $C_1$-$C_4$-Alkoxy

und Halogen;

$L^3$ CN; $COOR^{17}$; oder $CONR^{18}R^{19}$; und

$R^{17}$ unsubstituiertes oder durch Halogen substituiertes $C_1$-$C_7$-Alkyl, $C_3$-$C_7$-Alkenyl, $C_3$-$C_7$-Alkinyl, $C_1$-$C_7$-Alkoxy-$C_1$-$C_7$-alkyl, $C_3$-$C_7$-Cycloalkyl oder Aryl-$C_1$-$C_5$-alkyl; und

$R^{18}$ und $R^{19}$, die gleich oder verschieden sein können, Waserstoff; $C_1$-$C_4$-Alkyl; $C_3$-$C_7$-Alkenyl; $C_3$-$C_7$-Alkinyl; $C_1$-$C_7$-Alkoxy-$C_1$-$C_7$-alkyl; oder

$R^{18}$ und $R^{19}$ gemeinsam mit dem Stickstoffatom, an das sie gebunden sind, einen gesättigten oder ungesättigten drei- bis siebengliedrigen Ring, der bis zu drei Heteroatome aus der Gruppe O, N und S enthält und der unsubstituiert oder durch ($C_1$-$C_4$)-Alkyl oder Halogen substituiert ist und eine Carbonylgruppe enthalten kann; und

$X^3$ einen Rest

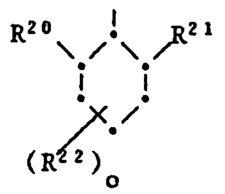

oder

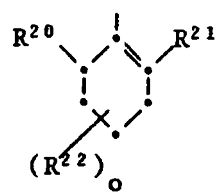

;

o 0 bis 6;

$R^{20}$ und $R^{21}$ unabhängig voneinander Wasserstoff oder $C_1$-$C_4$-Alkyl; und

$R^{22}$ jeweils unabhängig voneinander $C_1$-$C_4$-Alkyl, $C_1$-$C_4$-Alkoxy; $C_1$-$C_4$-Hydroxyalkyl; $C_1$-$C_4$-Alkoxy-$C_1$-$C_4$-alkyl; oder Halogen; und

$L^4$ CO-$R^{23}$; oder CN;

$R^{23}$ Hydroxy; $C_1$-$C_6$-Alkoxy; Hydroxy-($C_2$-$C_6$)-alkoxy; ($C_2$-$C_6$)-Alkoxyalkoxy; Amino; $C_1$-$C_6$-Alkylamino; Di-($C_1$-$C_6$)-Alkylamino; Di-($C_1$-$C_6$)-Alkylamino-($C_1$-$C_3$)-alkylamino; $C_1$-$C_3$-Alkoxyamino; Anilino; N-Pyrrolidino; N-Piperidino; N-Morpholino; Hydrazino; N'-($C_1$-$C_3$)-Alkylhydrazino; N,N'-Dimethylhydrazino; oder N'-Phenylhydrazino; und

$X^4$ den Rest

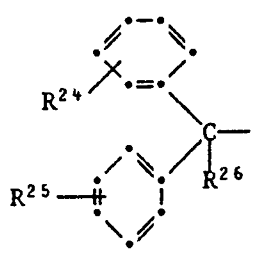

und

$R^{24}$ und $R^{25}$ die gleich oder verschiedene sein können Wasserstoff; Halogen, $C_1$-$C_3$-Alkyl, Trifluormethyl; Hydroxy; $C_1$-$C_3$-Alkoxy; Halogen-($C_1$-$C_3$)-alkoxy; $C_1$-$C_3$-Alkylthio; Cyano; Nitro; oder Acetamido; und

$R^{26}$ Wasserstoff; oder Phenyl;

bedeutet, dadurch gekennzeichnet, dass man N-Cyanoformamidine der Formel II

X - NH - CH = N - CN (II),

worin X wie zuvor definiert ist, mit einer Verbindung der Formel III

Z - $CH_2$ - L (III),

worin L wie zuvor definiert ist und Z für eine nucleofuge Gruppe steht zu einer Verbindung der Formel IV

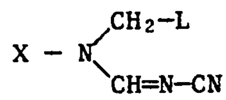

(IV)

N-alkyliert und IV unter Baseneinwirkung zu einem 4-Aminoimidazol der Formel V

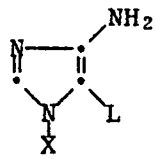

(V)

cyclisiert und das Aminoimidazol V zu einem Imidazol der Formel I reduziert.

**2.** N-Cyanoformamidine der Formel II,

X - NH - CH = N - CN (II),

worin der Rest X wie im Anspruch 1 definiert ist.

**3.** N,N-Disubstituierte N'-Cyanoformamidine der Formel IV

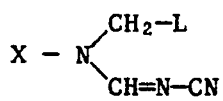

(IV),

worin die Reste X und L wie im Anspruch 1 definiert sind.

**4.** 4-Aminoimidazole der Formel V

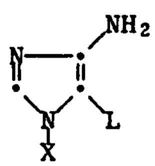

(V),

worin die Reste X und L wie im Anspruch 1 definiert sind.

**5.** Verfahren zur Herstellung von Imidazolen der Formel I

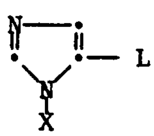

(I),

worin die Reste X und L wie im Anspruch 1 definiert sind, dadurch gekennzeichnet, dass man ein Aminoimidazol der Formel V

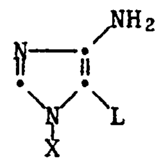

(V)

worin X und L wie zuvor definiert sind, in ein Diazoniumsalz der Formel VI,

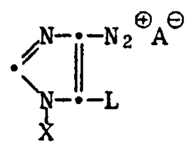

(VI),

in dem X und L wie zuvor definiert sind und $A^{\ominus}$ für ein Aequivalent eines Anions steht, überführt und unter Stickstoffabspaltung zu einer Verbindung der Formel I reduziert.

**6.** Verfahren gemäss Anspruch 5, dadurch gekennzeichnet, dass man die Diazotierung mit einem Alkylnitrit der Formel XIII

Alk—O—NO (XIII),

worin Alk für $C_1$-$C_8$-Alkyl steht, ohne Isolierung des Diazoniumsalzes VI in Gegenwart eines Reduktionsmittels durchführt.

**7.** Verfahren nach Anspruch 6, dadurch gekennzeichnet, dass man als Reduktionsmittel Dimethylformamid, Dioxan, Tetrahydrofuran oder einen $C_1$-$C_6$-Alkohol verwendet.

**8.** Verfahren gemäss Anspruch 5, dadurch gekennzeichnet, dass man die Diazotierung mit einem Alkalinitrit in Gegenwart einer Mineralsäure durchführt.

**9.** Diazoniumsalze der Formel VI,

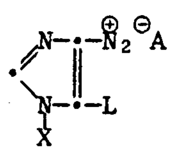

(VI),

worin die Reste X und L wie im Anspruch 1 definiert sind und $A^{\ominus}$ ein Aequivalent eines Anions bedeutet.

**10.** Verfahren zur Herstellung von Verbindungen der Formel II gemäss Anspruch 2, dadurch gekennzeichnet, dass man

a) ein Amin der Formel VIII

X - $NH_2$ (VIII)

mit einem Orthoester der Formel X

$CH(OD)_3$ (X)

worin D für $C_1$-$C_4$-Alkyl oder Phenyl steht mit einem Cyanamid der Formel IX

$H_2NCN$ (IX)

kondensiert, oder
b) ein Amin der Formel VIII

$X - NH_2$ (VIII)

mit einem Imidoester der Formel X

DO - CH = N - CN (X),

in dem D für eine $C_1$-$C_4$-Alkylgruppe oder für Phenyl steht, kondensiert oder
c) einen Imidoester der Formel XI

X - N = CH - OD (XI),

in dem D für eine $C_1$-$C_4$-Alkylgruppe oder für Phenyl steht, mit Cyanamid der Formel IX

$H_2NCN$ (IX)

kondensiert.

**11.** Verfahren zur Herstellung von N,N-disubstituierten N'-Cyanoformamidinen der Formel IV gemäss Anspruch 3, dadurch gekennzeichnet, dass man
a) ein N-Cyanoformamidin der Formel II

X - NH - CH = N - CN (II),

mit einer Verbindung der Formel III

$Z - CH_2 - L$ (III),

in der Z für eine nucleofuge Gruppe steht, in Gegenwart einer Base umsetzt oder
b) in einem ersten Reaktionsschritt das N-Cyanoformamidin der Formel II

X - NH - CH = N - CN (II),

mittels einer Base zu einem Salze der Formel VII

$$X - \overset{\ominus}{N}(B^{\oplus}) - CH = N - CN \qquad (VII),$$

in dem $B^{\oplus}$ für ein Kationenäquivalent steht, deprotoniert und danach mit einer Verbindung der Formel III

$Z - CH_2 - L$ (III),

worin Z für eine nucleofuge Gruppe steht, alkyliert.

**12.** Verfahren zur Herstellung von 4-Aminoimidazolen der Formel V gemäss Anspruch 4, dadurch gekennzeichnet, dass man ein N,N-disubstituiertes N'-Cyanoformamidin der Formel IV unter Baseneinwirkung cyclisiert.

**13.** Verfahren zur Herstellung von Diazoniumsalzen der Formel VI gemäss Anspruch 9, dadurch gekennzeichnet, dass man im 4-Aminoimidazol der Formel V diazotiert.

**14.** Die Verwendung von Verbindungen der Formeln II, IV, V oder VI gemäss den Ansprüchen 2 bis 4 oder 9 zur Durchführung eines Verfahrens gemäss Anspruch 1, 5 oder 10 bis 13.

**15.** Verfahren zur Herstellung von Imidazolen der Formel I

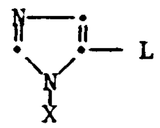

(I),

worin X und L wie in Anspruch 1 definiert sind, dadurch gekennzeichnet, dass man ein Amin der Formel VIII

X—$NH_2$ (VIII),

worin X wie zuvor definiert ist, mit einem Imidoester der Formel XI

DO-CH = N-CN (XI),

worin D für $C_1$-$C_4$-Alkyl oder Phenyl steht, zu einer Verbindung der Formel II

X-NH-CH = N-CN (II),

kondensiert und diese dann mit einer Verbindung der Formel III

Z-$CH_2$-L (III),

worin L wie zuvor definiert ist und R für eine nucleofuge Gruppe steht, zu einer Verbindung der Formel IV

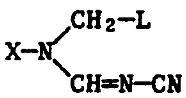

(IV),

kondensiert und diese dann unter Baseneinwirkung zu einem 4-Aminoimidazol der Formel V

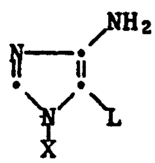

(V)

cyclisiert und anschliessend das so erhältliche Aminoimidazol V mit einem Alkylnitrit der Formel XIII

Alk—O—NO (III),

worin Alk für $C_1$-$C_8$-Alkyl steht, in Gegenwart eines geeigneten Reduktionsmittels zu einer Verbindung der Formel I deaminiert.

**16.** Verfahren gemäss Anspruch 11, dadurch gekennzeichnet, dass man die Umsetzung ohne Isolierung der Zwischenprodukte durchführt.

## Claims

**1.** A process for the preparation of an imidazole of the formula I

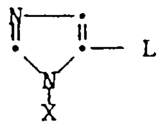

(I)

in which

L and X represent $L^1$ and $X^1$; $L^2$ and $X^2$; $L^3$ and $X^3$; or $L^4$ and $X^4$; respectively, and

$L^1$ represents $COOR^1$; $CONR^2R^3$; $CONR^4NHR^3$; or CN;

$R^1$ represents hydrogen; $C_1$-$C_7$alkyl; $C_3$-$C_7$alkenyl; $C_3$-$C_7$alkynyl; $C_3$-$C_7$cycloalkyl; $C_1$-$C_7$alkoxy-$C_1$-$C_7$alkyl; or aryl-$C_1$-$C_5$alkyl;

the radicals $R^2$, $R^3$ and $R^4$, independently of one another, each represents hydrogen; $C_1$-$C_5$alkyl; $C_3$-$C_5$alkenyl; $C_3$-$C_5$alkynyl; $C_3$-$C_7$cycloalkyl; aryl; or $C_1$-$C_5$alkyl substituted by aryl, $C_3$-$C_7$cycloalkyl, $C_3$-$C_7$cycloalkoxy, $C_1$-$C_5$-alkoxy, hydroxy, carboxy or by $C_1$-$C_5$alkoxycarbonyl; or

$R^2$ and $R^3$, together with the nitrogen atom to which they are bonded, represent a piperidinyl, pyrrolidinyl, 2-oxopiperidinyl, 2-oxopyrrolidinyl, morpholinyl, thiomorpholinyl, piperazinyl or 4-($C_1$-$C_4$)alkylpiperazinyl ring that is unsubstituted or is substituted by from 1 to 3 $C_1$-$C_5$alkyl groups;

$X^1$ represents 1-indanyl, 1-tetrahydronaphthalenyl, 5-benzocycloheptanyl, 4-tetrahydrobenzothienyl, 4-tetrahydrobenzofuranyl, 5-tetrahydroquinolinyl, 5-tetrahydroisoquinolinyl, 8-tetrahydroquinolinyl, 8-tetrahydroisoquinolinyl, 9,10-dihydro-9-anthracenyl, 9H-fluoren-9-yl, 5-dibenzo[a,d]cycloheptenyl, 5-dibenzo[a,d]cycloheptanyl or 1-dihydronaphthalenyl, each of which is unsubstituted or is substituted up to six times by the same or different substituents selected from $C_1$-$C_5$alkyl, aryl-$C_1$-$C_5$alkyl, diaryl-$C_1$-$C_5$alkyl, $C_1$-$C_5$alkoxy, halogen, $C_3$-$C_7$alkenyl, amino, nitro, $C_1$-$C_5$alkylcarbonylamino, trifluoromethyl and difluoromethoxy, or in which two geminally bonded substituents, together with the carbon atom to which they are bonded, form a $C_3$-$C_7$spirocycloalkyl group, or in which two substituents together represent a $C_1$-$C_5$alkylene or $C_5$-$C_7$cycloalkylene group, it being possible for this alkylene or cycloalkylene group in turn optionally to be substituted up to twice by the same or different substituents selected from $C_1$-$C_5$alkyl, aryl-$C_1$-$C_5$alkyl, diaryl-$C_1$-$C_5$alkyl, $C_1$-$C_5$alkoxy, halogen, $C_3$-$C_7$alkenyl, trifluoromethyl, difluoromethoxy and aryl; or

$X^1$ represents the group

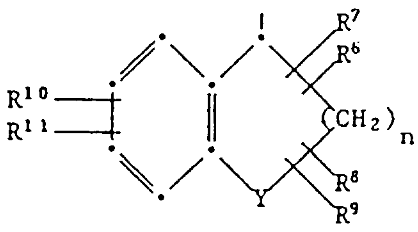

; or

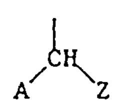

;

n represents 0; 1; or 2;

Y represents a group $-CH_2S(O)_m-$ or $-CH_2-N(R^{12})-$ in which the hetero atom is bonded to the benzene ring carbon atom and in which m represents 0, 1 or 2;

$R^6$, $R^7$, $R^8$ and $R^9$, independently of one another, each represents hydrogen; $C_1$-$C_5$alkyl; aryl-$C_1$-$C_5$alkyl; diaryl-$C_1$-$C_5$alkyl, $C_1$-$C_5$alkoxy; halogen; $C_3$-$C_7$alkenyl; trifluoromethyl; difluoromethoxy; or aryl; or

$R^6$ and $R^7$ together represent a fused benzene radical which can optionally be substituted up to twice by $C_1$-$C_5$alkyl, $C_1$-$C_5$alkoxy, halogen, mono- to tri-halo-substituted $C_1$-$C_5$alkyl, mono- to tri-halo-substituted $C_1$-$C_5$alkoxy, nitro, amino or by -NH-CO-M; or

| | |
|---|---|
| $R^6$ and $R^7$, | together with the carbon atom to which they are geminally bonded, represent a spirocyclic $C_3$-$C_7$-ring; or |
| $R^6$ and $R^7$ | together represent a $C_1$-$C_5$alkylene or $C_5$-$C_7$cycloalkylene group which can optionally be substituted up to twice by the same or different substituents selected from $C_1$-$C_5$alkyl, aryl-$C_1$-$C_5$alkyl, $C_1$-$C_5$alkoxy, halogen, $C_3$-$C_7$alkenyl, trifluoromethyl, difluoromethoxy and aryl; and |
| $R^{10}$ and $R^{11}$, | independently of one another, each represents hydrogen; $C_1$-$C_5$alkyl; $C_1$-$C_5$alkoxy; halogen; trifluoromethyl; difluoromethoxy; cyano; nitro; amino; mono-$C_1$-$C_5$alkylamino; di-$C_1$-$C_5$alkylamino; or -NH-CO-M; and |
| $R^{12}$ | represents hydrogen; $C_1$-$C_5$alkyl; $C_1$-$C_5$alkanoyl; or 4-methylphenylsulphonyl; and |
| A | represents hydrogen; $C_3$-$C_7$cycloalkyl optionally substituted up to twice by $C_1$-$C_5$alkyl; $C_1$-$C_7$alkyl optionally substituted by $C_1$-$C_7$alkoxy or by aryl; $C_1$-$C_7$alkyl substituted by a $C_1$-$C_7$alkoxy and by an aryl radical; or pyridinyl, pyrimidinyl, naphthalenyl, furanyl or thiophenyl, each of which is unsubstituted or is substituted up to twice by the same or different substituents selected from $C_1$-$C_5$alkyl, $C_1$-$C_5$alkoxy, halogen, nitro, amino, mono-$C_1$-$C_5$-alkylamino, di-$C_1$-$C_5$alkylamino, -NH-CO-M, cyano, trifluoromethyl and difluoromethoxy; |
| | and within the scope of the definition of A, aryl represents phenyl, pyridinyl, pyrimidinyl, naphthalenyl, furanyl or thiophenyl, and this radical can be substituted up to twice, or in the case when aryl is phenyl up to three times, by the same or different substituents selected from $C_1$-$C_5$alkyl, $C_1$-$C_5$alkoxy, halogen, nitro, amino, mono-$C_1$-$C_5$alkylamino, di-$C_1$-$C_5$alkylamino, -NH-CO-M, cyano, trifluoromethyl and difluoromethoxy; and |
| Z | represents naphthalenyl, thiophenyl, furanyl, pyrimidinyl, phenyl or pyridinyl, each of which is unsubstituted or is substituted up to three times by the same or different substituents selected from $C_1$-$C_5$alkyl, $C_1$-$C_5$alkoxy, halogen, cyano, nitro, amino, mono-$C_1$-$C_5$alkylamino, di-$C_1$-$C_5$alkylamino, -NH-CO-M, trifluoromethyl and difluoromethoxy; and |
| M | represents $C_1$-$C_5$alkyl; and |
| | aryl within the scope of the above definitions of the radicals X and L may also represent a phenyl radical that is unsubstituted or is substituted up to three times by the same or different substituents selected from $C_1$-$C_5$alkyl, $C_1$-$C_5$alkoxy and halogen; and |
| $L^2$ | represents CO-D-$R^{13}$; |
| D | represents O; or $NR^{13}$; |
| $R^{13}$ | represents hydrogen; phenyl; $C_3$-$C_6$alkenyl; or $C_1$-$C_{12}$alkyl that is unsubstituted or is substituted up to three times by the same or different substituents selected from $C_1$-$C_6$alkoxy, $C_1$-$C_3$dialkylamino and halogen; and |
| $R^{13}$, | when D is O, additionally represents a cation of a metal of group I, II or VII of the Periodic Table or ammonium; and |
| $X^2$ | represents a radical |

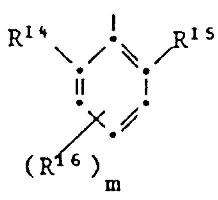

| | |
|---|---|
| m | represents 0, 1, 2 or 3; and |
| $R^{14}$ and $R^{15}$, | independently of one another, each represents $C_1$-$C_4$alkyl; |

R[16] represents the same or different radicals selected from the group comprising $C_1$-$C_4$alkyl, $C_1$-$C_4$alkoxy and halogen;

$L^3$ represents CN; $COOR^{17}$; or $CONR^{18}R^{19}$; and

$R^{17}$ represents unsubstituted or halo-substituted $C_1$-$C_7$-alkyl, $C_3$-$C_7$alkenyl, $C_3$-$C_7$alkynyl, $C_1$-$C_7$alkoxy-$C_1$-$C_7$alkyl, $C_3$-$C_7$cycloalkyl or aryl-$C_1$-$C_5$alkyl; and

$R^{18}$ and $R^{19}$, which may be the same or different, represent hydrogen; $C_1$-$C_4$alkyl; $C_3$-$C_7$alkenyl; $C_3$-$C_7$alkynyl; or $C_1$-$C_7$alkoxy-$C_1$-$C_7$alkyl; or

$R^{18}$ and $R^{19}$, together with the nitrogen atom to which they are bonded, represent a saturated or unsaturated three- to seven-membered ring that contains up to three hetero atoms selected from the group comprising O, N and S and that is unsubstituted or is substituted by ($C_1$-$C_4$)alkyl or by halogen and may contain a carbonyl group; and

$X^3$ represents a radical

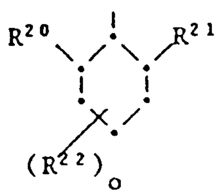

or

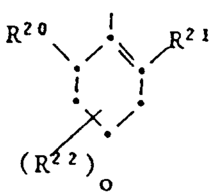

;

o represents from 0 to 6;

$R^{20}$ and $R^{21}$, independently of one another, each represents hydrogen or $C_1$-$C_4$alkyl; and

$R^{22}$ represents, independently of any other, $C_1$-$C_4$alkyl; $C_1$-$C_4$alkoxy; $C_1$-$C_4$hydroxyalkyl; $C_1$-$C_4$alkoxy-$C_1$-$C_4$-alkyl; or halogen; and

$L^4$ represents CO-$R^{23}$; or CN;

$R^{23}$ represents hydroxy; $C_1$-$C_6$alkoxy; hydroxy-($C_2$-$C_6$)alkoxy; ($C_2$-$C_6$)-alkoxyalkoxy; amino; $C_1$-$C_6$alkylamino; di($C_1$-$C_6$)alkylamino; di($C_1$-$C_6$)-alkylamino-($C_1$-$C_3$)alkylamino; $C_1$-$C_3$alkoxyamino; anilino; N-pyrrolidino; N-piperidino; N-morpholino; hydrazino; N'-($C_1$-$C_3$)alkylhydrazino; N,N'-dimethylhydrazino; or N'-phenylhydrazino; and

$X^4$ represents the radical

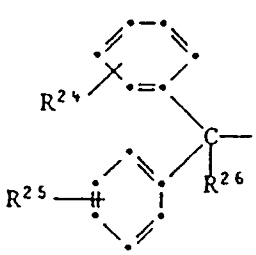

and

$R^{24}$ and $R^{25}$, which may be the same or different, represent hydrogen; halogen; $C_1$-$C_3$alkyl; trifluoromethyl; hydroxy; $C_1$-$C_3$alkoxy; halo-($C_1$-$C_3$)alkoxy; $C_1$-$C_3$alkylthio; cyano; nitro; or acetamido; and

$R^{26}$ represents hydrogen; or phenyl;

characterised in that an N-cyanoformamidine of the formula II

X - NH - CH = N - CN (II),

in which X has the definition given above, is N-alkylated with a compound of the formula III

Z - $CH_2$ - L (III),

in which L has the definition given above and Z represents a nucleofugal group, to form a compound of

the formula IV

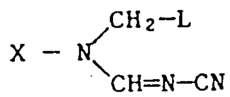

(IV),

and IV is cyclised under the action of a base to form a 4-aminoimidazole of the formula V

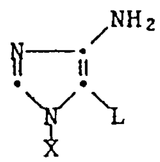

(V),

and the aminoimidazole V is reduced to form an imidazole of the formula I.

**2.** An N-cyanoformamidine of the formula II

X - NH - CH = N - CN (II)

in which the radical X has the definition given in claim 1.

**3.** An N,N-disubstituted N'-cyanoformamidine of the formula IV

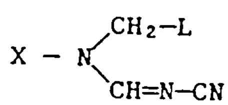

(IV)

in which the radicals X and L have the definitions given in claim 1.

**4.** A 4-aminoimidazole of the formula V

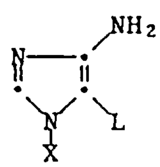

(V)

in which the radicals X and L have the definitions given in claim 1.

**5.** A process for the preparation of an imidazole of the formula I

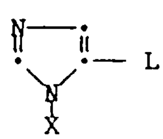

(I)

in which the radicals X and L have the definitions given in claim 1, characterised in that an aminoimidazole of the formula V

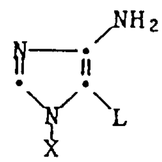

(V),

in which X and L have the definitions given above, is converted into a diazonium salt of the formula VI

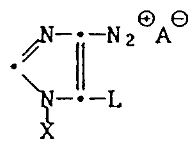

(VI)

in which X and L have the definitions given above and $A^{\ominus}$ represents an equivalent of an anion, and is reduced to form a compound of the formula I with nitrogen being removed.

**6.** A process according to claim 5, characterised in that the diazotisation is carried out with an alkyl nitrite of the formula XIII

Alk - O - NO (XIII),

in which Alk represents $C_1$-$C_8$alkyl, without isolation of the diazonium salt VI, in the presence of a reducing agent.

**7.** A process according to claim 6, characterised in that dimethylformamide, dioxane, tetrahydrofuran or a $C_1$-$C_6$-alcohol is used as reducing agent.

**8.** A process according to claim 5, characterised in that the diazotisation is carried out with an alkali metal nitrite in the presence of a mineral acid.

**9.** A diazonium salt of the formula VI

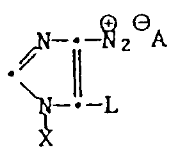

(VI)

in which the radicals X and L have the definitions given in claim 1 and $A^{\ominus}$ represents an equivalent of an anion.

**10.** A process for the preparation of a compound of the formula II according to claim 2, characterised in that

a) an amine of the formula VIII

X - $NH_2$ (VIII)

is condensed with an orthoester of the formula X

$CH(OD)_3$ (X),

in which D represents $C_1$-$C_4$alkyl or phenyl, and with a cyanamide of the formula IX

$H_2NCN$ (IX),

or

b) an amine of the formula VIII

$X - NH_2$ (VIII)

is condensed with an imido ester of the formula X

$DO - CH = N - CN$ (X),

in which D represents a $C_1$-$C_4$alkyl group or phenyl, or

c) an imido ester of the formula XI

$X - N = CH - OD$ (XI),

in which D represents a $C_1$-$C_4$alkyl group or phenyl, is condensed with cyanamide of the formula IX

$H_2NCN$ (IX).

**11.** A process for the preparation of an N,N-disubstituted N'-cyanoformamidine of the formula IV according to claim 3, characterised in that

a) an N-cyanoformamidine of the formula II

$X - NH - CH = N - CN$ (II)

is reacted with a compound of the formula III

$Z - CH_2 - L$ (III),

in which Z represents a nucleofugal group, in the presence of a base, or

b) in a first reaction step the N-cyanoformamidine of the formula II

$X - NH - CH = N - CN$ (II)

is deprotonated by means of a base to form a salt of the formula VII

$$X - \overset{\ominus}{N} - \overset{B^{\oplus}}{CH} = N - CN \qquad (VII),$$

in which $B^{\oplus}$ represents a cation equivalent, and is then alkylated with a compound of the formula III

$Z - CH_2 - L$ (III)

in which Z represents a nucleofugal group.

**12.** A process for the preparation of a 4-aminoimidazole of the formula V according to claim 4, characterised in that an N,N-disubstituted N'-cyanoformamidine of the formula IV is cyclised under the action of a base.

**13.** A process for the preparation of a diazonium salt of the formula VI according to claim 9, characterised in that diazotisation is effected in the 4-aminoimidazole of the formula V.

**14.** The use of a compound of the formula II, IV, V or VI according to claims 2 to 4 or 9 for carrying out a process according to claims 1, 5 or 10 to 13.

**15.** A process for the preparation of an imidazole of the formula I

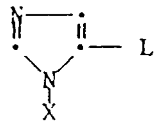

(I),

in which X and L have the definitions given in claim 1, characterised in that an amine of the formula VIII

$X - NH_2$ (VIII),

in which X has the definition given above, is condensed with an imido ester of the formula XI

$DO\text{-}CH = N\text{-}CN$ (XI),

in which D represents $C_1$-$C_4$ alkyl or phenyl, to form a compound of the formula II

$X\text{-}NH\text{-}CH = N\text{-}CN$ (II),

and this is then condensed with a compound of the formula III

$Z\text{-}CH_2\text{-}L$ (III),

in which L has the definition given above and R represents a nucleofugal group, to form a compound of the formula IV

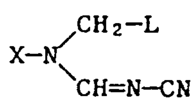

(IV),

and this is then cyclised under the action of a base to form a 4-aminoimidazole of the formula V

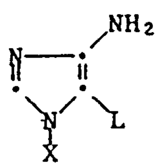

(V)

and then the aminoimidazole V so obtainable is deaminated with an alkyl nitrite of the formula XIII

$Alk - O - NO$ (XIII),

in which Alk represents $C_1$-$C_8$ alkyl, in the presence of a suitable reducing agent to form a compound of the formula I.

**16.** A process according to claim 11, characterised in that the reaction is carried out without isolation of the intermediates.

## Revendications

**1.** Procédé de préparation d'imidazoles de formule I

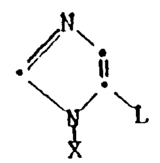

(I),

dans laquelle

| | |
|---|---|
| L et X | représentent ensemble $L^1$ et $X^1$; $L^2$ et $X^2$; $L^3$ et $X^3$; ou $L^4$ et $X^4$; et |
| $L^1$ | représente $COOR^1$; $CONR^2R^3$; $CONR^4NHR^3$; ou CN; |
| $R^1$ | un atome d'hydrogène, un radical alkyle en $C_1$-$C_7$; alcényle en $C_3$-$C_7$; alkynyle en $C_3$-$C_7$; cycloalkyle en $C_3$-$C_7$; alcoxy en $C_1$-$C_7$; alkyle en $C_1$-$C_7$ ou arylalkyle en $C_1$-$C_5$; |
| | les radicaux $R^2$, $R^3$ et $R^4$ représentent, indépendamment les uns des autres, un atome d'hydrogène, un radical alkyle en $C_1$-$C_5$; alcényle en $C_3$-$C_5$; alkynyle en $C_3$-$C_5$; cycloalkyle en $C_3$-$C_7$; aryle ou alkyle en $C_1$-$C_5$ substitué par un aryle, un cycloalkyle en $C_3$-$C_7$, un aryle ou un alkyle en $C_1$-$C_5$ substitué par un aryle, un cycloalkyle en $C_3$-$C_7$, un cycloalcoxy en $C_3$-$C_7$, un alcoxy en $C_1$-$C_5$, un hydroxy, un carboxyle ou un alkyloxycarbonyle en $C_1$-$C_5$; ou |
| $R_2$ et $R_3$ | représentent, avec l'atome d'azote auquel ils sont liés, un cycle pipéridinyle, pyrrolidinyle, 2-oxo-pipéridinyle, 2-oxo-pyrrolidinyle, morpholinyle, thiomorpholinyle, pipérazinyle ou 4-alkylpipérazinyle en $C_1$-$C_4$ non substitué ou substitué par 1 à 3 groupes alkyle en $C_1$-$C_5$; |
| $X^1$ | représente un radical 1-indanyle, 1-tétrahydronaphtalinyle, 5-benzocycloheptanyle, 4-tétrahydrobenzothiényle, 4-tétrahydrobenzofuranyle, 5-tétrahydroquinolinyle,5-tétrahydro-isoquilolinyle, 8-tétrahydroquinolinyle, 8-tétrahydro-isoquinolinyle, 9,10-dihydro-9-anthracényle, 9H-fluorène-9 yle, 2-dibenzo[a,d]-cycloheptényle, 5-dibenzo[a,d]- cycloheptanyle ou 1-dihydronaphta - linyle qui est non substitué ou qui est substitué jusqu'à 7 fois,de manière identique ou différente, par un radical alkyle en $C_1$-$C_5$, arylalkyle en $C_1$-$C_5$, di-arylalkyle en $C_1$-$C_5$, alcoxy en $C_1$-$C_5$, un atome d'halogène un radical alcényle en $C_3$-$C_7$, amino, nitro, alkylcarbonylamino en $C_1$-$C_5$, trifluorométhyle, ou difluorométhoxy ou dans lequel deux substituants à liaison géminale forment, avec l'atome de carbone auquel ils sont liés, un groupe spirocycloalkyle en $C_3$-$C_7$ ou dans lequel deux substituants représentent ensemble un groupe alkylène en $C_1$-$C_5$ ou un groupe cycloalkylène en $C_3$-$C_7$, tandis que ce groupe alkylène ou cycloalkylène peut éventuellement être substitué à son tour jusqu'à deux fois et indépendamment l'un de l'autre par un radical alkyle en $C_1$-$C_5$, arylalkyle en $C_1$-$C_5$, diarylalkyle en $C_1$-$C_5$; alcoxy en $C_1$-$C_5$, par un atome d'halogène, un radical alcényle en $C_3$-$C_7$,trifluorométhyle, difluorométhoxy ou aryle; ou |
| $X^1$ | représente le groupe |

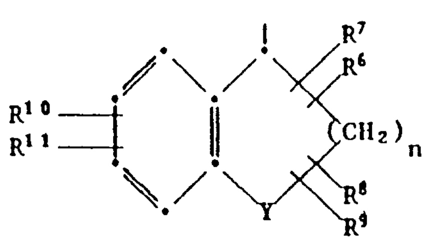

; ou

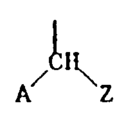

;

| | |
|---|---|
| n | représente le nombre zéro, un ou deux; |
| Y | représente les groupes $-CH_2S(O)_m-$, ou $-CH_2N(R^{12})$ dans lesquels l'hétéroatome |

est lié à l'atome de carbone du cycle benzénique et dans lequel m représente 0, 1 ou 2;

$R^6$, $R^7$, $R^8$ et $R^9$ représentent, indépendamment les uns des autres, un atome d'hydrogène, un alkyle en $C_1$-$C_5$, un arylalkyle en $C_1$-$C_5$, un diarylalkyle en $C_1$-$C_5$, un alcoxy en $C_1$-$C_5$, un atome d'halogène, un radical alcényle en $C_3$-$C_7$, trifluorométhyle, difluorométhoxy ou aryle ; ou

$R^6$ et $R^7$ représentent ensemble un radical benzénique à cycle qui peut éventuellement être substitué jusqu'à deux fois par un radical alkyle en $C_1$-$C_5$ alcoxy en $C_1$-$C_5$, par un atome d'halogène, par un radical alkyle en $C_1$-$C_5$ substitué de une à trois fois par un halogène, un radical alcoxy en $C_1$-$C_5$ substitué de une à trois fois par un halogène, un nitro, un amino ou -NH-CO-M; ou

$R^6$ et $R^7$ représentent, avec l'atome de carbone auquel ils sont liés géminalement, un cycle spirocyclique en $C_3$-$C_7$ ; ou

$R^6$ et $R^7$ représentent ensemble un groupe alkylène en $C_1$-$C_5$ ou cycloalkylène en $C_5$-$C_7$ qui peut éventuellement être substitué jusqu'à deux fois, indépendamment l'une de l'autre, par un radical alkyle en $C_1$-$C_5$, arylalkyle en $C_1$-$C_5$, alcoxy en $C_1$-$C_5$, par un atome d'halogène, par un radical alcényle en $C_3$-$C_7$, trifluorométhyle, difluorométhoxy ou aryle; et

$R^{10}$ et $R^{11}$ représentent, indépendamment l'un de l'autre, un atome d'hydrogène, un radical alkyle en $C_1$-$C_5$, alcoxy en $C_1$-$C_5$, un atome d'halogène, un radical trifluorométhyle, difluorométhoxy, cyano, nitro, amino, monoalkylamino en $C_1$-$C_5$, dialkylamino en $C_1$- $C_5$ ou -NH-CO-M; et

$R^{12}$ représente un atome d'hydrogène, un radical alkyle en $C_1$-$C_5$, alcanoyle en $C_1$-$C_5$ ou 4-méthylsulfonyle; et

A représente un atome d'hydrogène, un radical cycloalkyle en $C_3$-$C_7$ éventuellement substitué jusqu'à deux fois par un alkyle en $C_1$-$C_5$, un radical alkyle en $C_1$-$C_7$ éventuellement substitué par un alcoxy en $C_1$-$C_7$ ou un aryle, un radical alkyle en $C_1$-$C_7$ substitué par un alcoxy en $C_1$-$C_7$ et un radical aryle ou un radical pyridinyle, pyrimidinyle, naphtalinyle, furanyle ou thiophényle non substitué ou substitué jusqu'à deux fois, indépendamment l'une de l'autre, par un radical alkyle en $C_1$-$C_5$, alcoxy en $C_1$-$C_5$, par un atome d'halogène, un radical nitro, amino, mono, alkylamino en $C_1$-$C_5$, dialkylamino en $C_1$-$C_5$, -NH-CO-M, cyano, trifluorométhyle ou difluorométhoxy;

dans lequel, dans le cadre de la définition de A, le radical aryle peut être un radical phényle, pyridinyle, pyrimidinyle, naphtalinyle, furanyle ou thiophényle et dans lequel ce radical peut être substitué jusqu'à deux fois dans le cas où aryl = phényle et, également, jusqu'à trois fois indépendamment les unes des autres, par un radical alkyle en $C_1$-$C_5$, alcoxy en $C_1$-$C_5$, par un atome d'halogène, un radical nitro, amino, mono, alkylamino en $C_1$-$C_5$, dialkylamino en $C_1$-$C_5$, -NH-CO-M, cyano, trifluorométhyle ou difluorométhoxy; et

Z représente un radical naphtalinyle, thiophényle, furanyle, pyrimidinyle, phényle ou pyridinyle non substitué ou substitué jusqu'à trois fois, indépendamment les unes des autres, par un radical alkyle en $C_1$-$C_5$, alcoxy en $C_1$-$C_5$, par un atome d'halogène, un radical cyano, nitro, amino, mono-alkylamino en $C_1$-$C_5$, dialkylamino en $C_1$-$C_5$, -NH-CO-M, trifluorométhyle ou difluorométhoxy;et

M représente un radical alkyle en $C_1$-$C_5$; et

Aryle, dans le cadre des définitions précédentes des radicaux X et L, peut représenter en outre un radical phényle non substitué ou substitué jusqu'à trois fois, de manière identique ou différente,par un radical alkyle en $C_1$-$C_5$, alkyloxy en $C_1$-$C_5$ ou par un atome d'halogène; et

$L^2$ représente CO-D-$R^{13}$;

D représente un atome d'oxygène ou $NR^{13}$;

$R^{13}$ représente un atome d'hydrogène,un radical phényle, alcényle en $C_3$-$C_6$, alkyle en $C_1$-$C_{12}$ non substitué ou substitué jusqu'à trois fois de manière identique ou différente par un radical alcoxy en $C_1$-$C_6$; dialkylamino en $C_1$-$C_3$ ou par un atome d'halogène; et

$R^{13}$ représente, si D représente O, également un cation d'un métal du groupe I., II ou VII du système périodique ou de l'ammonium; et

$X_2$ représente un radical

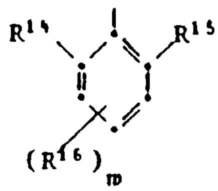

m représente les nombres 0, 1, 2 ou 3; et

$R^{14}$ et $R^{15}$ représentent, indépendamment l'un de l'autre, un radical alkyle en $C_1$-$C_4$;

$R^{16}$ représente des radicaux identiques ou différents choisis dans les groupes alkyle en $C_1$-$C_4$, alcoxy en $C_1$-$C_4$ et les atomes d'halogène;

$L^3$ représente CN, $COOR^{17}$ ou $CONR^{18}R^{19}$, et

$R^{17}$ représente un radical alkyle en $C_1$-$C_7$, alcényle en $C_3$-$C_7$, alkynyle en $C_3$-$C_7$, alcoxy-$C_1$-$C_7$-alkyle en $C_1$-$C_7$, cycloalkyle en $C_3$-$C_7$ ou arylalkyle en $C_1$-$C_5$ non substitué ou substitué par un atome d'halogène; et

$R^{18}$ et $R^{19}$ qui peuvent être identiques ou différents, représentent un atome d'hydrogène, un radical alkyle en $C_1$-$C_4$, alcényle en $C_3$-$C_7$, alkynyle en $C_3$-$C_7$, alcoxy -$C_1$-$C_7$-alkyle en $C_1$-$C_7$; ou

$R^{18}$ et $R^{19}$ constituent, avec l'atome d'azote auquel ils sont liés, un cycle saturé ou non saturé ayant de 3 à 7 éléments, qui contient jusqu'à trois hétéroatomes choisis dans le grope O, N et S et qui est non substitué ou est substitué par un radical alkyle ($C_1$-$C_4$) ou par un atome d'halogène et qui peut contenir un groupe carbonyle; et

$X^3$ représente un radical

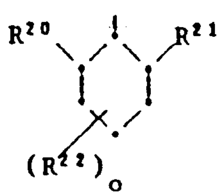

ou

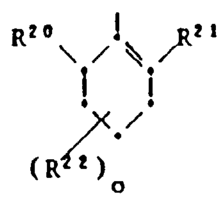

;

o représente un nombre allant de 0 à 6;

$R^{20}$ et $R^{21}$ représentent, indépendamment l'un de l'autre, un atome d'hydrogène ou un radical alkyle ($C_1$-$C_4$); et

$R^{22}$ représente, indépendamment les uns des autres, un radical alkyle en $C_1$-$C_4$, alcoxy en $C_1$-$C_4$, hydroxyalkyle en $C_1$-$C_4$, alcoxy-$C_1$-$C_4$-alkyle en $C_1$-$C_4$ ou un atome d'halogène; et

$L^4$ représente CO-$R^{23}$ ou CN;

$R^{23}$ représente un radical hydroxy, alcoxy en $C_1$-$C_6$,hydroxy-alcoxy($C_2$-$C_6$), alcoxy-($C_2$-$C_6$)-alcoxy, amino, alkylamino en $C_1$-$C_6$, dialkylamino en $C_1$-$C_6$, dialkylamino-($C_1$-$C_6$)-alkylamino($C_1$-$C_3$), alcoxyamino en $C_1$-$C_3$, anilino, N-pyrrolidino, N-pipéridino, N-morpholino, hydrazino, N'-($C_1$-$C_3$)-alkylhydrazino, N,N'-diméthylhydrazino ou N'-phénylhydrazino; et

$X^4$ représente le radical

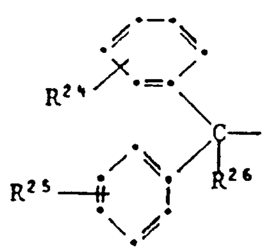

et

| | |
|---|---|
| $R^{24}$ et $R^{25}$, | qui peuvent être identiques ou différents, représentent un atome d'hydrogène ou d'halogène, un radical alkyle en $C_1$-$C_3$, trifluorométhyle, hydroxy, alcoxy en $C_1$-$C_3$, alcoxy en ($C_1$-$C_3$) halogéné, alkylthio en $C_1$-$C_3$, cyano, nitro ou acétamino; et |
| $R^{26}$ | représente un atome d'hydrogène ou un radical phényle, |

caractérisé en ce que l'on effectue l'alkylation des N-cyanoformamidines de formule II

X - NH - CH = N - CN (II)

dans laquelle X est tel que défini ci-dessus, avec un composé de la formule III

Z - $CH_2$ - L (III)

dans laquelle L est tel que défini ci-dessus et Z représente un groupe nucléofuge, pour obtenir un composé de la formule IV

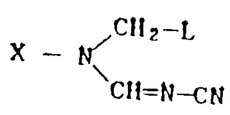

(IV)

et qu'on effectue la cyclisation du composé IV sous l'effet d'une base pour obtenir un 4-amino-imidazole de formule V

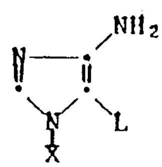

(V)

puis que l'on effectue la réduction de l'amino-imidazole V pour obtenir un imidazole de formule I.

**2.** N-cyanoformamidines de formule II

X - NH - CH = N - CN (II)

dans laquelle le radical X est tel que défini à la revendication 1.

**3.** N-cyanoformamidines N,N-disubstituées de formule IV

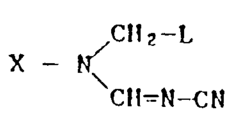

(IV)

dans laquelle les radicaux X et L sont tels que définis dans la revendication 1.

**4.** 4-amino-imidazoles de formule V

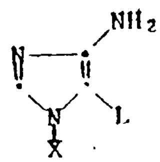

(V)

dans laquelle les radicaux X et L sont tels que définis dans la revendication 1.

**5.** Procédé de préparation d'imidazoles de la formule I

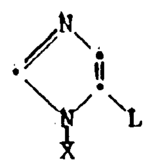

(I),

dans laquelle les radicaux X et L sont tels que définis dans la revendication 1, caractérisé en ce que l'on transforme un amino-imidazole de formule V

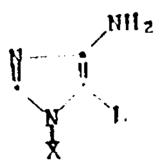

(V)

dans laquelle X et L sont tels que définis ci-dessus en un sel de diazonium de formule IV

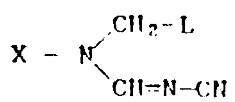

(IV)

dans laquelle X et L sont tels que définis ci-dessus et $A^{(-)}$ représente un équivalent d'un anion et que l'on réduit pour obtenir un composé de la formule I avec dégagement d'azote.

**6.** Procédé selon la revendication 5, caractérisé en ce que l'on effectue la diazotation avec un nitrite d'alkyle de formule XIII

Alk--O--NO (XIII)

dans laquelle Alk représente un radical alkyle en $C_1$-$C_8$ sans séparation du sel de diazonium VI en présence d'un agent de réduction.

**7.** Procédé selon la revendication 6, caractérisé en ce que l'on utilise comme agent de réduction du diméthylformamide, du dioxane, du tétraliydrofuranne ou un alcool en $C_1$-$C_6$.

**8.** Procédé selon la revendication 5, caractérisé en ce que l'on effectue la diazotation avec un nitrite alcalin en présence d'un acide minéral.

**9.** Sels de diazonium de formule VI

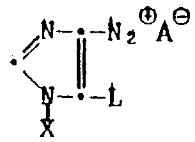

(VI),

dans laquelle les radicaux X et L sont tels que définis dans la revendication 1 et $A^{(-)}$ représente un équivalent d'un anion.

**10.** Procédé de préparation de composés de la formule II selon la revendication 2, caractérisé en ce que
a)on effectue la condensation d'une amine de formule VIII

$X - NH_2$ (VIII)

avec un orthoester de formule X

$CH(OD)_3$ (X)

dans laquelle D représente, de préférence, un radical alkyle en $C_1$-$C_4$ et avec le cyanamide de formule IX

$H_2NCN$ (IX)

ou
b)on effectue la condensation d'une amine de formule VIII

$X - NH_2$ (VIII)

avec un imidoester de formule XI

DO - CH = N - CN (XI)

dans laquelle D représente un groupe alkyle en $C_1$-$C_4$ ou un radical phényle, ou
c)on effectue la condensation d'un imidoester de formule XII

X - N = CH - OD (XII)

dans laquelle D représente un groupe alkyle en $C_1$-$C_4$ ou un radical phényle avec un cyanamide de formule IX

$H_2NCN$ (IX)

**11.** Procédé de préparation de N'-cyano-formamidine -N,N-disubstitué de formule IV selon la revendication 3, caractérisé en ce que
a) on fait réagir une N-cyanoformamidine de formule II

X - NH - CH = N - CN (II)

avec un composé de formule III

$Z - CH_2 L$ (III)

dans laquelle Z représente un groupe nucléofuge en présence d'une base , ou
b) on effectue, au cours d'une première étape de la réaction, la déprotonisation de la N-cyanoformamidine de formule II

X - NH - CH = N - CN (II)

au moyen d'une base pour obtenir un sel de la formule VII

$$X - \overset{\ominus}{N} - \overset{B^{\oplus}}{CH} = N - CN \qquad (VII),$$

dans laquelle $B^{(+)}$ représente un équivalent de cation et qu'on effectue ensuite l'alkylation avec un composé de formule III

Z - $CH_2$ L (III)

dans laquelle Z représente un groupe nucléofuge.

**12.** Procédé de préparation de 4-aminoimidazoles de formule V selon la revendication 4, caractérisé en ce que l'on effectue la cyclisation d'une N'-cyanoformamidine N,N-disubstituée de formule IV sous l'effet d'une base.

**13.** Procédé de préparation de sels de diazonium de formule VI selon la revendication 9, caractérisé en ce que l'on effectue la diazotation dans le 4-amino-imidazole de formule V.

**14.** Utilisation de composés des formules II, IV, V ou VI selon les revendications 2 à 4 ou 9 pour mettre en oeuvre un procédé selon la revendication 1, 5 ou 10 à 13.

**15.** Procédé de préparation d'imidazoles de formule I

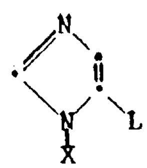

(I),

dans laquelle X et L sont tels que définis dans la revendication 1, caractérisé en ce qu'on condense une amine de formule VIII

X--$NH_2$ (VIII)

dans laquelle X est tel que défini ci-dessus, avec un imidoester de formule XI

DO-CH=N-CN (XI)

dans laquelle D représente un radical alkyle en $C_1$-$C_4$ ou phényle pour obtenir un composé de la formule II

X-NH-CH=N-CN (II)

et qu'on condense ensuite celui-ci avec un composé de la formule III

Z-$CH_2$-L (III)

dans laquelle L est tel que défini ci-dessus et R représente un groupe nucléofuge pour obtenir un composé de la formule IV

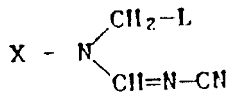

(IV)

et qu'on effectue ensuite la cyclisation de celui-ci sous l'effet d'une base pour obtenir un 4-amino-imidazole de formule V

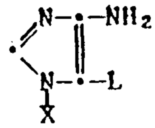

(V),

et qu'on effectue ensuite la désaminisation de l'amino-imidazole V ainsi obtenu avec un nitrite d'alkyle de formule XIII

Alk--O--NO (XIII)

dans laquelle Alk représente un radical alkyle en $C_1$-$C_8$ en présence d'un agent de réduction approprié pour obtenir un composé de formule I.

**16.** Procédé selon la revendication 11, caractérisé en ce que l'on effectue la réaction sans séparation des produits intermédiaires.